(19) Europäisches Patentamt
European Patent Office
Office européen des brevets

(11) EP 1 567 496 B1

(12) EUROPEAN PATENT SPECIFICATION

(45) Date of publication and mention of the grant of the patent:
**11.04.2007 Bulletin 2007/15**

(21) Application number: **03759165.8**

(22) Date of filing: **05.11.2003**

(51) Int Cl.:
***C07D 211/70*** *(2006.01)* ***A61K 31/4465*** *(2006.01)*
***A61P 25/04*** *(2006.01)*

(86) International application number:
**PCT/SE2003/001705**

(87) International publication number:
**WO 2004/041784 (21.05.2004 Gazette 2004/21)**

# (54) PHENYL-PIPERIDIN-4-YLIDENE-METHYL-BENZAMIDE DERIVATIVES FOR THE TREATMENT OF PAIN OR GASTROINTESTINAL DISORDERS

PHENYL-PIPERIDIN-4-YLIDEN-METHYL-BENZAMIDDERIVATE ZUR BEHANDLUNG VON SCHMERZ ODER MAGEN-DARM-ERKRANKUNGEN

DERIVES DE PHENYL-PIPERIDIN-4-YLIDENE-METHYL-BENZAMIDE POUR LE TRAITEMENT DE LA DOULEUR ET DE TROUBLES GASTRO-INTESTINAUX

(84) Designated Contracting States:
**AT BE BG CH CY CZ DE DK EE ES FI FR GB GR HU IE IT LI LU MC NL PT RO SE SI SK TR**

(30) Priority: **07.11.2002 SE 0203301**

(43) Date of publication of application:
**31.08.2005 Bulletin 2005/35**

(73) Proprietor: **AstraZeneca AB**
**151 85 Södertälje (SE)**

(72) Inventors:
- **BROWN, William,**
**AstraZeneca R & D Montreal**
**Montreal,**
**Québec H4S 1Z9 (CA)**
- **GRIFFIN, Andrew,AstraZeneca R & D Montreal**
**Montreal,**
**Québec H4S 1Z9 (CA)**

(56) References cited:
**WO-A1-98/28275**

Remarks:
The file contains technical information submitted after the application was filed and not included in this specification

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 567 496 B1

## Description

## BACKGROUND OF THE INVENTION

### 1. Field of the Invention

The present invention is directed to novel compounds, to a process for their preparation, their use and pharmaceutical compositions comprising the novel compounds. The novel compounds are useful in therapy, and in particular for the treatment of pain, anxiety and functional gastrointestinal disorders.

### 2. Discussion of Relevant Art

The δ receptor has been identified as having a role in many bodily functions such as circulatory and pain systems. Ligands for the δ receptor may therefore find potential use as analgesics, and/or as antihypertensive agents. Ligands for the δ receptor have also been shown to possess immunomodulatory activities.

The identification of at least three different populations of opioid receptors (μ, δ and κ) is now well established and all three are apparent in both central and peripheral nervous systems of many species including man. Analgesia has been observed in various animal models when one or more of these receptors has been activated.

With few exceptions, currently available selective opioid δ ligands are peptidic in nature and are unsuitable for administration by systemic routes. One example of a non-peptidic δ-agonist is SNC80 (Bilsky E.J. et al., Journal of Pharmacology and Experimental Therapeutics, 273(1), pp. 359-366 (1995)).

Many δ agonist compounds that have been identified in the prior art have many disadvantages in that they suffer from poor pharmacokinetics and are not analgesic when administered by systemic routes. Also, it has been documented that many of these δ agonist compounds show significant convulsive effects when administered systemically.

U.S. Patent No. 6,187,792 to Delorme et al. describes some δ-agonists.

However, there is still a need for improved δ-agonists.

## DETAILED DESCRIPTION OF THE INVENTION

### Definitions

Unless specified otherwise within this specification, the nomenclature used in this specification generally follows the examples and rules stated in *Nomenclature of Organic Chemistry, Sections A, B, C, D, E, F, and H,* Pergamon Press, Oxford, 1979, which is incorporated by references herein for its exemplary chemical structure names and rules on naming chemical structures.

The term "$C_{m-n}$" or "$C_{m-n}$ group" used alone or as a prefix, refers to any group having m to n carbon atoms.

The term "hydrocarbon" used alone or as a suffix or prefix, refers to any structure comprising only carbon and hydrogen atoms up to 14 carbon atoms.

The term "hydrocarbon radical" or "hydrocarbyl" used alone or as a suffix or prefix, refers to any structure as a result of removing one or more hydrogens from a hydrocarbon.

The term "alkyl" used alone or as a suffix or prefix, refers to monovalent straight or branched chain hydrocarbon radicals comprising 1 to about 12 carbon atoms. An "alkyl" may optionally contain one or more unsaturated carbon-carbon bonds.

The term "alkylene" used alone or as suffix or prefix, refers to divalent straight or branched chain hydrocarbon radicals comprising 1 to about 12 carbon atoms, which serves to links two structures together.

The term "alkenyl" used alone or as suffix or prefix, refers to a monovalent straight or branched chain hydrocarbon radical having at least one carbon-carbon double bond and comprising at least 2 up to about 12 carbon atoms.

The term "alkynyl" used alone or as suffix or prefix, refers to a monovalent straight or branched chain hydrocarbon radical having at least one carbon-carbon triple bond and comprising at least 2 up to about 12 carbon atoms.

The term "cycloalkyl," used alone or as suffix or prefix, refers to a monovalent ring-containing hydrocarbon radical comprising at least 3 up to about 12 carbon atoms.

The term "cycloalkenyl" used alone or as suffix or prefix, refers to a monovalent ring-containing hydrocarbon radical having at least one carbon-carbon double bond and comprising at least 3 up to about 12 carbon atoms.

The term "cycloalkynyl" used alone or as suffix or prefix, refers to a monovalent ring-containing hydrocarbon radical having at least one carbon-carbon triple bond and comprising about 7 up to about 12 carbon atoms.

The term "aryl" used alone or as suffix or prefix, refers to a monovalent hydrocarbon radical having one or more polyunsaturated carbon rings having aromatic character, (*e.g.*, 4n + 2 delocalized electrons) and comprising 5 up to about 14 carbon atoms.

The term "arylene" used alone or as suffix or prefix, refers to a divalent hydrocarbon radical having one or more polyunsaturated carbon rings having aromatic character, (*e.g.*, 4n + 2 delocalized electrons) and comprising 5 up to about 14 carbon atoms, which serves to link two structures together.

The term "heterocycle" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s). Heterocycle may be saturated or unsaturated, containing one or more double bonds, and heterocycle may contain more than one ring. When a heterocycle contains more than one ring, the rings may be fused or unfused. Fused rings generally refer to at least two rings share two atoms therebetween. Heterocycle may have aromatic character or may not have aromatic character.

The term "heteroaromatic" used alone or as a suffix or prefix, refers to a ring-containing structure or molecule having one or more multivalent heteroatoms, independently selected from N, O, P and S, as a part of the ring structure and including at least 3 and up to about 20 atoms in the ring(s), wherein the ring-containing structure or molecule has an aromatic character (*e.g.*, 4n + 2 delocalized electrons).

The term "heterocyclic group," "heterocyclic moiety," "heterocyclic," or "heterocyclo" used alone or as a suffix or prefix, refers to a radical derived from a heterocycle by removing one or more hydrogens therefrom.

The term "heterocyclyl" used alone or as a suffix or prefix, refers a monovalent radical derived from a heterocycle by removing one hydrogen therefrom.

The term "heterocyclylene" used alone or as a suffix or prefix, refers to a divalent radical derived from a heterocycle by removing two hydrogens therefrom, which serves to links two structures together.

The term "heteroaryl" used alone or as a suffix or prefix, refers to a heterocyclyl having aromatic character.

The term "heterocylcoalkyl" used alone or as a suffix or prefix, refers to a heterocyclyl that does not have aromatic character.

The term "heteroarylene" used alone or as a suffix or prefix, refers to a heterocyclylene having aromatic character.

The term "heterocycloalkylene" used alone or as a suffix or prefix, refers to a heterocyclylene that does not have aromatic character.

The term "six-membered" used as prefix refers to a group having a ring that contains six ring atoms.

The term "five-membered" used as prefix refers to a group having a ring that contains five ring atoms.

A five-membered ring heteroaryl is a heteroaryl with a ring having five ring atoms wherein 1, 2 or 3 ring atoms are independently selected from N, O and S.

Exemplary five-membered ring heteroaryls are thienyl, furyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4- oxadiazolyl.

A six-membered ring heteroaryl is a heteroaryl with a ring having six ring atoms wherein 1, 2 or 3 ring atoms are independently selected from N, O and S.

Exemplary six-membered ring heteroaryls are pyridyl, pyrazinyl, pyrimidinyl, triazinyl and pyridazinyl.

The term "substituted" used as a prefix refers to a structure, molecule or group, wherein one or more hydrogens are replaced with one or more $C_{1-6}$hydrocarbon groups, or one or more chemical groups containing one or more heteroatoms selected from N, O, S, F, Cl, Br, I, and P. Exemplary chemical groups containing one or more heteroatoms include $-NO_2$, -OR, -Cl, -Br, -I, -F, $-CF_3$,- C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, oxo (=O), imino (=NR), thio (=S), and oximino (=N-OR), wherein each "R" is a $C_{1-6}$hydrocarbyl. For example, substituted phenyl may refer to nitrophenyl, methoxyphenyl, chlorophenyl, aminophenyl, etc., wherein the nitro, methoxy, chloro, and amino groups may replace any suitable hydrogen on the phenyl ring.

The term "substituted" used as a suffix of a first structure, molecule or group, followed by one or more names of chemical groups refers to a second structure, molecule or group, which is a result of replacing one or more hydrogens of the first structure, molecule or group with the one or more named chemical groups. For example, a "phenyl substituted by nitro" refers to nitrophenyl.

Heterocycle includes, for example, monocyclic heterocycles such as: aziridine, oxirane, thiirane, azetidine, oxetane, thietane, pyrrolidine, pyrroline, imidazolidine, pyrazolidine, pyrazoline, dioxolane, sulfolane 2,3-dihydrofuran, 2,5-dihydrofuran tetrahydrofuran, thiophane, piperidine, 1,2,3,6-tetrahydro-pyridine, piperazine, morpholine, thiomorpholine, pyran, thiopyran, 2,3-dihydropyran, tetrahydropyran, 1,4-dihydropyridine, 1,4-dioxane, 1,3-dioxane, dioxane, homopiperidine, 2,3,4,7-tetrahydro-1*H*-azepine homopiperazine, 1,3-dioxepane, 4,7-dihydro-1,3-dioxepin, and hexamethylene oxide.

In addition, heterocycle includes aromatic heterocycles, for example, pyridine, pyrazine, pyrimidine, pyridazine, thiophene, furan, furazan, pyrrole, imidazole, thiazole, oxazole, pyrazole, isothiazole, isoxazole, 1,2,3-triazole, tetrazole, 1,2,3-thiadiazole, 1,2,3-oxadiazole, 1,2,4-triazole, 1,2,4-thiadiazole, 1,2,4-oxadiazole, 1,3,4-triazole, 1,3,4-thiadiazole, and 1,3,4- oxadiazole.

Additionally, heterocycle encompass polycyclic heterocycles, for example, indole, indoline, isoindoline, quinoline, tetrahydroquinoline, isoquinoline, tetrahydroisoquinoline, 1,4-benzodioxan, coumarin, dihydrocoumarin, benzofuran, 2,3-dihydrobenzofuran, isobenzofuran, chromene, chroman, isochroman, xanthene, phenoxathiin, thianthrene, indolizine, isoindole, indazole, purine, phthalazine, naphthyridine, quinoxaline, quinazoline, cinnoline, pteridine, phenanthridine, perimidine, phenanthroline, phenazine, phenothiazine, phenoxazine, 1,2-benzisoxazole, benzothiophene, benzoxazole, benzthiazole, benzimidazole, benztriazole, thioxanthine, carbazole, carboline, acridine, pyrolizidine, and quinolizidine.

In addition to the polycyclic heterocycles described above, heterocycle includes polycyclic heterocycles wherein the ring fusion between two or more rings includes more than one bond common to both rings and more than two atoms common to both rings. Examples of such bridged heterocycles include quinuclidine, diazabicyclo[2.2.1]heptane and 7-oxabicyclo[2.2.1]heptane.

Heterocyclyl includes, for example, monocyclic heterocyclyls, such as: aziridinyl, oxiranyl, thiiranyl, azetidinyl, oxetanyl, thietanyl, pyrrolidinyl, pyrrolinyl, imidazolidinyl, pyrazolidinyl, pyrazolinyl, dioxolanyl, sulfolanyl, 2,3-dihydrofuranyl, 2,5-dihydrofuranyl, tetrahydrofuranyl, thiophanyl, piperidinyl, 1,2,3,6-tetrahydro-pyridinyl, piperazinyl, morpholinyl, thiomorpholinyl, pyranyl, thiopyranyl, 2,3-dihydropyranyl, tetrahydropyranyl, 1,4-dihydropyridinyl, 1,4-dioxanyl, 1,3-dioxanyl, dioxanyl, homopiperidinyl, 2,3,4,7-tetrahydro-1*H*-azepinyl, homopiperazinyl, 1,3-dioxepanyl, 4,7-dihydro-1,3-dioxepinyl, and hexamethylene oxidyl.

In addition, heterocyclyl includes aromatic heterocyclyls or heteroaryl, for example, pyridinyl, pyrazinyl, pyrimidinyl, pyridazinyl, thienyl, furyl, furazanyl, pyrrolyl, imidazolyl, thiazolyl, oxazolyl, pyrazolyl, isothiazolyl, isoxazolyl, 1,2,3-triazolyl, tetrazolyl, 1,2,3-thiadiazolyl, 1,2,3-oxadiazolyl, 1,2,4-triazolyl, 1,2,4-thiadiazolyl, 1,2,4-oxadiazolyl, 1,3,4-triazolyl, 1,3,4-thiadiazolyl, and 1,3,4 oxadiazolyl.

Additionally, heterocyclyl encompasses polycyclic heterocyclyls (including both aromatic or non-aromatic), for example, indolyl, indolinyl, isoindolinyl, quinolinyl, tetrahydroquinolinyl, isoquinolinyl, tetrahydroisoquinolinyl, 1,4-benzodioxanyl, coumarinyl, dihydrocoumarinyl, benzofuranyl, 2,3-dihydrobenzofuranyl, isobenzofuranyl, chromenyl, chromanyl, isochromanyl, xanthenyl, phenoxathiinyl, thianthrenyl, indolizinyl, isoindolyl, indazolyl, purinyl, phthalazinyl, naphthyridinyl, quinoxalinyl, quinazolinyl, cinnolinyl, pteridinyl, phenanthridinyl, perimidinyl, phenanthrolinyl, phenazinyl, phenothiazinyl, phenoxazinyl, 1,2-benzisoxazolyl, benzothiophenyl, benzoxazolyl, benzthiazolyl, benzimidazolyl, benztriazolyl, thioxanthinyl, carbazolyl, carbolinyl, acridinyl, pyrolizidinyl, and quinolizidinyl.

In addition to the polycyclic heterocyclyls described above, heterocyclyl includes polycyclic heterocyclyls wherein the ring fusion between two or more rings includes more than one bond common to both rings and more than two atoms common to both rings. Examples of such bridged heterocycles include quinuclidinyl, diazabicyclo[2.2.1]heptyl; and 7-oxabicyclo[2.2.1]heptyl.

The term "alkoxy" used alone or as a suffix or prefix, refers to radicals of the general formula -O-R, wherein R is selected from a hydrocarbon radical. Exemplary alkoxy includes methoxy, ethoxy, propoxy, isopropoxy, butoxy, t-butoxy, isobutoxy, cyclopropylmethoxy, allyloxy, and propargyloxy.

The term "amine" or "amino" used alone or as a suffix or prefix, refers to radicals of the general formula -NRR', wherein R and R' are independently selected from hydrogen or a hydrocarbon radical.

Halogen includes fluorine, chlorine, bromine and iodine.

"Halogenated," used as a prefix of a group, means one or more hydrogens on the group is replaced with one or more halogens.

"RT" or "rt" means room temperature.

Description of Embodiments

In one aspect, the invention provides a compound of formula I, diastereomers thereof and pharmaceutically acceptable salts thereof:

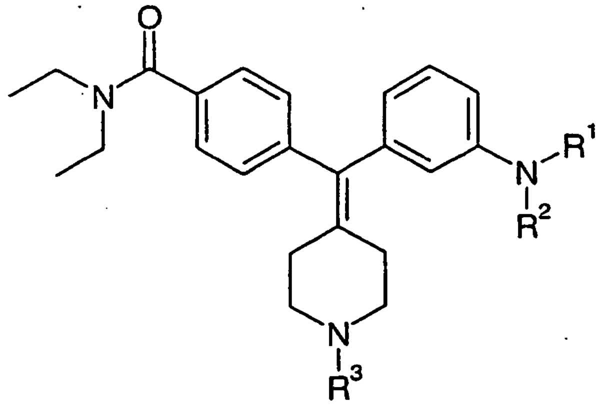

I

wherein

$R^1$ is selected from $C_{3-6}$alkyl, $C_{6-10}$aryl, $C_{2-9}$heteroaryl, $C_{6-10}$aryl-$C_{1-4}$alkyl, $C_{2-9}$heteroaryl-$C_{1-4}$alkyl, $C_{3-10}$cycloalkyl, $C_{3-10}$cycloalkyl-$C_{1-4}$alkyl, $R^8$-C(=O)-, $R^8$-$S(=O)_2$-, $R^8$-S(=O)-, $R^8$-NHC(=O)-, $R^8$-C(=S)- and $R^8$-NH-C(=S)-, wherein $R^8$ is selected from $C_{3-6}$alkyl, $C_{6-10}$aryl, $C_{2-9}$heteroaryl, $C_{6-10}$aryl-$C_{1-4}$alkyl, $C_{2-9}$heteroaryl-$C_{1-4}$alkyl, $C_{3-10}$cycloalkyl, and $C_{3-10}$cycloalkyl-$C_{1-4}$alkyl, wherein said $C_{3-6}$alkyl, $C_{6-10}$aryl, $C_{2-9}$heteroaryl, $C_{6-10}$aryl-$C_{1-4}$alkyl, $C_{2-9}$heteroaryl-$C_{1-4}$alkyl, $C_{3-10}$cycloalkyl, and $C_{3-10}$cycloalkyl-$C_{1-4}$alkyl used in defining $R^1$ and $R^8$ are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, $-C(=O)NR_2$, -NRC(=O)R, and -NRC(=O)-OR, wherein R is, independently, selected from -H, $C_{1-6}$alkyl and phenyl;
$R^2$ is selected from -H and $C_{1-6}$alkyl optionally substituted with one or more groups selected from $-CF_3$, -OH, $C_{1-3}$alkoxy, and halogen; and
$R^3$ is selected from -H, $C_{1-6}$alkyl-O-C(=O)-, $C_{1-6}$alkyl, $C_{3-6}$cycloalkyl, and $C_{3-6}$cycloalkyl-$C_{1-4}$alkyl wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

In one embodiment, the compounds of the present invention are represented by formula I, wherein

$R^1$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl, wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen;
$R^2$ is selected from -H and $C_{1-3}$alkyl; and
$R^3$ is selected from-H and $C_{1-6}$alkyl-O-C(=O)-.

In another embodiment, the compounds of the present invention are represented by formula I, wherein $R^1$ is $R^9$-$CH_2$-, wherein $R^9$ is selected from phenyl, pyridyl, thienyl, furyl, imidazolyl, triazolyl, pyrrolyl, thiazolyl, N-oxido-pyridyl, benzyl, pyridylmethyl, thienylmethyl, furylmethyl, imidazolylmethyl, triazolylmethyl, pyrrolylmethyl, thiazolylmethyl and N-oxido-pyridylmethyl, optionally substituted with one or more groups selected from $C_{1-4}$alkyl, $-CF_3$, -OH, $C_{1-3}$alkoxy, phenoxy and halogen; and
$R^2$ and $R^3$ are hydrogen.

In another embodiment, the compounds of the present invention are represented by formula I,
wherein $R^1$ is $R^9$-$CH_2$-, wherein $R^9$ is selected from benzyl, phenyl, pyridyl, thienyl, furyl, imidazolyl, pyrrolyl and thiazolyl, optionally substituted with one or more groups selected from $C_{1-4}$alkyl, $-CF_3$, -OH, $C_{1-3}$alkoxy, phenoxy, and halogen; and $R^2$ and $R^3$ are hydrogen.

In another embodiment, the compounds of the present invention are represented by formula I, wherein
$R^1$ is $R^9$-$CH_2$-, wherein $R^9$ is selected from benzyl, phenyl, pyridyl, thienyl, furyl, imidazolyl, pyrrolyl and thiazolyl; and $R^2$ and $R^3$ are hydrogen.

In another embodiment, the compounds of the present invention are represented by formula I, wherein
$R^1$ is selected from $C_{3-6}$alkyl, $C_{3-10}$Cycloalkyl, and $C_{3-10}$cycloalkyl-$C_{1-4}$alkyl,

wherein said $C_{3-6}$alkyl, $C_{3-10}$cycloalkyl, and $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen;
$R^2$ is -H or $C_{1-3}$alkyl; and
$R^3$ is -H, $C_{1-6}$alkyl, $C_{3-6}$cycloalkyl, and $C_{3-6}$cycloalkyl-$C_{1-4}$alkyl, wherein said $C_{1-6}$alkyl, $C_{3-6}$cycloalkyl, $C_{3-6}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen.

In another embodiment, the compounds of the present invention are represented by formula I, wherein

$R^1$ is selected from 1-propyl, 2-propyl, 1-butyl, 2-butyl, t-butyl, 2-methyl-1-propyl, cyclopentyl, cyclohexyl, cycloheptyl, cyclooctyl, and cyclononyl;
$R^2$ is selected from -H, methyl, ethyl, 1 propyl and 2-propyl; and
$R^3$ is selected from-H, methyl, ethyl, allyl, 3,3-dimethyl-allyl, cyclopropylmethyl, 2-methoxy-ethyl, and 3-methoxy-1-propyl.

In another embodiment, the compounds of the present invention are represented by formula I, wherein

$R^1$ is selected from $R^8$-C(=O)-, $R^8$-S(=O)$_2$-, $R^8$-S(=O)-, $R^8$-NHC(=O)-, $R^8$-C(=S)- and $R^8$-NH-C(=S)-, wherein $R^8$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl; wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl are optionally substituted with $C_{1-4}$alkyl, $-CF_3$, -OH, $C_{1-3}$alkoxy, phenoxy, and halogen;
$R^2$ is -H; and
$R^3$ is selected from -H and $C_{1-6}$alkyl-O-C(=O)-.

In a further embodiment, the compounds of the present invention are represented by formula I, wherein

$R^1$ is selected from $R^8$-C(=O)-, $R^8$-S(=O)$_2$-, $R^8$-S(=O)-, $R^8$-NHC(=O)-, $R^8$-C(=S)- and $R^8$-NH-C(=S)-, wherein $R^8$ is selected from phenyl, benzyl, phenethyl and cyclohexyl, wherein said phenyl, benzyl, phenethyl and cyclohexyl are optionally substituted with one or more groups selected from methyl, methoxy and halogen;
$R^2$ is -H; and
$R^3$ is selected from-H and $C_{1-6}$alkyl-O-C(=O)-.

In an even further embodiment, the compounds of the present invention are those of formula I, wherein

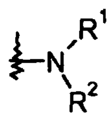

of formula I is selected from

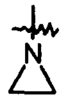 , 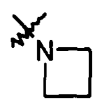 , 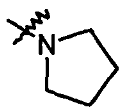 and 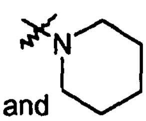 ;

and
$R^3$ is selected from -H and $C_{1-6}$alkyl-O-C(=O)-.

It will be understood that when compounds of the present invention contain one or more chiral centers, the compounds of the invention may exist in, and be isolated as, enantiomeric or diastereomeric forms, or as a racemic mixture. The present invention includes any possible enantiomers, diastereomers, racemates or mixtures thereof, of a compound of Formula I. The optically active forms of the compound of the invention may be prepared, for example, by chiral chromatographic separation of a racemate, by synthesis from optically active starting materials or by asymmetric synthesis based on the procedures described thereafter.

It will also be appreciated that certain compounds of the present invention may exist as geometrical isomers,

for example E and Z isomers of alkenes. The present invention includes any geometrical isomer of a compound of Formula I. It will further be understood that the present invention encompasses tautomers of the compounds of the formula 1.

It will also be understood that certain compounds of the present invention may exist in solvated, for example hydrated, as well as unsolvated forms. It will further be understood that the present-invention encompasses all such solvated forms of the compounds of the formula I.

Within the scope of the invention are also salts of the compounds of the formula I. Generally, pharmaceutically acceptable salts of compounds of the present invention may be obtained using standard procedures well known in the art, for example by reacting a sufficiently basic compound, for example an alkyl amine with a suitable acid, for example, HCl or acetic acid, to afford a physiologically acceptable anion. It may also be possible to make a corresponding alkali metal (such as sodium, potassium, or lithium) or an alkaline earth metal (such as a calcium) salt by treating a compound of the present invention having a suitably acidic proton, such as a carboxylic acid or a phenol with one equivalent of an alkali metal or alkaline earth metal hydroxide or alkoxide (such as the ethoxide or methoxide), or a suitably basic organic amine (such as choline or meglumine) in an aqueous medium, followed by conventional purification techniques.

In one embodiment, the compound of formula I above may be converted to a pharmaceutically acceptable salt or solvate thereof, particularly, an acid addition salt such as a hydrochloride, hydrobromide, phosphate, acetate, fumarate, maleate, tartrate, citrate, methanesulphonate or *p*-toluenesulphonate.

The novel compounds of the present invention are useful in therapy, especially for the treatment of various pain conditions such as chronic pain, neuropathic pain, acute pain, cancer pain, pain caused by rheumatoid arthritis, migraine, visceral pain etc. This list should however not be interpreted as exhaustive.

Compounds of the invention are useful as immunomodulators, especially for autoimmune diseases, such as arthritis, for skin grafts, organ transplants and similar surgical needs, for collagen diseases, various allergies, for use as anti-tumour agents and anti viral agents.

Compounds of the invention are useful in disease states where degeneration or dysfunction of opioid receptors is present or implicated in that paradigm. This may involve the use of isotopically labelled versions of the compounds of the invention in diagnostic techniques and imaging applications such as positron emission tomography (PET).

Compounds of the invention are useful for the treatment of diarrhoea, depression, anxiety and stress-related disorders such as post-traumatic stress disorders, panic disorder, generalized anxiety disorder, social phobia, and obsessive compulsive disorder, urinary incontinence, premature ejaculation, various mental illnesses, cough, lung oedema, various gastro-intestinal disorders, e.g. constipation, functional gastrointestinal disorders such as Irritable Bowel Syndrome and Functional Dyspepsia, Parkinson's disease and other motor disorders, traumatic brain injury, stroke, cardioprotection following miocardial infarction, spinal injury and drug addiction, including the treatment of alcohol, nicotine, opioid and other drug abuse and for disorders of the sympathetic nervous system for example hypertension.

Compounds of the invention are useful as an analgesic agent for use during general anaesthesia and monitored anaesthesia care. Combinations of agents with different properties are often used to achieve a balance of effects needed to maintain the anaesthetic state (e.g. amnesia, analgesia, muscle relaxation and sedation). Included in this combination are inhaled anaesthetics, hypnotics, anxiolytics, neuromuscular blockers and opioids.

Also within the scope of the invention is the use of any of the compounds according to the formula I above, for the manufacture of a medicament for the treatment of any of the conditions discussed above.

Thus, the invention provides a compound of formula I, or pharmaceutically acceptable salt or solvate thereof, as hereinbefore defined for use in therapy.

In a further aspect, the present invention provides the use of a compound of formula I, or a pharmaceutically acceptable salt or solvate thereof, as hereinbefore defined in the manufacture of a medicament for use in therapy.

In the context of the present specification, the term "therapy" also includes "prophylaxis" unless there are specific indications to the contrary. The term "therapeutic" and "therapeutically" should be contrued accordingly. The term "therapy" within the context of the present invention further encompasses to administer an effective amount of a compound of the present invention, to mitigate either a pre-existing disease state, acute or chronic, or a recurring condition. This definition also encompasses prophylactic therapies for prevention of recurring conditions and continued therapy for chronic disorders.

The compounds of the present invention are useful in therapy, especially for the therapy of various pain conditions including, but not limited to: chronic pain, neuropathic pain, acute pain, back pain, cancer pain, and visceral pain.

In use for therapy in a warm-blooded animal such as a human, the compound of the invention may be administered in the form of a conventional pharmaceutical composition by any route including orally, intramuscularly, subcutaneously, topically, intranasally, intraperitoneally, intrathoracially, intravenously, epidurally, intrathecally, intracerebroventricularly and by injection into the joints.

In one embodiment of the invention, the route of administration may be orally, intravenously or intramuscularly.

The dosage will depend on the route of administration, the severity of the disease, age and weight of the patient and other factors normally considered by the attending physician, when determining the individual regimen and dosage

level at the most appropriate for a particular patient.

For preparing pharmaceutical compositions from the compounds of this invention, inert, pharmaceutically acceptable carriers can be either solid and liquid. Solid form preparations include powders, tablets, dispersible granules, capsules, cachets, and suppositories.

A solid carrier can be one or more substances, which may also act as diluents, flavoring agents, solubilizers, lubricants, suspending agents, binders, or table disintegrating agents; it can also be an encapsulating material.

In powders, the carrier is a finely divided solid, which is in a mixture with the finely divided compound of the invention, or the active component. In tablets, the active component is mixed with the carrier having the necessary binding properties in suitable proportions and compacted in the shape and size desired.

For preparing suppository compositions, a low-melting wax such as a mixture of fatty acid glycerides and cocoa butter is first melted and the active ingredient is dispersed therein by, for example, stirring. The molten homogeneous mixture in then poured into convenient sized moulds and allowed to cool and solidify.

Suitable carriers are magnesium carbonate, magnesium stearate, talc, lactose, sugar, pectin, dextrin, starch, tragacanth, methyl cellulose, sodium carboxymethyl cellulose, a low-melting wax, cocoa butter, and the like.

The term composition is also intended to include the formulation of the active component with encapsulating material as a carrier providing a capsule in which the active component (with or without other carriers) is surrounded by a carrier which is thus in association with it. Similarly, cachets are included.

Tablets, powders, cachets, and capsules can be used as solid dosage forms suitable for oral administration.

Liquid form compositions include solutions, suspensions, and emulsions. For example, sterile water or water propylene glycol solutions of the active compounds may be liquid preparations suitable for parenteral administration. Liquid compositions can also be formulated in solution in aqueous polyethylene glycol solution.

Aqueous solutions for oral administration can be prepared by dissolving the active component in water and adding suitable colorants, flavoring agents, stabilizers, and thickening agents as desired. Aqueous suspensions for oral use can be made by dispersing the finely divided active component in water together with a viscous material such as natural synthetic gums, resins, methyl cellulose, sodium carboxymethyl cellulose, and other suspending agents known to the pharmaceutical formulation art.

Depending on the mode of administration, the pharmaceutical composition will preferably include from 0.05% to 99%w (per cent by weight), more preferably from 0.10 to 50%w, of the compound of the invention, all percentages by weight being based on total composition.

A therapeutically effective amount for the practice of the present invention may be determined, by the use of known criteria including the age, weight and response of the individual patient, and interpreted within the context of the disease which is being treated or which is being prevented, by one of ordinary skills in the art.

Within the scope of the invention is the use of any compound of formula I as defined above for the manufacture of a medicament.

Also within the scope of the invention is the use of any compound of formula I for the manufacture of a medicament for the therapy of pain.

Additionally provided is the use of any compound according to Formula I for the manufacture of a medicament for the therapy of various pain conditions including, but not limited to: chronic pain, neuropathic pain, acute pain, back pain, cancer pain, and visceral pain.

A further aspect of the invention is a method for therapy of a subject suffering from any of the conditions discussed above, whereby an effective amount of a compound according to the formula I above, is administered to a patient in need of such therapy.

Additionally, there is provided a pharmaceutical composition comprising a compound of Formula I, or a pharmaceutically acceptable salt thereof, in association with a pharmaceutically acceptable carrier.

Particularly, there is provided a pharmaceutical composition comprising a compound of Formula I, or a pharmaceutically acceptable salt thereof, in association with a pharmaceutically acceptable carrier for therapy, more particularly for therapy of pain.

Further, there is provided a pharmaceutical composition comprising a compound of Formula I, or a pharmaceutically acceptable salt thereof, in association with a pharmaceutically acceptable carrier use in any of the conditions discussed above.

In a further aspect, the present invention provides a method of preparing a compound of formula I.

In one embodiment, the present invention provides a process for preparing a compound of formula III,

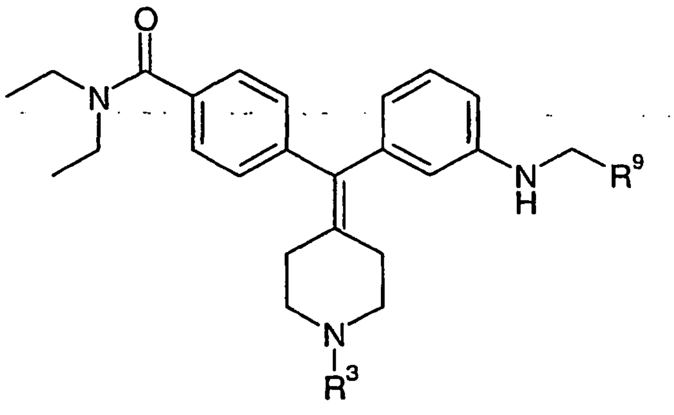

III

comprising:

reacting a compound of formula II,

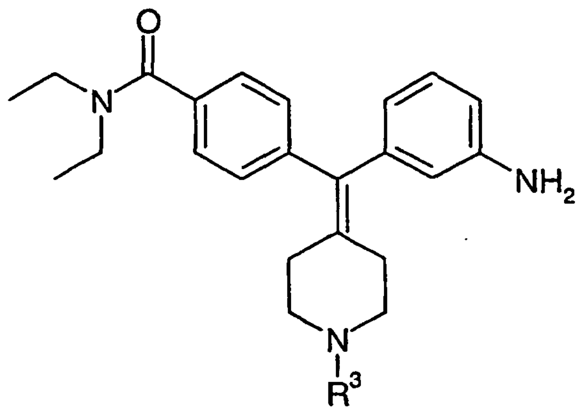

II

with $R^9$-CHO in the presence of a reducing agent to form the compound of formula III,

wherein

$R^9$ is selected from phenyl, pyridyl, thienyl, furyl, imidazolyl, triazolyl, pyrrolyl, thiazolyl, N-oxido-pyridyl, benzyl, pyridylmethyl, thienylmethyl, furylmethyl, imidazolylmethyl, triazolylmethyl, pyrrolylmethyl, thiazolylmethyl and N-oxido-pyridylmethyl, optionally substituted with one or more groups selected from $C_{1-4}$alkyl-$CF_3$, -OH, $C_{1-3}$alkoxy, phenoxy and halogen; and
$R^3$ is selected from $C_{1-6}$aklyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

In another embodiment, the present invention provides a process for preparing a compound of formula IV,

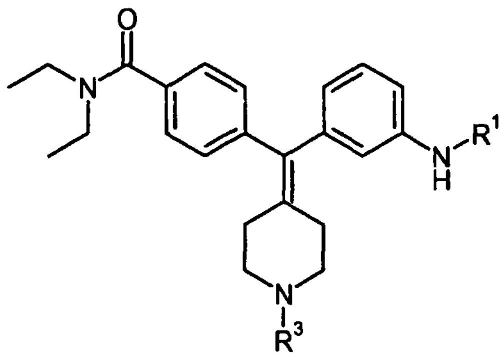

**IV**

comprising: reacting a compound of formula II,

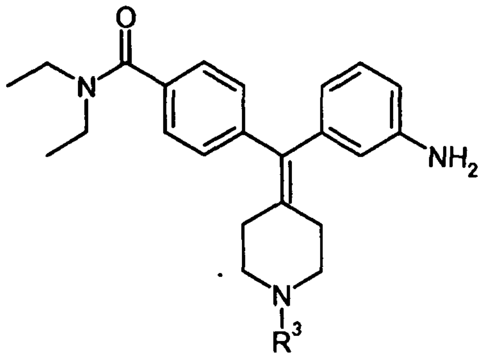

**II**

with $R^1$-X to form the compound of formula IV,
wherein

X is halogen;
$R^1$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl, wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

In another embodiment, the present invention provides a process for preparing a compound of formula I,

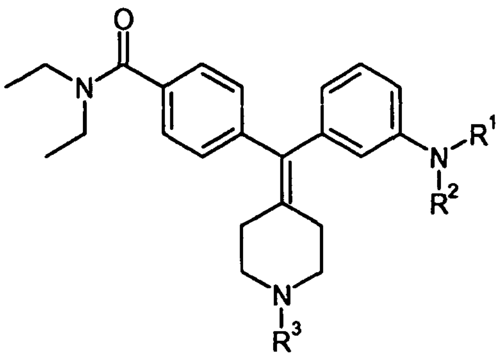

I

comprising: reacting a compound of formula V,

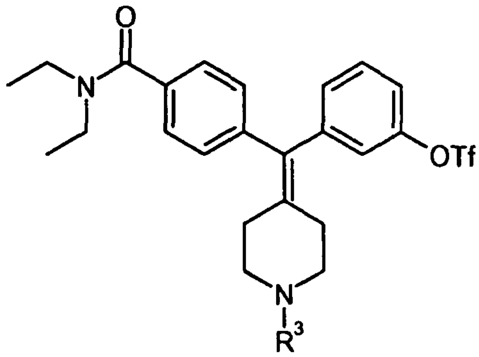

V

with $R^1R^2NH$ to form the compound of formula I,
wherein

$R^1$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl, wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen;
$R^2$ is selected from -H and $C_{1-6}$alkyl optionally substituted with one or more groups selected from $-CF_3$, -OH, $C_{1-3}$alkoxy, and halogen, or $R^1$ and $R^2$ are $C_{1-3}$alkylene that together form a portion of a ring; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

In another embodiment, the present invention provides a process for preparing a compound of formula VI,

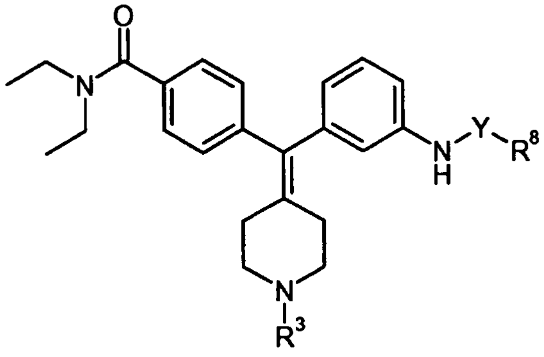

VI

comprising: reacting a compound of formula VII,

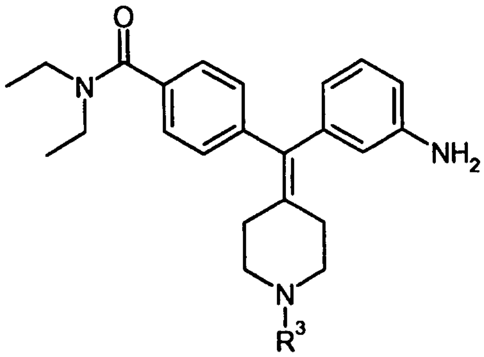

VII

with $R^8$-Y-X or $R^8$-Y-O-Y-$R^8$ to form the compound of formula VI:
wherein

X is halogen;
Y is selected from -C(=O)- and -$S(=O)_2$-;
$R^8$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl; wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl. $C_{3-10}$cycloalkyl, and $C_{3-10}$cycloalkyl-$C_{1-4}$alkyl are optionally substituted with $C_{1-4}$alkyl, -$CF_3$, -OH, $C_{1-3}$alkoxy, phenoxy, and halogen; and
$R^3$ is selected from $C_{1-6}$alkyl-O-C(=O)-, $C_{1-6}$alkyl, $C_{3-6}$cycloalkyl, and $C_{3-6}$cycloallcyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, -$NO_2$, -$CF_3$, $C_{1-6}$alkoxy and halogen.

In a further embodiment, the present invention provides a process for preparing a compound of formula VIII,

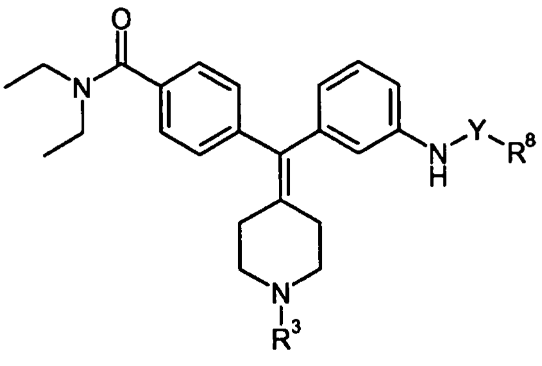

VIII

comprising: reacting a compound of formula VII,

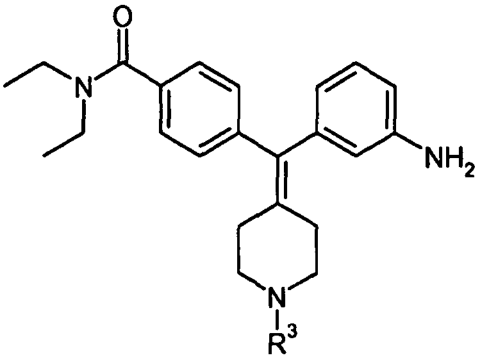

VII

with $R^8$-Z to form the compound of formula VIII:
wherein

Z is selected from -NCO and -NCS;
Y is selected from -C(=O)NH- and -C(=S)NH-;
$R^8$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl; wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl are optionally substituted with $C_{1-4}$alkyl, $-CF_3$, -OH, $C_{1-3}$alkoxy, phenoxy, and halogen; and
$R^3$ is selected from $C_{1-3}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-. $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

Particularly, the compounds of the present invention can be prepared according to the synthetic routes as exemplified in Schemes 1-14.

## Scheme 1

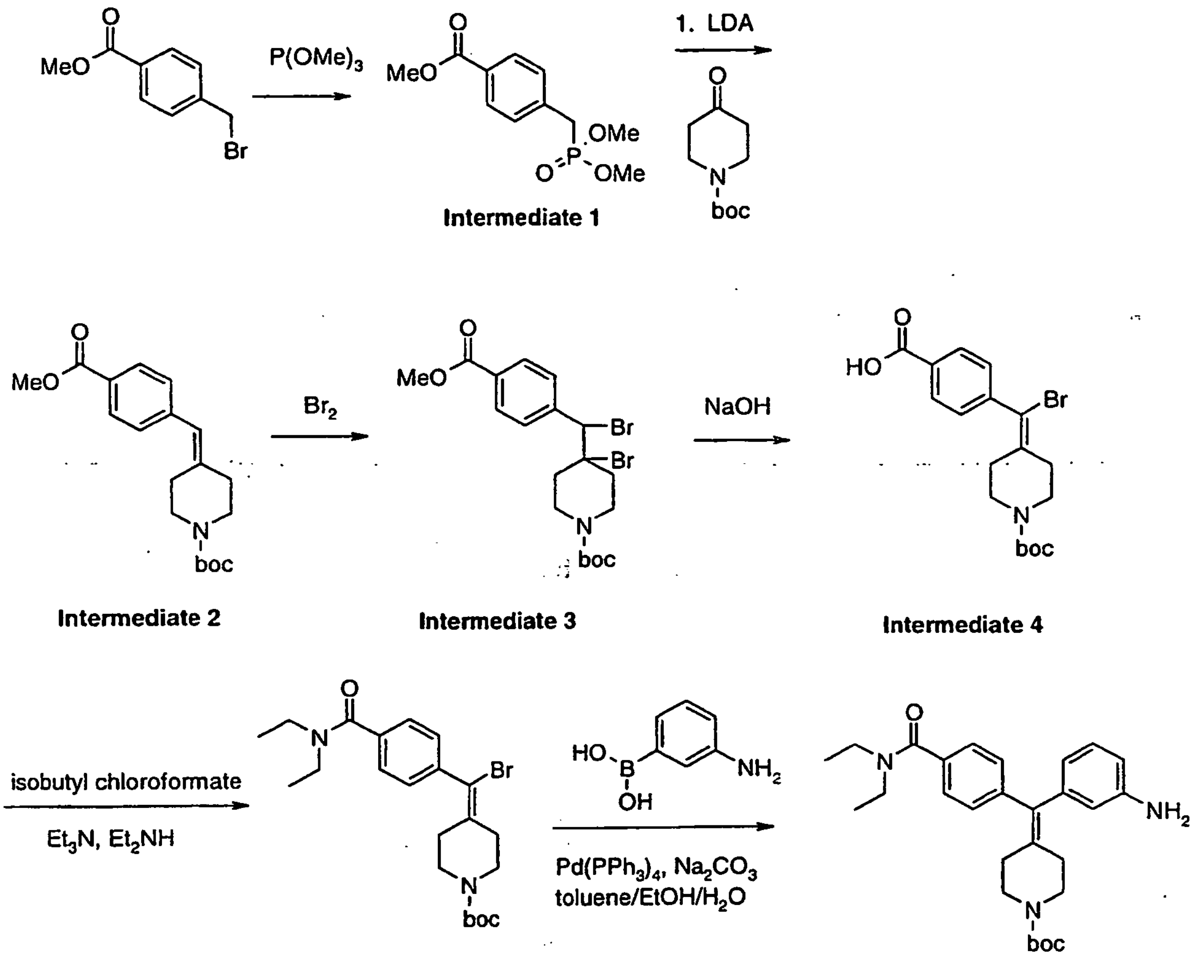


Intermediate 1

Intermediate 2

Intermediate 3

Intermediate 4

Intermediate 5

Intermediate 6

## Scheme 2

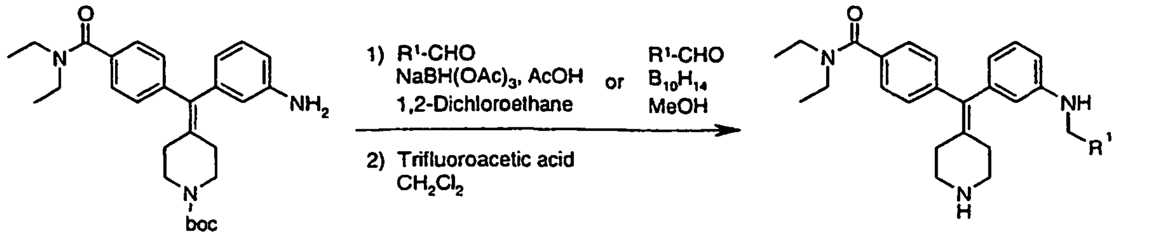


**Intermediate 6**

**Compound 1: $R^1$=phenyl**
**Compound 2: $R^1$=3-furyl**
**Compound 3: $R^1$=3-thienyl**
**Compound 4: $R^1$=benzyl**
**Compound 5: $R^1$=4-chlorophenyl**
**Compound 6: $R^1$=3-trifluoromethylphenyl**
**Compound 7: $R^1$=2-chlorophenyl**
**Compound 8: $R^1$=4-trifluoromethylphenyl**
**Compound 9: $R^1$=2-furyl**
**Compound 10: $R^1$=2-thienyl**
**Compound 11: $R^1$=cyclohexyl**
**Compound 12: $R^1$=ethyl**

## Scheme 3

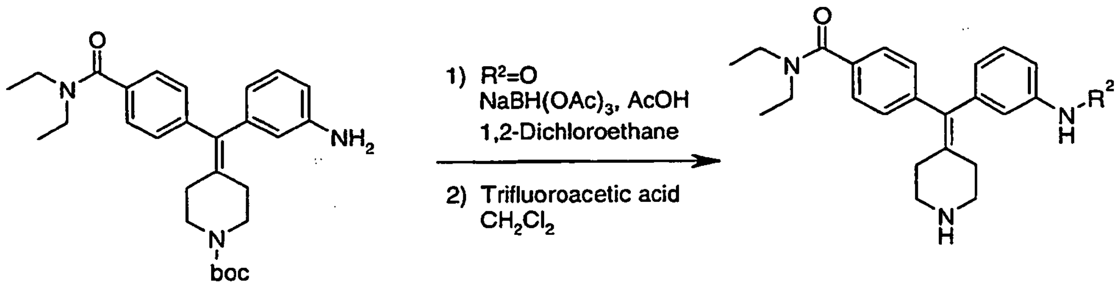


**Intermediate 6**

**Compound 13: $R^2$=cyclohexyl**
**Compound 14: $R^2$=cyclopentyl**
**Compound 15: $R^2$=cycloheptyl**

## Scheme 4

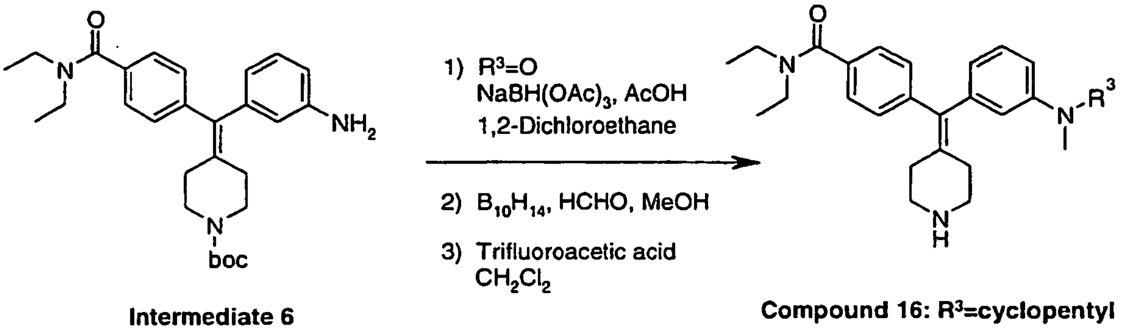


**Intermediate 6**

**Compound 16: $R^3$=cyclopentyl**

## Scheme 5

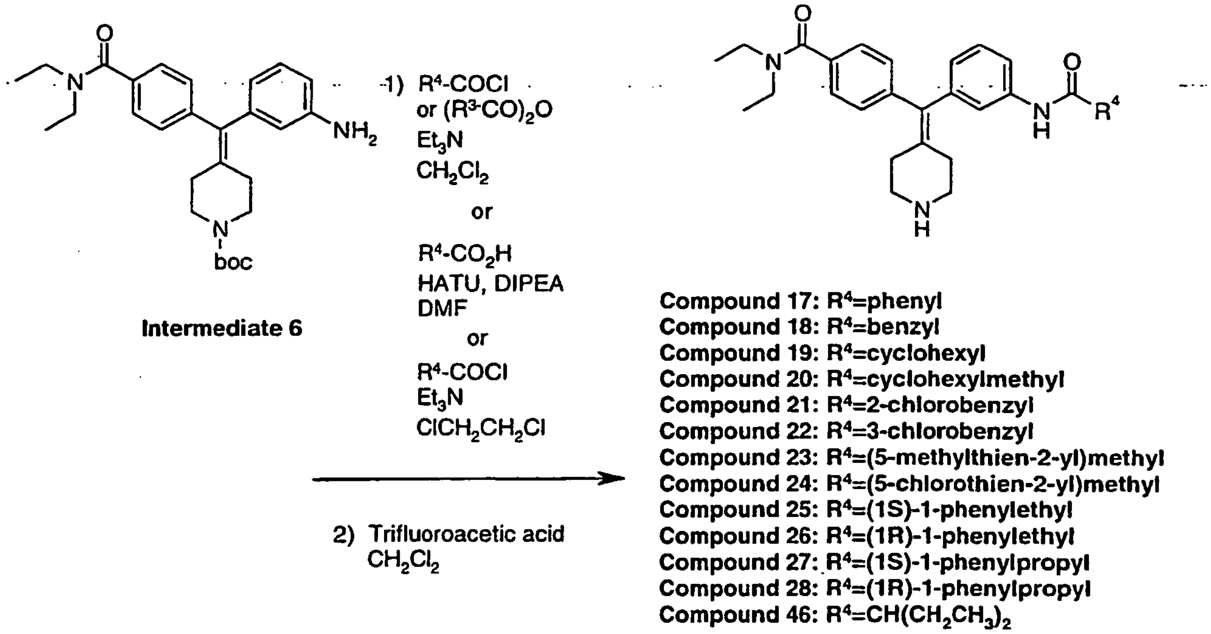


**Intermediate 6**

**Compound 17: $R^4$=phenyl**
**Compound 18: $R^4$=benzyl**
**Compound 19: $R^4$=cyclohexyl**
**Compound 20: $R^4$=cyclohexylmethyl**
**Compound 21: $R^4$=2-chlorobenzyl**
**Compound 22: $R^4$=3-chlorobenzyl**
**Compound 23: $R^4$=(5-methylthien-2-yl)methyl**
**Compound 24: $R^4$=(5-chlorothien-2-yl)methyl**
**Compound 25: $R^4$=(1S)-1-phenylethyl**
**Compound 26: $R^4$=(1R)-1-phenylethyl**
**Compound 27: $R^4$=(1S)-1-phenylpropyl**
**Compound 28: $R^4$=(1R)-1-phenylpropyl**
**Compound 46: $R^4$=$CH(CH_2CH_3)_2$**
**Compound 47: $R^4$=5-(1-methyl-1*H*-1,2,3-benzotriazole)**
**Compound 48: $R^4$=3-(6-chloropyridine)**
**Compound 49: $R^4$=2-methoxyphenyl**
**Compound 50: $R^4$=2-quinoxaline**
**Compound 51: $R^4$=2,5-difluorophenyl**
**Compound 52: $R^4$=2-(3-chlorothiophene)**
**Compound 53: $R^4$=3-methylphenyl**

## Scheme 6

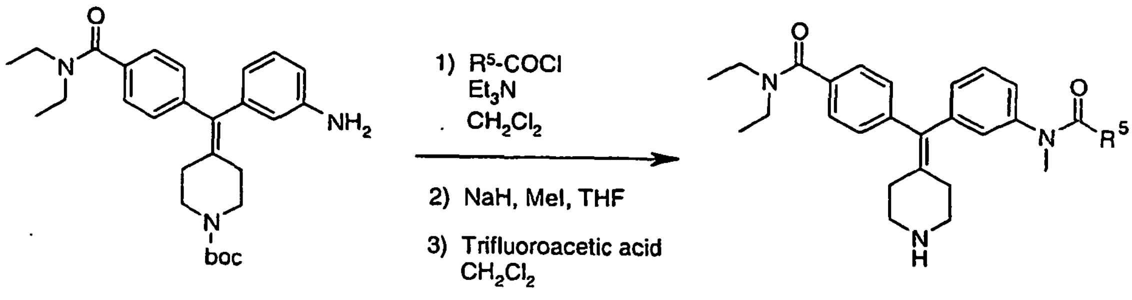


**Intermediate 6**

**Compound 29: $R^5$=phenyl**

## Scheme 7

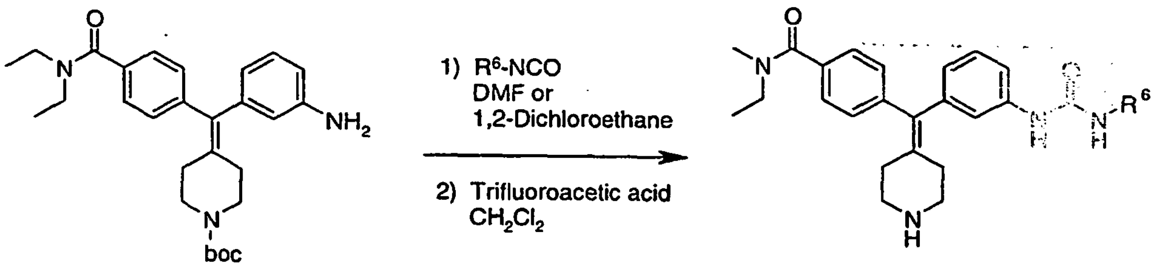


**Intermediate 6**

**Compound 30: $R^6$=phenyl**
**Compound 31: $R^6$=benzyl**

Scheme 8

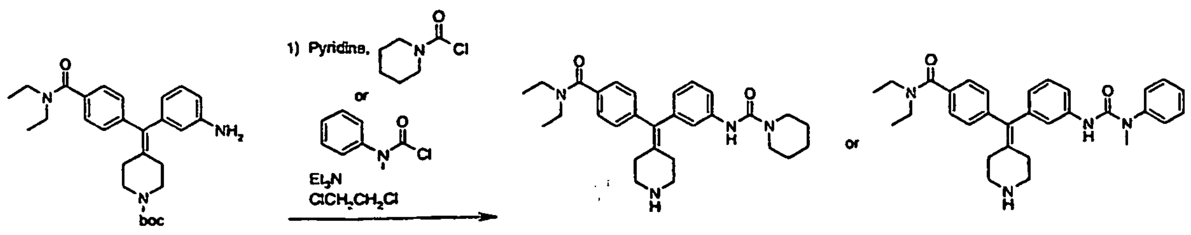


or

Intermediate 6

Compound 32

Compound 54

## Scheme 9

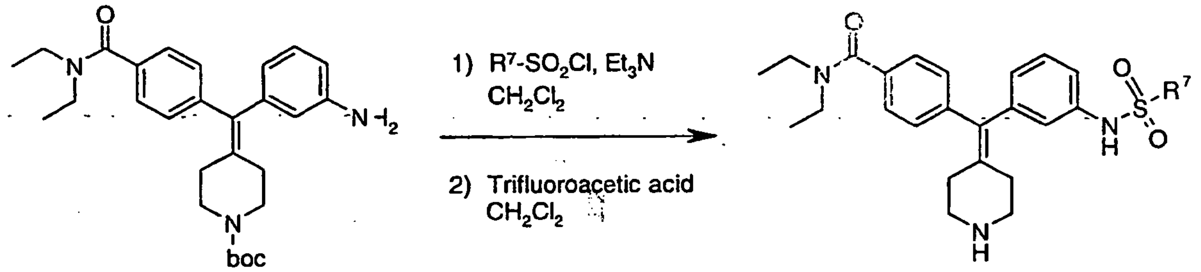


**Intermediate 6**

**Compound 33: $R^7$=phenyl**
**Compound 34: $R^7$=benzyl**

## Scheme 10

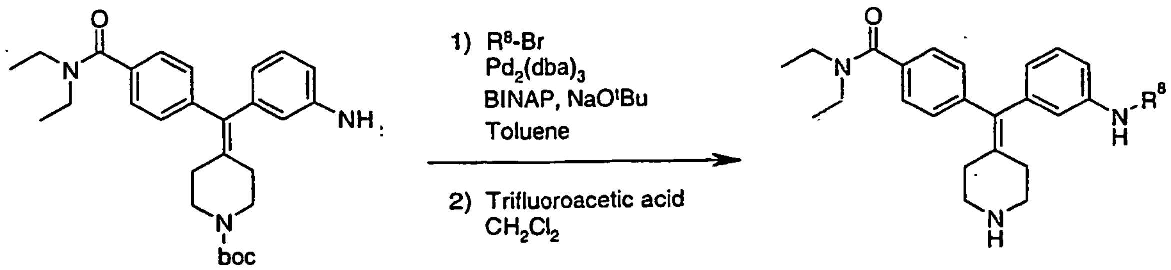


**Intermediate 6**

**Compound 35: $R^8$=phenyl**

## Scheme 11

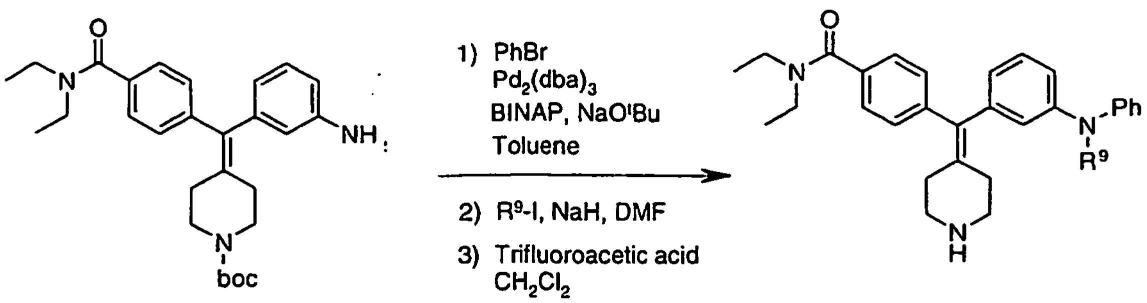


**Intermediate 6**

**Compound 36: $R^9$=methyl**
**Compound 37: $R^9$=ethyl**

Scheme 12

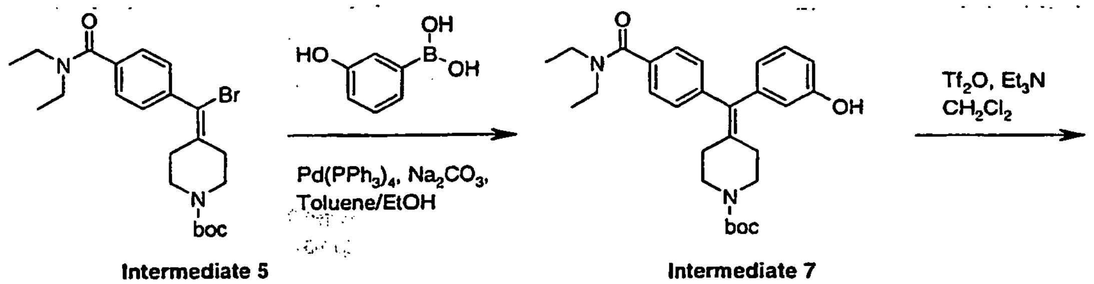


Intermediate 5

Intermediate 7

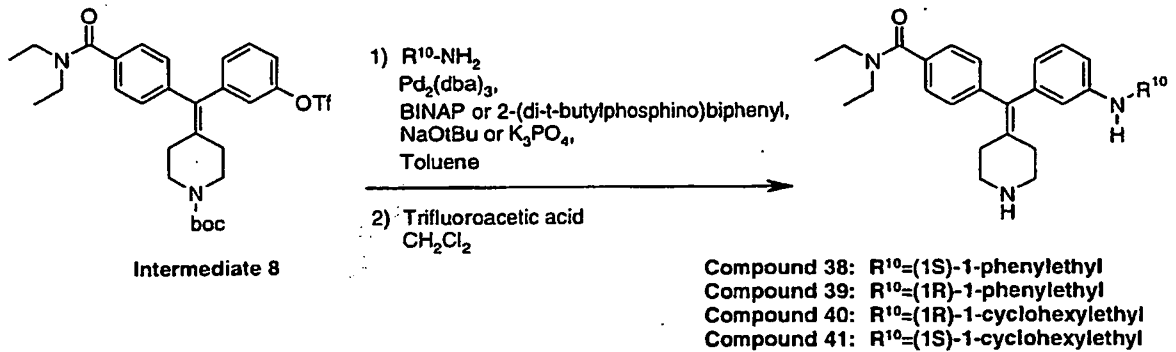


Intermediate 8

**Compound 38:** R$^{10}$=(1S)-1-phenylethyl
**Compound 39:** R$^{10}$=(1R)-1-phenylethyl
**Compound 40:** R$^{10}$=(1R)-1-cyclohexylethyl
**Compound 41:** R$^{10}$=(1S)-1-cyclohexylethyl
**Compound 42:** R$^{10}$=1-methyl-1-phenylethyl

Scheme 13

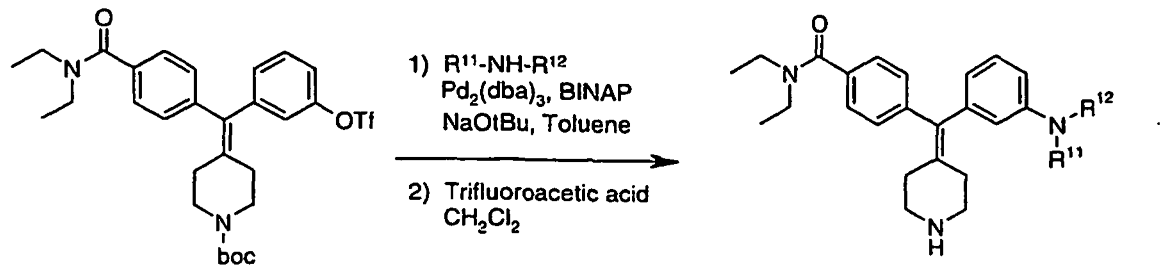


Intermediate 8

**Compound 43:** R$^{11}$=methyl, R$^{12}$=cyclohexyl

Scheme 14

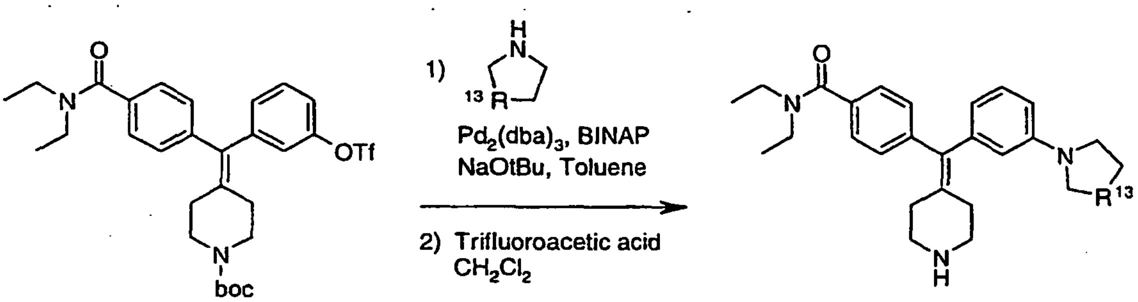


Intermediate 8

Compound 44: $R^{13}$=$CH_2CH_2$
Compound 45: $R^{14}$=$CH_2$

## BIOLOGICAL EVALUATION

The compounds of the invention are found to be active towards δ receptors in warm-blooded animal, e.g., human. Particularly the compounds of the invention are found to be effective δ receptor ligands. *In vitro* assays, *infra*, demonstrate these surprising activities, especially with regard to agonists potency and efficacy as demonstrated in the rat brain functional assay and/or the human δ receptor functional assay (low). This feature may be related to in vivo activity and may not be linearly correlated with binding affinity. In these *in vitro* assays, a compound is tested for their activity toward δ receptors and $IC_{50}$ is obtained to determine the selective activity for a particular compound towards δ receptors. In the current context, $IC_{50}$ generally refers to the concentration of the compound at which 50% displacement of a standard radioactive δ receptor ligand has been observed.

The activities of the compound towards κ and μ receptors are also measured in a similar assay.

In vitro model

Cell culture

Human 293S cells expressing cloned human κ, δ and μ receptors and neomycin resistance are grown in suspension at 37°C and 5% $CO_2$ in shaker flasks containing calcium-free DMEM10% FBS, 5% BCS, 0.1% Pluronic F-68, and 600 μg/ml geneticin.

Rat brains are weighed and rinsed in ice-cold PBS (containing 2.5mM EDTA, pH 7.4). The brains are homogenized with a polytron for 30 sec (rat) in ice-cold lysis buffer (50mM Tris, pH 7.0,2.5mM EDTA, with phenylmethylsulfonyl fluoride added just prior use to 0.5MmM from a 0.5M stock in DMSO:ethanol).

Membrane preparation

Cells are pelleted and resuspended in lysis buffer (50 mM Tris, pH 7.0, 2.5 mM EDTA, with PMSF added just prior to use to 0.1 mM from a 0.1 M stock in ethanol), incubated on ice for 15. min, then homogenized with a polytron for 30 sec. The suspension is spun at 1000g (max) for 10 min at 4°C. The supernatant is saved on ice and the pellets resuspended and spun as before. The supernatants from both spins are combined and spun at 46,000 g(max) for 30 min. The pellets are resuspended in cold Tris buffer (50 mM Tris/Cl, pH 7.0) and spun again. The final pellets are resuspended in membrane buffer (50 mM Tris, 0.32 M sucrose, pH 7.0). Aliquots (1 ml) in polypropylene tubes are frozen in dry ice/ethanol and stored at -70°C until use. The protein concentrations are determined by a modified Lowry assay with sodium dodecyl sulfate.

Binding assays

Membranes are thawed at 37°C, cooled on ice, passed 3 times through a 25-gauge needle, and diluted into binding buffer (50 mM Tris, 3 mM $MgCl_2$, 1 mg/ml BSA (Sigma A-7888), pH 7.4, which is stored at 4°C after filtration

through a 0.22 m filter, and to which has been freshly added 5 μg/ml aprotinin, 10 μM bestatin, 10 μM diprotin A, no DTT). Aliquots of 100 μl are added to iced 12x75 mm polypropylene tubes containing 100 μl of the appropriate radioligand and 100 μl of test compound at various concentrations. Total (TB) and nonspecific (NS) binding are determined in the absence and presence of 10 μM naloxone respectively. The tubes are vortexed and incubated at 25°C for 60-75 min, after which time the contents are rapidly vacuum-filtered and washed with about 12 ml/tube iced wash buffer (50 mM Tris, pH 7.0, 3 mM $MgCl_2$) through GF/B filters (Whatman) presoaked for at least 2h in 0:1 % polyethyleneimine. The radioactivity (dpm) retained on the filters is measured with a beta counter after soaking the filters for at least 12h in minivials containing 6-7 ml scintillation fluid. If the assay is set up in 96-place deep well plates, the filtration is over 96-place PEI-soaked unifilters, which are washed with 3 x 1 ml wash buffer, and dried in an oven at 55°C for 2h. The filter plates are counted in a TopCbunt (Packard) after adding 50 μl MS-20 scintillation fluid/well.

Functional Assays

The agonist activity of the compounds is measured by determining the degree to which the compounds receptor complex activates the binding of GTP to G-proteins to which the receptors are coupled. In the GTP binding assay, GTP $[\gamma]^{35}S$ is combined with test compounds and membranes from HEK-293S cells expressing the cloned human opioid receptors or from homogenised rat and mouse brain. Agonists stimulate GTP$[\gamma]^{35}$S binding in these membranes. The $EC_{50}$ and $E_{max}$ values of compounds are determined from dose-response curves. Right shifts of the dose response curve by the delta antagonist naltrindole are performed to verify that agonist activity is mediated through delta receptors. For human δ receptor functional assays, $EC_{50}$ (low) is measured when the human δ receptors used in the assay were expressed at lower levels in comparison with those used in determining $EC_{50}$ (high). The $E_{max}$ values were determined in relation to the standard δ agonist SNC80, i.e., higher than 100% is a compound that have better efficacy than SNC80.

Procedure for rat brain GTP

Rat brain membranes are thawed at 37°C, passed 3 times through a 25-gauge blunt-end needle and diluted in the GTPγS binding (50 mM Hepes, 20 mM NaOH, 100 mM NaCl, 1 mM EDTA, 5 mM $MgCl_2$, pH 7.4, Add fresh: 1 mM DTT, 0.1 % BSA). 120μM GDP final is added membranes dilutions. The EC50 and Emax of compounds are evaluated from 10-point dose-response curves done in 300μl with the appropriate amount of membrane protein (20μg/well) and 100000-130000 dpm of GTP$\gamma^{35}$S per well (0.11 -0.14nM). The basal and maximal stimulated binding are determined in absence and presence of 3 μM SNC-80

Data analysis

The specific binding (SB) was calculated as TB-NS, and the SB in the presence of various test compounds was expressed as percentage of control SB. Values of $IC_{50}$ and Hill coefficient ($n_H$) for ligands in displacing specifically bound radioligand were calculated from logit plots or curve fitting programs such as Ligand, GraphPad Prism, SigmaPlot, or ReceptorFit. Values of $K_i$ were calculated from the Cheng-Prussoff equation. Mean ± S.E.M. values of $IC_{50}$, $K_i$ and $n_H$ were reported for ligands tested in at least three displacement curves.

Based on the above testing protocols, we find that the compounds of the present invention and some of the intermediates used in the preparation thereof are active toward human δ receptors. Generally, the $IC_{50}$ towards human δ receptor for most compounds of the present invention is in the range of 0.14 nM - 31.2 nM. The $EC_{50}$ and $\%E_{max}$ towards human δ receptor for these compounds are generally in the range of 2.11 nM -390 nM and 89 - 118, respectively. The $IC_{50}$ towards human κ and μ receptors for the compounds of the invention is generally in the ranges of 36 nM-9680 nM and 3 nM - 5975 nM, respectively.

Receptor Saturation Experiments

Radioligand $K_\delta$ values are determined by performing the binding assays on cell membranes with the appropriate radioligands at concentrations ranging from 0.2 to 5 times the estimated $K_\delta$ (up to 10 times if amounts of radioligand required are feasible). The specific radioligand binding is expressed as pmole/mg membrane protein. Values of $K_\delta$ and $B_{max}$ from individual experiments are obtained from nonlinear fits of specifically bound (B) vs. nM free (F) radioligand from individual according to a one-site model.

Determination Of Mechano-Allodynia Using Von Frey Testing

Testing is performed between 08:00 and 16:00h using the method described by Chaplan et al. (1994). Rats are placed in Plexiglas cages on top of a wire mesh bottom which allows access to the paw, and are left to habituate

for 10-15 min. The area tested is the mid-plantar left hind paw, avoiding the less sensitive foot pads. The paw is touched with a series of 8 Von Frey hairs with logarithmically incremental stiffness (0.41, 0.69, 1.20, 2.04, 3.63, 5.50, 8.51, and 15.14 grams; Stoelting, III, USA). The von Frey hair is applied from underneath the mesh floor perpendicular to the plantar surface with sufficient force to cause a slight buckling against the paw, and held for approximately 6-8 seconds. A positive response is noted if the paw is sharply withdrawn. Flinching immediately upon removal of the hair is also considered a positive response. Ambulation is considered an ambiguous response, and in such cases the stimulus is repeated.

Testing Protocol

The animals are tested on postoperative day 1 for the FCA-treated group. The 50% withdrawal threshold is determined using the up-down method of Dixon (1980). Testing is started with the 2.04 g hair, in the middle of the series. Stimuli are always presented in a consecutive way, whether ascending or descending. In the absence of a paw withdrawal response to the initially selected hair, a stronger stimulus is presented; in the event of paw withdrawal, the next weaker stimulus is chosen. Optimal threshold calculation by this method requires 6 responses in the immediate vicinity of the 50% threshold, and counting of these 6 responses begins when the first change in response occurs, e.g. the threshold is first crossed. In cases where thresholds fall outside the range of stimuli, values of 15.14 (normal sensitivity) or 0.41 (maximally allodynic) are respectively assigned. The resulting pattern of positive and negative responses is tabulated using the convention, X = no withdrawal; O = withdrawal, and the 50% withdrawal threshold is interpolated using the formula:

$$50\%\ \text{g threshold} = 10^{(Xf + k\delta)} / 10{,}000$$

where Xf = value of the last von Frey hair used (log units); k = tabular value (from Chaplan et al. (1994)) for the pattern of positive / negative responses; and δ = mean difference between stimuli (log units). Here δ = 0.224.

Von Frey thresholds are converted to percent of maximum possible effect (% MPE), according to Chaplan et al. 1994. The following equation is used to compute % MPE:

$$\%\ \text{MPE} = \frac{\text{Drug treated threshold (g) - allodynia threshold (g)}}{\text{Control threshold (g) - allodynia threshold (g)}} \times 100$$

Administration Of Test Substance

Rats are injected (subcutaneously, intraperitoneally, intravenously or orally) with a test substance prior to von Frey testing, the time between administration of test compound and the von Frey test varies depending upon the nature of the test compound.

Writhing Test

Acetic acid will bring abdominal contractions when administered intraperitoneally in mice. These will then extend their body in a typical pattern. When analgesic drugs are administered, this described movement is less frequently observed and the drug selected as a potential good candidate.

A complete and typical Writhing reflex is considered only when the following elements are present: the animal is not in movement; the lower back is slightly depressed; the plantar aspect of *both* paws is observable. In this assay, compounds of the present invention demonstrate significant inhibition of writhing responses after oral dosing of 1-100 μmol/kg.

(i) Solutions preparation

Acetic acid (AcOH): 120 μL of Acetic Acid is added to 19.88 ml of distilled water in order to obtain a final volume of 20 ml with a final concentration of 0.6% AcOH. The solution is then mixed (vortex) and ready for injection.

Compound (drug): Each compound is prepared and dissolved in the most suitable vehicle according to standard procedures.

(ii) Solutions administration

The compound (drug) is administered orally, intraperitoneally (i.p.), subcutaneously (s.c.) or intravenously (i.v.)) at 10 ml/kg (considering the average mice body weight) 20, 30 or 40 minutes (according to the class of compound and its characteristics) prior to testing. When the compound is delivered centrally: Intraventricularly (i.c.v.) or intrathecally (i.t.) a volume of 5 μL is administered.

The AcOH is administered intraperitoneally (i.p.) in two sites at 10 ml/kg. (considering the average mice body weight) immediately prior to testing.

(iii) Testing

The animal (mouse) is observed for a period of 20 minutes and the number of occasions (Writhing reflex) noted and compiled at the end of the experiment. Mice are kept in individual "shoe box" cages with contact bedding. A total of 4 mice are usually observed at the same time: one control and three doses of drug.

For the anxiety and anxiety-like indications, efficacy has been established in the geller-seifter conflict test in the rat.

For the functional gastrointestina disorder indication, efficacy can be established in the assay described by Coutinho SV *et al,* in American Journal of Physiology - Gastrointestinal & Liver Physiology. 282(2):G307-16, 2002 Feb, in the rat.

ADDITIONAL IN VIVO TESTING PROTOCOLS

Subjects and housing

Naïve male Sprague Dawley rats (175-200g) are housed in groups of 5 in a temperature controlled room (22°C, 40-70% humidity, 12-h light/dark). Experiments are performed during the light phase of the cycle. Animals have food and water ad libitum and are sacrificed immediately after data acquisition.

Sample

Compound (Drug) testing includes groups of rats that do not receive any treatment and others that are treated with E. coli lipopolysaccharide(LPS). For the LPS-treated experiment, four groups are injected with LPS, one of the four groups is then vehicle-treated whilst the other three groups are injected with the drug and its vehicle. A second set of experiments are conducted involving five groups of rats; all of which receive no LPS treatment. The naïve group receives no compound (drug) or vehicle; the other four groups are treated with vehicle with or without drug. These are performed to determine anxiolytic or sedative effects of drugs which can contribute to a reduction in USV.

Administration of LPS

Rats are allowed to habituate in the experimental laboratory for 15-20 min prior to treatment. Inflammation is induced by administration of LPS (endotoxin of gram-negative E. coli bacteria serotype 0111:B4, Sigma). LPS (2.4μg) is injected intracerebro-ventricularly (i.c.v.), in a volume of 10μl, using standard stereotaxic surgical techniques under isoflurane anaesthesia. The skin between the ears is pushed rostrally and a longitudinal incision of about 1cm is made to expose the skull surface. The puncture site is determined by the coordinates: 0.8 mm posterior to the bregma, 1.5 mm lateral (left) to the lambda (sagittal suture), and 5 mm below the surface of the skull (vertical) in the lateral ventricle. LPS is injected via a sterile stainless steel needle (26-G 3/8) of 5 mm long attached to a 100-μl Hamilton syringe by polyethylene tubing (PE20; 10-15 cm). A 4 mm stopper made from a cut needle (20-G) is placed over and secured to the 26-G needle by silicone glue to create the desired 5mm depth.

Following the injection of LPS, the needle remains in place for an additional 10 s to allow diffusion of the compound, then is removed. The incision is closed, and the rat is returned to its original cage and allowed to rest for a minimum of 3.5h prior to testing.

Experimental setup for air-puff stimulation

The rats remains in the experimental laboratory following LPS injection and compound (drug) administration. At the time of testing all rats are removed and placed outside the laboratory. One rat at a time is brought into the testing laboratory and placed in a clear box (9 × 9 × 18 cm) which is then placed in a sound-attenuating ventilated cubicle measuring 62(w) ×35(d) ×46(h) cm (BRS/LVE, Div. Tech-Serv Inc). The delivery of air-puffs, through an air output

nozzle of 0.32 cm, is controlled by a system (AirStim, San Diego Intruments) capable of delivering puffs of air of fixed duration (0.2 s) and fixed intensity with a frequency of 1 puff per 10s. A maximun of 10 puffs are administered, or until vocalisation starts, which ever comes first. The first air puff marks the start of recording.

Experimental setup for and ultrasound recording

The vocalisations are recorded for 10 minutes using microphones (G.R.A.S. sound and vibrations, Vedbaek, Denmark) placed inside each cubicle and controlled by LMS (LMS CADA-X 3.5B, Data Acquisition Monitor, Troy, Michigan) software. The frequencies between 0 and 32000Hz are recorded, saved and analysed by the same software (LMS CADA-X 3.5B, Time Data Processing Monitor and UPA (User Programming and Analysis)).

Compounds (Drugs)

All compounds (drugs) are pH-adjusted between 6.5 and 7.5 and administered at a volume of 4 ml/kg. Following compound (drug) administration, animals are returned to their original cages until time of testing.

Analysis

The recording is run through a series of statistical and Fourier analyses to filter (between 20-24kHz) and to calculate the parameters of interest. The data are expressed as the mean $\pm$ SEM. Statistical significance is assessed using T-test for comparison between naive and LPS-treated rats, and one way ANOVA followed by Dunnett's multiple comparison test (post-hoc) for drug effectiveness. A difference between groups is considered significant with a minimum p value of $\leq 0.05$. Experiments are repeated a minimum of two times.

EXAMPLES

The invention will further be described in more detail by the following Examples which describe methods whereby compounds of the present invention may be prepared, purified, analyzed and biologically tested, and which are not to be construed as limiting the invention.

**INTERMEDIATE 1**

A mixture of 4-(bromomethyl)benzoic acid, methyl ester (11.2 g, 49 mmol) and trimethyl phosphite (25 mL) was refluxed under $N_2$ for 5 hrs. Excess trimethyl phosphite was removed by co-distillation with toluene to give INTERMEDIATE 1 in quantitative yield. $^1$H NMR ($CDCl_3$) $\delta$ 3.20 (d, 2H, J=22 Hz, $CH_2$), 3.68 (d, 3H 10.8 Hz, $OCH_3$), 3.78 (d, 3H, 11.2 Hz, $OCH_3$), 3.91 (s, 3H, $OCH_3$), 7.38 (m, 2H, Ar-H), 8.00 (d, 2H, J=8 Hz, Ar-H).

**INTERMEDIATE 2: 4-(4-Methoxycarbonyl-benzylidene)-piperidine-1-carboxylic acid *tert*-butyl ester**

To a solution of INTERMEDIATE 1 in dry THF (200 mL) was added dropwise lithium diisopropylamide (32.7 mL 1.5 M in hexanes, 49 mmol) at -78 °C. The reaction mixture was then allowed to warm to room temperature prior to addition of *N-tert*-butoxycarbonyl-4-piperidone (9.76 g, 49 mmol in 100 mL dry THF). After 12 hrs, the reaction mixture was quenched with water (300 mL) and extracted with ethyl acetate (3 x 300 mL). The combined organic phases were dried over $MgSO_4$ and evaporated to give a crude product, which was purified by flash chromatography to provide INTERMEDIATE 2 as a white solid (5.64 g, 35%). IR (NaCl) 3424, 2974, 2855, 1718, 1 688, 1606, 1427, 1362, 1276 cm$^{-1}$; $^1$H NMR ($CDCl_3$) $\delta$ 1.44 (s, 9H), 2.31 (t, J=5.5 Hz, 2H), 2.42 (t, J=5.5 Hz, 2H), 3.37 (t, J=5.5 Hz, 2H), 3.48 (t, J=5.5 Hz, 2H), 3.87 (s, 3H, $OCH_3$), 6.33 (s, 1H, CH), 7.20 (d J=6.7 Hz, 2H, Ar-H), 7.94 (d, J,=6.7 Hz, 2H, Ar-H); $^{13}$C NMR ($CDCl_3$) $\delta$ 28.3, 29.2, 36.19, 51.9, 123.7, 127.8, 128.7, 129.4, 140.5, 142.1, 154.6, 166.8.

**INTERMEDIATE 3: 4-Bromo-4-[bromo-(4-methoxycarbonyl-phenyl)-methyl]-piperidine-1-carboxylic acid tert-butyl ester**

To a mixture of INTERMEDIATE 2 (5.2 g, 16 mmol) and $K_2CO_3$ (1.0 g) in dry dichloromethane (200 mL) was added a solution of bromine (2.9 g, 18 mmol) in 30 mL $CH_2C1_2$ at 0 °C. after 1.5 hrs at room temperature, the solution after filtration of $K_2CO_3$ was condensed. The residue was then dissolved in ethyl acetate (200 mL), washed with water (200 mL), 0.5 M HC1 (200 mL) and brine (200 mL), and dried over $MgSO_4$. Removal of solvents provided a crude product, which was recrystallized from methanol to give INTERMEDIATE 3 as a white solid (6.07 g, 78%). IR (NaCl) 3425, 2969, 1725, 1669, 1426, 1365, 1279, 1243 cm$^{-1}$; $^1$H NMR ($CDCl_3$) $\delta$ 1.28 (s, 9H), 1.75 (m, 1H), 1.90 (m, 1H), 2.1

(m, 2H), 3.08 (br, 2H), 3.90 (s, 3H, $OCH_3$), 4.08 (br, 3H), 7.57 (d, J=8.4 Hz, 2H, Ar-H) 7.98 (d, J=8.4 Hz, 2H, Ar-H); $^{13}C$ NMR ($CDCl_3$) δ 28.3, 36.6, 38.3, 40.3, 52.1, 63.2, 72.9, 129.0, 130.3, 130.4, 141.9, 154.4, 166.3.

**INTERMEDIATE 4: 4-[bromo-(4-carboxy-phenyl)-methylene]-piperidine-1-carboxylic acid tert-butyl ester**

A solution of INTERMEDIATE 3 (5.4 g 11 mmol) in methanol (300 mL) and 2.0 M NaOH (100 mL) was heated at 40 °C for 3 hrs. The solid was collected by filtration, and dried overnight under vacuum. The dry salt was dissolved in 40% acetonitrile/water, and was adjusted to pH 2 using concentrated HCl. INTERMEDIATE 4 (3.8 g, 87%) was isolated as a white powder by filtration. $^{1}H$ NMR ($CDCl_3$) δ 1.45 (s, 9H, $^{t}Bu$), 2.22 (dd, J=5.5 Hz, 6.1 Hz, 2H), 2.64 (dd, J=5.5 Hz, 6.1 Hz, 2H), 3.34 (dd, J=5.5 Hz, 6.1 Hz, 2H), 3.54 (dd, J=5.5 Hz, 6.1 Hz, 2H), 7.35 (d, J=6.7 Hz, 2H, Ar-H), 8.08 (d, J=6.7 Hz, 2H, Ar-H); $^{13}C$ NMR ($CDCl_3$) δ 28.3, 31.5, 34.2, 44.0, 115.3, 128.7, 129.4, 130.2, 137.7, 145.2, 154.6, 170.3.

**INTERMEDIATE 5: 4-[bromo-(4-diethylcarbamoyl-phenyl)-methylene]-piperidine-1-carboxylic acid tert-butyl ester**

To a solution of INTERMEDIATE 4 (1.0 g, 2.5 mmol) in dry dichloromethane (10 mL) at - 20 °C was added isobutylchloroformate (450 mg, 3.3 mmol). After 20 min at -20 °C diethylamine (4 mL) was added and the reaction was allowed to warm to room temperature. After 1.5 hrs the solvents were evaporated and the residue was partitioned between ethyl acetate and water. The organic phase was washed with brine and dried over $MgSO_4$. Removal of solvents provided a crude product, which was purified by flash chromatography to give INTERMEDIATE 5 as white needles (800 mg, 73%). IR (NaCl) 3051, 2975, 1694, 1633, 1416, 1281, 1168, 1115 $cm^{-1}$; $^{1}H$ NMR ($CDCl_3$) δ 1.13 (br, 3H, $CH_3$), 1.22 (br, 3H, $CH_3$), 1.44 (s, 9H, $^{1}Bu$), 2.22 (t, J=5.5 Hz, 2H), 2.62 (t, J=5.5 Hz, 2H), 3.33 (m, 4H), 3.55 (m, 4H), 7.31 (d, J=8.0 Hz, 2H, Ar-H), 7.36 (d, J=8.0 Hz, 2H, Ar-H);$^{13}C$ NMR ($CDCl_3$) δ 12.71, 14.13, 28.3, 31.5, 34.2, 39.1, 43.2, 79.7, 115.9, 126.3, 129.3, 136.8, 137.1, 140.6, 154.6, 170.5.

**INTERMEDIATE 6: N,N-diethyl-4-(3-aminophenyl-piperidin-4-ylidene-methyl)-benzamide**

To a flask containing INTERMEDIATE 5 (5.94 g, 13.2 mmol) was added toluene (130 mL), ethanol (25 mL), 2.0 M sodium carbonate (16 mL, 32.4 mmol) and 3-aminophenylboronic acid (3.09 g, 19.9 mmol). The solution was degassed for 20 minutes, and then palladium tetrakistriphenylphosphine (1.53 g, 1.32 mmol) was added. The reaction mixture was heated at 90 °C overnight under $N_2$. The reaction was concentrated and the residue was diluted with ethyl acetate. The solution was washed with two portions of brine and the organic layer was dried ($Na_2SO_4$), filtered, and concentrated. The residue was purified by flash chromatography, eluting 3% methanol in dichloromethane, to yield INTERMEDIATE 6 as a colourless solid (6.12 g, 97%). '(400MHz, $CDCl_3$) δ 1.08-1.18 (m, 3H), 1.18-1.28 (m, 3H), 2.27-2.36 (m, 4H), 3.23-3.34 (m, 2H), 3.40-3.48 (m, 2H), 3.49-3.58 (m, 2H), 3.60-3.66 (m, 2H), 6.38-6.41 (m, 1H), 6.50-6.59 (m, 2H), 7.08 (t, J = 7.60 Hz, 1H), 7.14 (d, J = 8.32 Hz, 2H), 7.30 (d, J = 8.17 Hz, 2H).

**COMPOUND 1: 4-[[3-(benzylamino)phenyl](piperidin-4-ylidene)methyl]-*N,N*-diethylbenzamide**

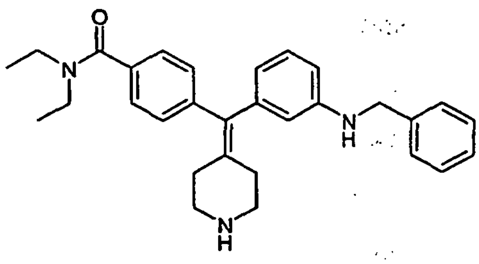

To a solution of INTERMEDIATE 6 (150 mg, 0.324 mmol) in 1,2-dichloroethane (10 mL) was added benzaldehyde (36 μL, 0.356 mmol) and sodium triacetoxyborohydride (75 mg, 0.356 mmol). The reaction was stirred at room temperature under nitrogen. After 18 hours the reaction was diluted with dichloromethane and washed with saturated aqueous sodium bicarbonate. The aqueous layer was extracted with two portions of dichloromethane and the combined organic extracts were dried over anhydrous sodium sulfate, filtered and concentrated. The residue was dissolved in dichloromethane (10 mL) and trifluoroacetic acid (1 mL) was added. After 18 hours the solution was diluted with dichloromethane and washed with saturated aqueous sodium bicarbonate. The aqueous layer was extracted with two portions of dichloromethane and the combined organic extracts were dried over anhydrous sodium sulfate, filtered and concentrated. The residue was purified by reverse phase chromatography, eluting 5% to 50% acetonitrile in water containing 0.1 % trifluoroacetic acid. The product was obtained as its TFA salt and was lyophilized to give COMPOUND 1 (116 mg, 53%) as a colourless solid. Purity (HPLC) >99%; $^1H$ NMR (400 MHz, $CDCl_3$) δ 1.12-1.18 (m, 3H), 1.22-1.29 (m, 3H), 2.32 (br s, 2H), 2.46 (br s, 2H), 2.92-3.07 (m,4H), 3.24-3.35 (m, 2H), 3.50-3.61 (m, 2H), 4.34 (s, 2H), 6.80-6.87 (m, 1H), 6.94 (s, 1H), 6.99 (d, J = 7.75 Hz, 2H), 7.16-7.21 (m, 1H), 7.22-7.34 (m, 9H). Found: C, 54.84; H, 5.13; N, 5.55. $C_{30}H_{35}N_3O$ x $2.7CF_3CO_2H$ x $0.8H_2O$ has C, 54.80; H, 5.11; N, 5.42%.

**COMPOUND 2: *N,N*-diethyl-4-[{3-[(3-furylmethyl)amino]phenyl}(piperidin-4-ylidene)methyl]benzamide**

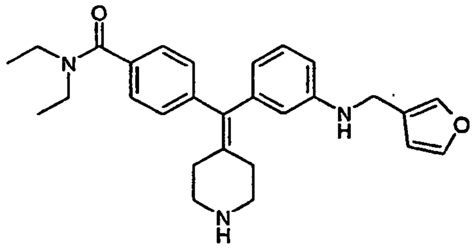

Using the same method as for COMPOUND 1 and using INTERMEDIATE 6 (0.315 g, 0.680 mmol) and 3-furaldehyde (67 μL, 0.77 mmol) provided COMPOUND 2. The crude material was purified by reverse phase HPLC (gradient 10-45% acetonitrile in water containing 0.1% trifluoroacetic acid) to give COMPOUND 2 (100 mg, 22%) as its TFA salt. This material was lyophilized to produce a pale yellow solid. Purity (HPLC): >99%; $^1H$ NMR (400MHz, $CD_3OD$) δ 0.97 (br t, J = 6.25 Hz, 3H), 1.09 (br t, J = 6.25 Hz, 3H), 2.31-2.47 (m, 4H), 2.98-3.24 (m, 6H), 3.29-3.48 (m, 2H), 4.21 (s, 2H), 6.16-6.31 (m, 1H), 6.72-6.81 (m, 1H), 6.85-6.91 (m, 1H), 6.95-7.01 (m, 1H), 7.08 (d, J = 6.44 Hz, 2H), 7.16-7.35 (m, 5H). Found: C, 54.84; H, 5.08; N, 6.86. $C_{28}H_{33}N_3O_2$ x 2.1 $CF_3CO_2H$ x 1.1 $H_2O$ has C, 55.03; H, 5.35; N, 5.98%.

**COMPOUND 3: *N,N*-diethyl-4-(piperidin-4-ylidene{3-[(thien-3-ylmethyl)amino]phenyl}methyl)benzamide**

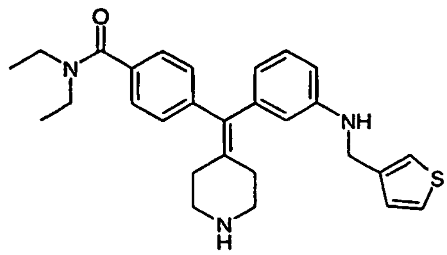

Using the same method as for COMPOUND 1 and using INTERMEDIATE 6 (0.299 g, 0.646 mmol) and 3-thiophenecarboxaldehyde (62 μL, 0.71 mmol) provided COMPOUND 3. The crude material was purified by reverse phase HPLC (gradient 10-45% acetonitrile in water containing 0.1 % trifluoroacetic acid) to give COMPOUND 3 (266 mg, 60%) as its TFA salt. This material was lyophilized to produce a pale yellow solid. Purity (HPLC): >96% (215 nm), >96% (254 nm), >97% (280 nm); $^1H$ NMR (400MHz, $CD_3OD$) δ 0.86-1.21 (m, 6H), 2.22-2.55 (m, 4H), 3.01-3.11 (m, 4 H), 3.12-3.21 (m, 2H), 3.34-3.46 (m, 2H), 4.36 (s, 2H), 6.65-6.70 (m, 1H), 6.75-6.82 (m, 1 H), 6.87-6.96 (m, 2H), 7.04-7.12 (m, 2H), 7.13-7.30 (m, 5 H). Found: C, 52.30; H, 5.02; N, 5.56. $C_{28}H_{33}N_3OS$ x 2.4 $CF_3CO_2H$ x 1.1 $H_2O$ has C, 52.31; H, 5.03; N, 5.58%.

**COMPOUND 4: *N,N*-diethyl-4-[{3-[(2-phenylethyl)amino]phenyl}(piperidin-4-ylidene)methyl]benzamide**

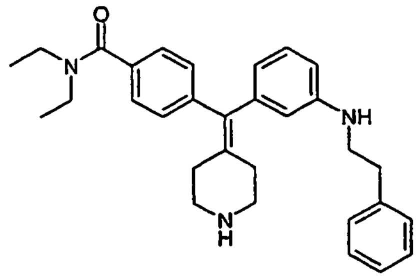

Using the same method as for COMPOUND 1 and using INTERMEDIATE 6 (0.300 g, 0.647 mmol) and phenylacetaldehyde (0.90 mL, 0.80 M solution in DCE, 0.72 mmol) provided COMPOUND 4. The crude material was purified by reverse phase chromatography (gradient 10-45% $CH_3CN$ in $H_2O$ containing 0.1 % trifluoroacetic acid) to give COMPOUND 4 (211 mg, 47%) as its TFA salt. This material was lyophilized to produce a colourless solid. Purity (HPLC): >97% (215 nm), >97% (254 nm), >99% (280 nm); $^1H$ NMR (400 MHz, $CD_3OD$) δ 1.09 (br t, J = 6.83 Hz, 3H), 1.22 (br t, J = 6.83 Hz, 3H), 2.55-2.65 (m, 4H), 2.84-2.92 (m, 2H), 3.22-3.29 (m, 6H), 3.38-3.44 (m, 2H), 3.48-3.57 (m, 2H), 6.74-6.77 (m, 1H), 6.81-6.86 (m, 1H), 6.89-6.94 (m, 1H), 7.17-7.24 (m, 3H), 7.25-7.33 (m, 5H), 7.36 (d, J = 8.40 Hz, 2H). Found: C, 57.43; H, 5.32; N, 5.62. $C_{31}H_{37}N_3O$ x 2.5 $CF_3CO_2H$ has C, 57.45; H, 5.29; N,5.58%.

**COMPOUND 5: 4-[{3-[(4-chlorobenzyl)amino]phenyl}(piperidin-4-ylidene)methyl]-*N,N*-diethylbenzamide**

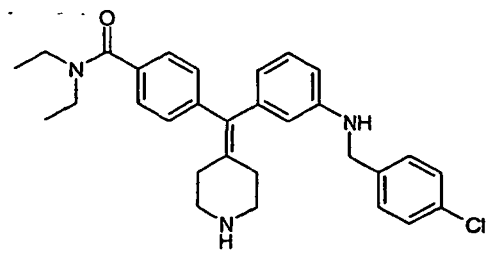

To a solution of INTERMEDIATE 6 (0.298 g, 0.643 mmol) in 1,2-dichloroethane (20 mL) was added 4-chlorobenzaldehyde (0.191 g, 1.36 mmol), sodium triacetoxyborohydride (0.286 g, 1.35 mmol) and glacial acetic acid (37 μL, 0.64 mmol). The reaction was stirred at room temperature under nitrogen. After 18 hours, trifluoroacetic acid (2.0 mL) was added and the reaction mixture was stirred for an additional 2 hours. Saturated aqueous sodium bicarbonate was slowly added until bubbling ceased. The organic layer was separated and the aqueous layer was extracted with two portions of dichloromethane. The combined organic extracts were dried ($Na_2SO_4$) filtered, and concentrated. The residue was purified by reverse phase chromatography (gradient 10-45% acetonitrile in water containing 0.1% trifluoroacetic acid). The product was obtained as the trifluoroacetic acid salt and was lyophilized to give COMPOUND 5 (209 mg, 45%) as a colourless solid. Purity (HPLC) >99%; $^1H$ NMR (400 MHz, $CD_3OD$) δ 1.12 (br t, J = 7.03 Hz, 3H), 1.25 (br t, J = 6.64 Hz, 3H), 2.42-2.57 (m, 4 H), 3.07-3.15 (m, 2H), 3.15-3.24 (m, 2H), 3.24-3.36 (m, 2H), 3.48-3.59 (m, 2H), 4.34 (s, 2H), 6.41-6.44 (m, 1 H), 6.49-6.54 (m, 1H), 6.65-6.70 (m, 1H), 7.12 (t, J =7.81 1 Hz, 1 H), 7.18 (d, J = 8.01 Hz, 2H), 7.29 (s, 4H), 7.33 (d, J = 8.20 Hz, 2H) 7.22-7.34 (m, 9H). Found: C, 54.59; H, 4.97; N, 5.53. $C_{30}H_{34}N_3OCl$ x 2.3 $CF_3CO_2H$ x 0.6 $H_2O$ has C, 54.60; H, 4.97; N, 5.52%.

**COMPOUND 6: *N,N*-diethyl-4-[piperidin-4-ylidene(3-{[3-(trifluoromethyl)benzyl]amino}phenyl)methyl]benzamide**

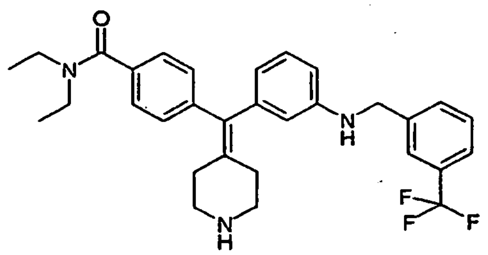

Using the same method as for COMPOUND 5 and using INTERMEDIATE 6 (0.299 g, 0.645 mmol) and trifluoro-m-tolualdehyde (174 μL, 1.30 mmol) provided COMPOUND 6 (166 mg, 34%) as a colourless solid. Purity (HPLC): >99%; $^1$H NMR (400 MHz, $CD_3OD$) δ 1.12 (br t, J = 6.44 Hz, 3H), 1.25 (br t, J = 6.44 Hz, 3H), 2.43-2.57 (m, 4H), 3.05-3.14 (m, 2H), 3.16-3.23 (m, 2H), 3.23-3.37 (m, 2H), 3.46-3.61 (m, 2H), 4.41 (s, 2H), 6. 36-6.40 (m, 1H), 6.41-6.47 (m, 1H), 6.57-6.63 (m, 1H), 7.09 (t, J = 8.01 Hz, 1H), 7.19 (d, J = 8.20 Hz, 2H), 7.31 (d, J = 8.20 Hz, 2H), 7.44-7.66 (m, 4H). Found: C, 54.84; H, 4.76; N, 5.46. $C_{31}H_{34}N_3OF_3$ x 2.2 $CF_3CO_2H$ x 0.2 $H_2O$ has C, 54.79; H, 4.75; N, 5.41%.

**COMPOUND 7: 4-[{3-[(2-chlorobenzyl)amino]phenyl}(piperidin-4-ylidene)methyl]-*N,N*-diethylbenzamide**

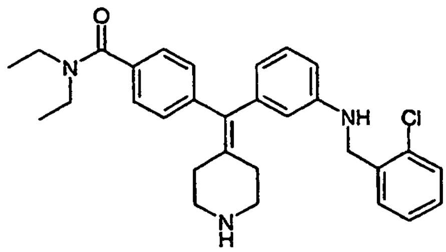

Using the same method as for COMPOUND 5 and using INTERMEDIATE 6 (0.301 g, 0.649 mmol) and 2-chlorobenzaldehyde (153 μL, 1.36 mmol) provided COMPOUND 7 (185 mg, 40%) as a yellow solid. Purity (HPLC): >99%; $^1$H NMR (400 MHz, $CD_3OD$) δ 1.12 (br t, J = 6.83 Hz, 3H), 1.24 (br t, J = 6.83 Hz, 3H), 2.44-2.56 (m, 4H), 3.06-3.12 (m, 2H), 3.16-3.22 (m, 2H), 3.23-3.35 (m, 4H), 4.38-4.42 (s, 2H), 6.31-6.35 (m, 1H), 6.40-6.45 (m, 1H), 6.56-6.61 (m, 1H), 7.04-7.12 (m, 1H), 7.17-7.26 (m, 4H), 7.32 (d, J = 8.40 Hz, 2H), 7.34-7.41 (m, 2H). Found: C, 59.06; H, 5.41; N, 6.34. $C_{30}H_{34}N_3OCl$ x 1.60 $CF_3CO_2H$ x 0.30 $H_2O$ has C, 59.00; H, 5.40; N, 6.22%.

**COMPOUND 8: *N,N*-diethyl-4-[piperidin-4-ylidene(3-{[4-(tritluoromethyl)benzyl]amino}phenyl)methyl]benzamide**

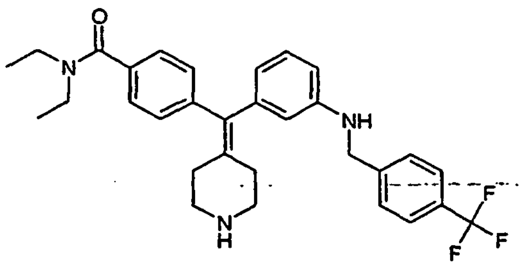

Using the same method as for COMPOUND 5 and using INTERMEDIATE 6 (0.300 g, 0.649 mmol) and trifluoro-p-tolualdehyde (187 μL, 1.36 mmol) provided COMPOUND 8 (258 mg, 53%) as a colourless solid. Purity (HPLC): >99%; $^1$H NMR (400 MHz, $CD_3OD$) δ 1.12 (br t, J = 7.23 Hz, 3H), 1.23 (br t, J = 7.03 Hz, 3H), 2.43-2.56 (m, 4H), 3.05-3.13 (m, 2H), 3.24-3.34 (m, 4H), 3.48-3.59 (m, 2H), 4.41 (s, 2H), 6.34-6.38 (m, 1H), 6.6.39-6.44 (m, 1H), 6.57 (ddd, J =

8.20,2.34,0.78 Hz, 1H), 7.07 (t, J = 8.01 Hz, 1H), 7.18 (d, J = 8.40 Hz, 2H), 7.31 (d, J = 8.40 Hz, 2H), 7.51 (d, J = 8.01 Hz, 2H), 7.60 (d, J = 8.01 Hz, 2H). Found: C, 55.12; H, 4.79; N, 5.46. $C_{31}H_{34}N_3OF_3$ x 2.1 $CF_3CO_2H$ x 0.3 $H_2O$ has C, 55.16; H, 4.83; N, 5.48%.

**COMPOUND 9: *N,N*-diethyl-4-[{3-[(2-furylmethyl)amino]phenyl}(piperidin-4-ylidene)methyl]benzamide**

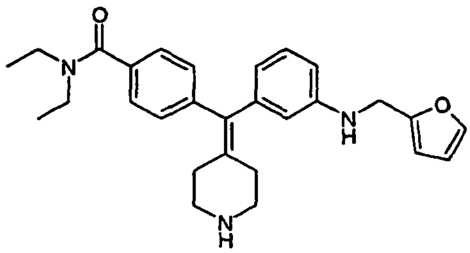

Using the same method as for COMPOUND 5 and using INTERMEDIATE 6 (0.297 g, 0.641 mmol) and furfural (110 μL, 1.33 mmol) provided COMPOUND 9. The crude material was purified by reverse phase chromatography (gradient 20-80% acetonitrile in water containing 0.1% trifluoroacetic acid) to give COMPOUND 9 (100 mg, 23%) as its TFA salt. This material was lyophilized to produce a yellow solid. Purity (HPLC): >99%; $^1$H NMR (400 MHz, $CD_3OD$) δ 1.13 (br t, J = 7.42 Hz, 3H), 1.23 (t, J = 7.42 Hz, 3H), 2.52-2.59 (m, 4H), 3.17-3.26 (m, 4H), 3.26-3.34 (m, 2H), 3.48-3.59 (m, 2H), 4.30 (s, 2H), 6.17-6.19 (m, 1H), 6.31 (dd, J = 3.12, 1.76 Hz, 1H), 6.50-6.56 (m, 2H), 6.69 (ddd, J = 8.20, 2.15, 0.98 Hz, 1H), 7.13 (t, J = 8.01 Hz, 1H), 7.24 (d, J = 8.40 Hz, 2H), 7.34 (d, J = 8.40 Hz, 2H), 7.40 (dd, J = 1.95, 0.78 Hz, 1H). Found: C, 54.91; H, 5.03; N, 5.96. $C_{28}H_{33}N_3O_2$ x 2.30 $CF_3CO_2H$ x 0.40 $H_2O$ has C, 54.91; H, 5.10; N, 5.89%.

**COMPOUND 10: *N,N*-diethyl-4-(piperidin-4-ylidene{3-[(thien-2-ylmethyl)amino]phenyl}methyl)benzamide**

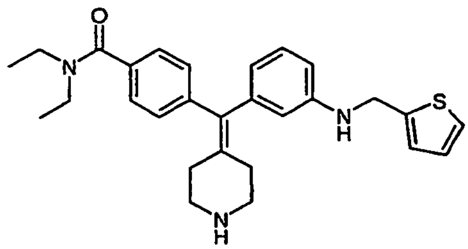

Using the same method as for COMPOUND 5 and using INTERMEDIATE 6 (0.289 g, 624 mmol) and 2-thiophenecarboxaldehyde (0.12 mL, 1.28 mmol) provided COMPOUND 10. The crude material was purified by reverse phase chromatography (gradient 10-60% acetonitrile in water containing 0.1 % trifluoroacetic acid) to give COMPOUND 10 (239 mg, 56%) as its TFA salt. This material was lyophilized to produce a colourless solid. Purity (HPLC): >94% (215 nm), >97% (254 nm), >99% (280 nm); $^1$H NMR (400 MHz, $CD_3OD$) δ 1.12 (br t, J = 7.03 Hz, 3H), 1.22 (br t, J = 7.03 Hz, 3H), 2.47-2.59 (m, 4H), 3.11-3.17 (m, 2H), 3.17-3.24 (m, 2H), 3.24-3.36 (m, 2H), 3.48-3.58 (m, 2H), 4.50-4.55 (s, 2H), 6.49-6.55 (m, 2H), 6.68-6.73 (m, 1H), 6.91-6.99 (m, 2H), 7.13 (d, J = 7.23 Hz, 1H), 7.22 (d, J = 7.42 Hz, 2H), 7.24-7.27 (m, 1H), 7.33 (d, J = 7.62 Hz, 2H). Found: C, 55.30; H, 5.23; N, 6.06. $C_28H_{33}N_3OS$ x 2.0 $CF_3CO_2H$ x 0.4 $H_2O$ has C, 55.31; H, 5.19; N, 6.05%.

**COMPOUND 11: 4-[{3-[(cyclohexylmethyl)amino]phenyl}(piperidin-4-ylidene)methyl]-*N,N*-diethylbenzamide**

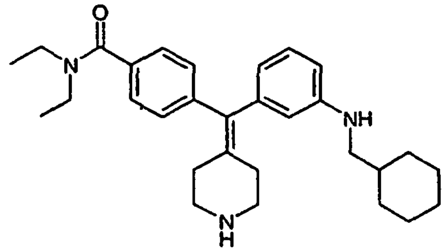

Using the same method as for COMPOUND 5 and using INTERMEDIATE 6 (0.198 g, 0.428 mmol) and cyclohexanecarboxaldehyde (0.10 mL, 0.83 mmol) provided COMPOUND 11 (147 mg, 50%) as a yellow solid. Purity (HPLC): >99%; $^1H$ NMR (400 MHz, $CD_3OD$) δ 0.96-1.08 (m, 2H), 1.12 (t; J = 6.83 Hz., 3H), 1.17-1.32 (m, 6H), 1.57-1.85 (m, 6H), 2.56-2.64 (m, 4H), 3.07 (d, J = 6.83 Hz, 2H), 3.22-3.35 (m, 6H), 3.49-3.58 (m, 2H), 6.90-6.93 (m, 1H), 6.95-6.99 (m, 1H), 7.02-7.07 (m, 1H), 7.27 (d, J = 8.20 Hz, 2H), 7.33-7.40 (m, 3H). Found: C, 56.22; H, 6.30; N, 5.36. $C_{30}H_{41}N_3O$ x 2.3 $CF_3CO_2H$ x 1.0 $H_2O$ has C, 56.16; H, 6.17; N, 5.68%.

**COMPOUND 12: *N,N*-diethyl-4-{piperidin-4-ylidene[3-(propylamino)phenyl]methyl}benzamide**

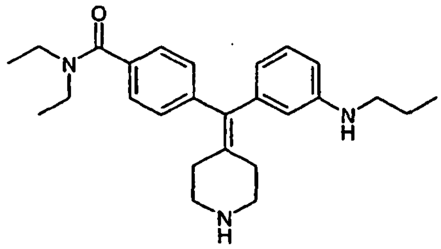

To a solution of INTERMEDIATE 6 (0.299 g, 0.645 mmol) in methanol (5 mL) was added propionaldehyde (47 μL, 0.64 mmol). The reaction was stirred for 30 minutes and then decaborane (24 mg, 0.19 mmol) was added. After 2 hours, the reaction was complete. The mixture was diluted with dichloromethane and washed with saturated aqueous sodium bicarbonate. The aqueous layer was extracted with two portions of dichloromethane and the combined organic extracts were dried ($Na_2SO_4$), filtered and concentrated. The residue was dissolved in dichloromethane (10 mL) and trifluoroacetic acid (1.5 mL) was added. The reaction was stirred overnight. The reaction mixture was concentrated *in vacuo* and purified by reverse phase chromatography (gradient 10-50% acetonitrile in water containing 0.1 % trifluoroacetic acid). The product was obtained as the trifluoroacetic acid salt and was lyophilized to give COMPOUND 12 (107 mg, 26%) as a colourless solid. Purity (HPLC): >99%; $^1H$ NMR (400 MHz, $CD_3OD$) δ 0.99 (t, J = 7.42 Hz, 3H), 1.12 (br t, J = 6.64 Hz, 3H), 1.24 (br t, J = 6.64 Hz, 3H), 1.62-1.73 (sextet, 2H), 2.57-2.63 (m, 4H), 3.18-3.24 (m, 2H), 3.18-3.24 (m, 2H), 3.24-3.33 (m, 6H), 3.49-3.58 (m, 2H), 6.97-6.99 (m, 1H), 7.06-7.10 (m, 1H), 7.10-7.14 (m, 1H), 7.27 (d, J = 8.40 Hz, 2H), 7.37 (d, J = 8.40 Hz, 2H), 7.42 (t, J = 7.81 Hz, 1H). Found: C, 51.78; H, 4.74; N, 5.85. $C_{26}H_{35}N_3O$ x 2.9 $CF_3CO_2H$ has C, 51.88; H, 5.19; N, 5.71%.

**COMPOUND 13: 4-[[3-(cyclohexylamino)phenyl](piperidin-4-ylidene)methyl]-*N,N*-diethylbenzamide**

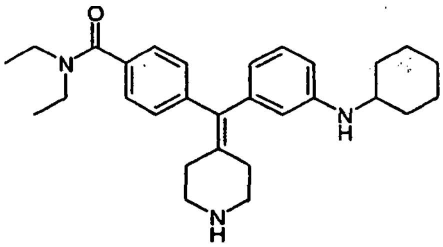

To a solution of INTERMEDIATE 6 (0.200 g, 0.432 mmol) in 1,2-dichloroethane (15 mL) was added cyclohexanone (50 μL, 0.482 mmol), sodium triacetoxyborohydride (0.138 g, 0.651 mmol) and glacial acetic acid (25 μL, 0.44 mmol). The reaction was stirred at room temperature under nitrogen. After 24 hours, trifluoroacetic acid (2.0 mL) was added and the reaction mixture was stirred for an additional 2 hours. Saturated aqueous sodium bicarbonate was slowly added until bubbling ceased. The organic layer was separated and the aqueous layer was extracted with two portions of dichloromethane. The combined organic extracts were dried ($Na_2SO_4$), filtered, and concentrated. The residue was purified by reverse phase chromatography (gradient 10-45% acetonitrile in water containing 0.1% trifluoroacetic acid). The product was obtained as the trifluoroacetic acid salt and was lyophilized to give COMPOUND 13 (187 mg, 64%) as a colourless solid. Purity (HPLC) >99%; $^1$H NMR (400 MHz, $CD_3OD$) δ 1.07-1.16 (m, 3H), 1.17-1.28 (m, 4H), 1 .28-1 . 39 (m, 4H), 1.64-1.72 (m, 1H), 1.77-1.87 (m, 2H), 1.88-1.98 (m, 2H), 2.58-2.64 (m, 4H), 3.24-3.41 (m, 7H), 3.49-3.58 (m, 2H), 7.07-7.07 (m, 1H), 7.19-7.25 (m, 2H), 7.27 (d, J = 8.40 Hz, 2H), 7.38 (d, J = 8.40 Hz, 2H), 7.50 (t, J = 7.81 Hz, 1H). Found: C, 55.13; H, 5.78; N, 5.58. $C_{29}H_{39}N_3O$ x 2.5 $CF_3CO_2H$ x 0.6 $H_2O$ has C, 55.07; H, 5.80; N, 5.67%.

**COMPOUND 14: 4-[[3-(cyclopentylamino)phenyl](piperidin-4-ylidene)methyl]-*N,N*-diethylbenzamide**

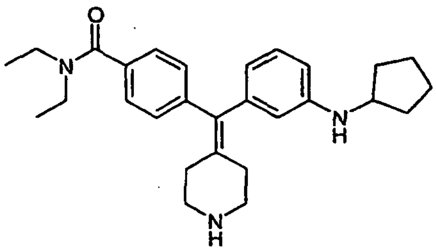

To a solution of INTERMEDIATE 6 (0.117 g, 0.252 mmol) in 1,2-dichloroethane (10 mL) was added cyclopentanone (70 μL, 0.78 mmol), sodium triacetoxyborohydride (0.187 g, 0.882 mmol) and glacial acetic acid (30 μL, 0.52 mmol). The reaction was stirred at room temperature under an atmosphere of nitrogen. After 24 hours, trifluoroacetic acid (1.0 mL) was added and the reaction mixture was stirred for an additional 2 hours. Saturated aqueous sodium bicarbonate was slowly added until bubbling ceased. The organic layer was separated and the aqueous layer was extracted with two portions of dichloromethane. The combined organic extracts were dried ($Na_2SO_4$), filtered, and concentrated. The residue was purified by reverse phase chromatography (gradient 10-45% acetonitrile in water containing 0.1% trifluoroacetic acid). The product was obtained as the trifluoroacetic acid salt and was lyophilized to give COMPOUND 14 (120 mg, 72%) as a colourless solid. Purity (HPLC) >99%; $^1$H NMR (400 MHz, $CD_3OD$) δ 1.09 (t, J = 6.64 Hz, 3H), 1.21 (t, J = 6.64 Hz, 3H), 1.52-1.68 (m, 4H), 1.69-1.82 (m, 2H), 1.88-2.00 (m, 2H), 2.54-2.61 (m, 4H), 3.20-3.31 (m, 6H), 3.46-3.56 (m, 2H), 3.80-3.90 (m, 1H), 6.98-7.01 (m, 1H), 7.09-7.17 (m, 2H), 7.24 (d, J = 8.40 Hz, 2H), 7.34 (d, J = 8.40 Hz, 2H), 7.43 (t, J = 8.01 Hz, 1H). Found: C, 55.10; H, 5.87; N, 5.65. $C_{28}H_{37}N_3O$ x 2.3 $CF_3CO_2H$ x 1.0 $H_2O$ has C, 55.00; H, 5.85; N, 5.90%.

**COMPOUND 15: 4-[[3-(cycloheptylamino)phenyl](piperidin-4-ylidene)methyl]-*N,N*-diethylbenzamide**

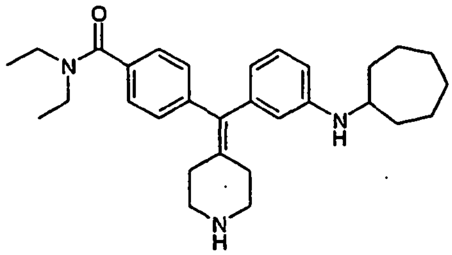

Using the same method as for COMPOUND 14 and using INTERMEDIATE 6 (0.409 g, 0.883 mmol) and cycloheptanone (0.32 mL, 2.7 mmol) provided COMPOUND 15 (292 mg, 48%) as a colourless solid. Purity (HPLC): >99%; $^1$H NMR (400 MHz, $CD_3OD$) δ 1.13 (br t, J = 7.62 Hz, 3H), 1.23 (br t, J = 7.03 Hz, 3H), 1.39-1.68 (m, 8H), 1.68-1.79 (m, 2H), 1.92-2.02 (m, 2H), 2.58-2.64 (m, 4H), 3.24-3.34 (m, 6H), 3.49-3.63 (m, 3H), 7.11-7.14 (m, 1H), 7.24-7.31 (m, 4H), 7.38 (d, J = 8.40 Hz, 2H), 7.49-7.55 (m, 1H). Found: C,55.82; H, 5.86; N, 5.73. $C_{30}H_{41}N_3O$ x 2.5 $CF_3CO_2H$ x 0.4 $H_2O$ has C, 55.91; H, 5.94; N, 5.59%.

**COMPOUND 16: 4-[{3-[cyclopentyl(methyl)amino]phenyl}(piperidin-4-ylidene)methyl]-*N,N*-diethylbenzamide**

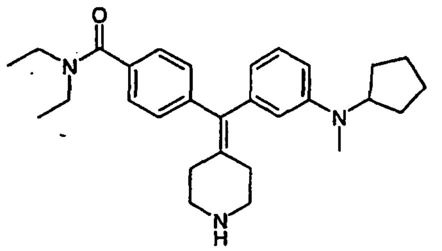

To a solution of INTERMEDIATE 6 (0.390 g, 0.841 mmol) in 1,2-dichloroethane (20 mL) was added cyclopentanone (0.22 mL, 2.5 mmol), sodium triacetoxyborohydride (0.570 g, 2.69 mmol) and glacial acetic acid (50 μL, 0.84 mmol). The reaction was stirred overnight at room temperature under an atmosphere of nitrogen. The mixture was washed with two portions of saturated aqueous sodium bicarbonate and one portion of brine. The organic phase was dried ($Na_2SO_4$), filtered, and concentrated. The residue was purified by flash chromatography, eluting 0% to 50% ethyl acetate in hexanes to yield 0.356 g of product as a colourless foam. The material was dissolved in methanol (5 mL) and formaldehyde (37% in water) (150 μL, 2.01 mmol) was added. The reaction was stirred for 30 minutes and then decaborane (49 mg, 0.40 mmol) was added. After 18 h, the reaction mixture was concentrated. The residue was dissolved in dichloromethane (10 mL) and trifluoroacetic acid (1.5 mL) was added. The reaction was stirred for 4 hours at room temperature and then saturated aqueous sodium bicarbonate was added. The aqueous layer was extracted with two portions of dichloromethane and the combined organic extracts were dried ($Na_2SO_4$), filtered and concentrated. The residue was purified by reverse phase chromatography (gradient 10-50% acetonitrile in water containing 0.1% trifluoroacetic acid). The product was obtained as the trifluoroacetic acid salt and was lyophilized to give COMPOUND 16 (260 mg, 58%) as a colourless solid. Purity (HPLC) >93% (215 nm), >99% (254 nm), >99% (280 nm); $^1$H NMR (400 MHz, $CD_3OD$) δ 1.12 (br t, J = 6.64 Hz, 3H), 1.24 (br t, J = 7.62 Hz, 3H), 1.58-1.72 (m, 4H), 1.73-1.84 (m, 2H), 1.85-2.03 (m, 2H), 2.57-2.66 (m, 4H), 3.22-3.35 (m, 9H), 3.49-3.59 (m, 2H), 4.09-4.20 (m, 1H), 7.28 (d, J = 8.01 Hz, 2H), 7.35-7.43 (m, 4H), 7.51-7.56 (m, 1H), 7.58-7.65 (m, 1H). Found: C, 52.68; H, 5.63; N, 5.33. $C_{29}H_{39}N_3O$ x 2.8 $CF_3CO_2H$ x 1.3 $H_2O$ has C, 52.72; H, 5.68; N, 5.33%.

**COMPOUND 17: 4-[[3-(benzoylamino)phenyl](piperidin-4-ylidene)methyl]-*N,N*-diethylbenzamide**

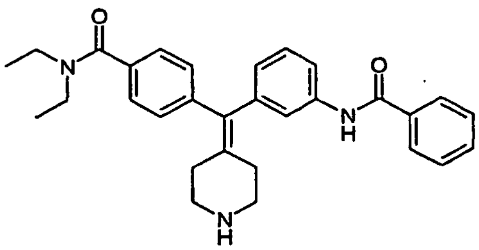

To a solution of INTERMEDIATE 6 (0.158 g, 0.341 mmol) in dichloromethane (10 mL) was added benzoic anhydride (86 mg, 0.38 mmol) and triethylamine (0.15 mL, 1.08 mmol). The reaction was stirred for 24 h at room temperature under an atmosphere of nitrogen. The mixture was washed with saturated aqueous sodium bicarbonate. The aqueous layer was extracted with two portions of dichloromethane and the combined organic extracts were dried ($Na_2SO_4$), filtered and concentrated. The residue was dissolved in dichloromethane (10 mL) and trifluoroacetic acid (1.0 mL) was added. After 2 hours, the solution was concentrated and the residue was purified by reverse phase chromatography, eluting 10% to 45% acetonitrile in water containing 0.1% trifluoroacetic acid. The product was obtained as its TFA salt and was lyophilized to give COMPOUND 17 (140 mg, 71%) as a colourless solid. Purity (HPLC) >99; $^1$H NMR (400 MHz, $CD_3OD$) δ 1.13 (br t, J = 6.83 Hz, 3H), 1.23 (br t, J = 7.23 Hz, 3H), 2.58-2.63 (m, 2H), 2.64-2.69 (m, 2H), 3.23-3.35 (m, 6H), 3.49-3.58 (m, 2H), 6.92-6.96 (m, 1H), 7.27-7.33 (m, 2H), 7.33-7.39 (m, 3H), 7.47-7.54 (m, 3H), 7.55-7.61 (m, 1H), 7.69-7.73 (m, 1H), 7.88.7.94 (m, 2H). Found: C, 61.96; H, 5.57; N, 6.59. $C_{30}H_{33}N_3O_2$ x 1.4 $CF_3CO_2H$ x 0.5 $H_2O$ has C, 61.92; H, 5.61; N,6.60%.

**COMPOUND 18: *N,N*-diethyl-4-[{3-[(phenylacetyl)amino]phenyl}(piperidin-4-ylidene)methyl]benzamide**

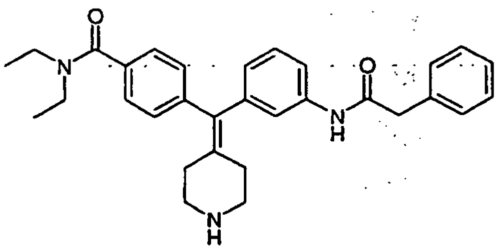

To a solution of iNTERMEDIATE 6 (0.154 g, 0.332 mmol) in dichloromethane (10 mL) was added phenylacetyl chloride (48 μL, 0.36 mmol) and triethylamine (0.15 mL, 1.08 mmol). The reaction was stirred for 20 hours at room temperature under an atmosphere of nitrogen. The mixture was washed with saturated aqueous sodium bicarbonate. The aqueous layer was extracted with two portions of dichloromethane and the combined organic extracts were dried ($Na_2SO_4$), filtered and concentrated. The residue was dissolved in dichloromethane (10 mL) and trifluoroacetic acid (1.0 mL) was added. After 2 hours, the solution was concentrated and the residue was purified by reverse phase chromatography, eluting 10% to 45% acetonitrile in water containing-0.1 % trifluoroacetic acid. The product was obtained as its TFA salt and was lyophilized to give COMPOUND 18 (181 mg, 91 %) as a colourless solid. Purity (HPLC) >99%; $^1$H NMR (400 MHz, $CD_3OD$) δ 1.12 (br t, J = 6.25 Hz, 3H), 1.23 (br t, J = 6.25 Hz, 3H), 2.54-2.64 (m, 4H), 3.20-3.26 (m, 4H), 3.27-3.34 (m, 2H), 3.48.3.58 (m, 2H), 3.65 (s, 2H), 6.86-6.91 (m, 1H), 7.21-7.40 (m, 11H), 7.56-7.60 (m, 1H). Found: C, 59.65; H, 5.46; N, 5.91. $C_{31}H_{35}N_3O_2$ x 1.8 $CF_3CO_2H$ x 0.6 $H_2O$ has C, 59.57; H, 5.49; N, 6.02%.

**COMPOUND 19: 4-[{3-[(cyclohexylcarbonyl)amino]phenyl}(piperidin-4-ylidene)methyl]-*N,N*-diethylbenzamide**

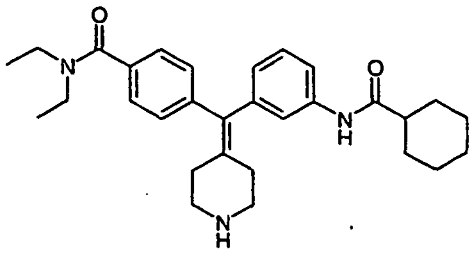

Using the same method as for COMPOUND 18 and using INTERMEDIATE 6 (0.105 g, 0.227 mmol) and cyclohexanecarbonyl chloride (33 μL, 0.25 mmol) provided COMPOUND 19 (110 mg, 83%) as a colourless solid. Purity (HPLC): >99%; $^1H$ NMR (400 MHz, $CD_3OD$) δ 1.12 (m, 3H), 1.18-1.41 (m, 6H), 1.49 (m, 2H), 1.72 (m, 1H), 1.83 (m, 4H), 2.33 (m, 1H), 2.60 (m, 4H); 3.22-3.34 (m, 6H); 3.53 (m, 2H), 6.87 (dt, J = 7.42, 1.56 Hz, 1H), 7.24-7.29 (m, 3H), 7.32 (m, 1H), 7.35 (d, J = 8.59 Hz, 2H), 7.57 (m, 1H). Found: C, 59.33; H, 6.31; N, 6.21. $C_{30}H_{39}N_3O_2$ x 1.6 $CF_3CO_2H$ x 0.9 $H_2O$ has C, 59.31; H, 6.36; N, 6.25%.

**COMPOUND 20: 4-[{3-[(cyclohexylacetyl)amino]phenyl}(piperidin-4-ylidene)methyl]-*N,N*-diethylbenzamide**

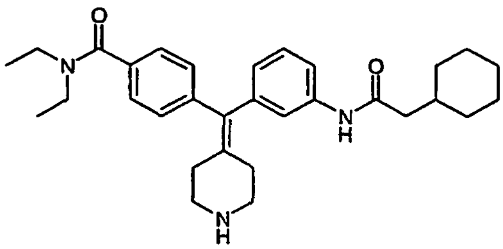

To a solution of INTERMEDIATE 6 (0.105 g, 0.226 mmol) in dry DMF (5 mL) was added cyclohexylacetic acid (50 mg, 0.35 mmol), HATU (0.122 g, 0.321 mmol), and *N,N*-diisopropylethylamine (100 μL, 0.574 mmol). The reaction was stirred overnight at room temperature under nitrogen, then concentrated *in vacuo.* The residue was dissolved in dichloromethane and washed with 2N NaOH (1x) and brine (1x). The organic phase was dried ($Na_2SO_4$), filtered and concentrated. The residue was dissolved in dichloromethane (5 mL) and trifluoroacetic acid (0.5 mL) was added. The reaction was stirred for 4 hours at room temperature. The solution was concentrated and the residue was purified by reverse phase chromatography (gradient 10-45% acetonitrile in water containing 0.1% trifluoroacetic acid). The product was obtained as its TFA salt and was lyophilized to give COMPOUND 20 (97 mg, 71%) as a colourless solid. Purity (HPLC) >99%; $^1H$ NMR (400 MHz, $CD_3OD$) δ 0.96-1.37 (m, 11H), 1.62-1.88 (m, 6H), 2.21 (d, J = 7.03 Hz, 2H), 2.56-2.24 (m, 4H), 3.21-3.34 (m, 6H), 3.48-3.59 (m, 2H), 6.88 (dt, J = 7.42, 1.37 Hz, 1H), 7.24-7.30 (m, 3H), 7.32 (m, 1H), 7.36 (d, J = 8.40 Hz, 2H), 7.56 (m, 1H). Found: C, 60.39; H, 6.68; N, 6.10. $C_{31}H_{41}N_3O_2$ x 1.5 $CF_3CO_2H$ x 1.0 $H_2O$ has C, 60.34; H, 6.63; N, 6.21 %.

**COMPOUND 21: 4-[(3-{[(2-chlorophenyl)acetyl]amino}phenyl)(piperidin-4-ylidene)methyl]-*N,N*-diethylbenzamide**

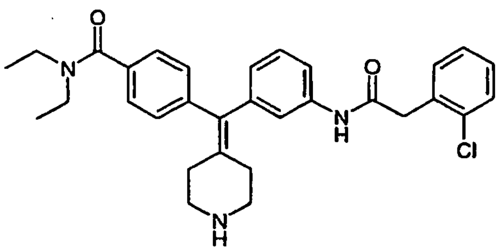

Using the same method as for COMPOUND 20 and using INTERMEDIATE 6 (0.138 g, 0.298 mmol) and 2-chlorophenylacetic acid (77 mg, 0.45 mmol) provided COMPOUND 20 (115 mg, 61%) as a colourless solid. Purity (HPLC): >96% (215 nm), >96% (254 nm), >99% (280 nm); $^1H$ NMR (400 MHz, $CD_3OD$) δ 1.12 (br t, J = 6.83 Hz, 3H), 1.23 (br t, J = 6.83 Hz, 3H), 2.54-2.65 (m, 4H), 3.20-3.26 (m, 4H), 3.26-3.34 (m, 2H), 3.48-3.59 (m, 2H), 3.85 (s, 2H), 6.86-6.90 (m, 1H), 7.22-7.31 (m, 5H), 7.31-7.42 (m, 5H), 7.56-7.60 (m, 1H). Found: C, 59.20; H, 5.20; N, 6.08. $C_{31}H_{34}N_3O_2Cl$ x 1.5 $CF_3CO_2H$ x 0.1 $H_2O$ has C, 59.28; H, 5.22; N, 6.10%.

**COMPOUND 22: 4-[(3-{[(3-chlorophenyl)acetyl]amino}phenyl)(piperidin-4-ylidene)methyl]-N,N-diethylbenzamide**

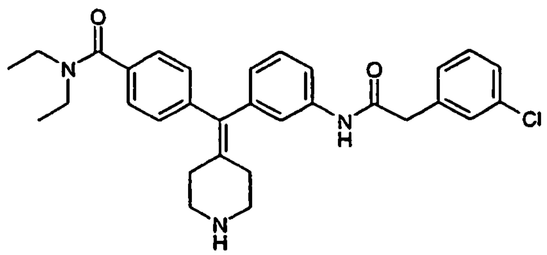

Using the same method as for COMPOUND 20 and using INTERMEDIATE 6 (0.139 g, 0.300 mmol) and 3-chlorophenylacetic acid (77 mg, 0.45 mmol) provided COMPOUND 22 (125 mg, 66%) as a colourless solid. Purity (HPLC): >99%; $^1H$ NMR (400 MHz, $CD_3OD$) δ 1.10 (br t, J = 6.83 Hz, 3H), 1.22 (br t, J = 6.83 Hz, 3H), 2.53-2.65 (m, 4H), 3.20-3.33 (m, 6H), 3.48-3.58 (m, 2H), 3.66 (s, 2H), 6.86-6.91 (m, 1H), 7.21-7.32 (m, 7H), 7.32-7.39 (m, 3H), 7.56-7.60 (m, 1H). Found: C, 57.66; H, 5.21; N, 5.88. $C_{31}H_{34}N_3O_2Cl$ x 1.6 $CF_3CO_2H$ x 0.8 $H_2O$ has C, 57.62; H, 5.26; N, 5.89%.

**COMPOUND 23: *N,N*-diethyl-4-[(3-{[(5-methylthien-2-yl)acetyl]amino}phenyl)(piperidin-4-ylidene)methyl]benzamide**

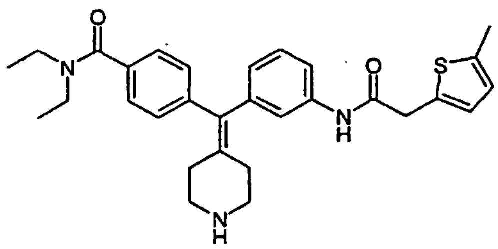

Using the same method as for COMPOUND 20 and using INTERMEDIATE 6 (0.140 g, 0.302 mmol) and (5-methylthien-2-yl)acetic acid (71 mg, 0.46 mmol) provided COMPOUND 23 (121 mg, 65%) as a colourless solid. Purity (HPLC): >99% (215 nm), >99% (254 nm), >99% (280 nm); $^1H$ NMR (400 MHz, $CD_3OD$) δ 1.12 (br t, J = 6.83 Hz, 3H), 1.23 (br t, J = 6.83 Hz, 3H), 2.41 (d, J = 0.78 Hz, 3H), 2.55-2.64 (m, 4H), 3.20-3.34 (m, 6H), 3.48-3.58 (m, 2H), 3.76 (s, 2H), 6.59-6.62 (m, 1H), 6.71-6.75 (m, 1H), 6.86-6.91 (m, 1H), 7.26 (d, J = 8.40 Hz, 2H), 7.27-7.33 (m, 2H), 7.35 (d, J = 8.40 Hz, 2H), 7.56-7.59 (m, 1H). Found: C, 59.92; H, 5.74; N, 6.46. $C_{30}H_{35}N_3O_2S$ x 1.2 $CF_3CO_2H$ x 0.6 $H_2O$ has C, 59.93; H, 5.81; N, 6.47%.

**COMPOUND 24: 4-[(3-{[(5-chlorothien-2-yl)acetyl]amino}phenyl)(piperidin-4-ylidene)methyl]-N,N-diethylbenzamide**

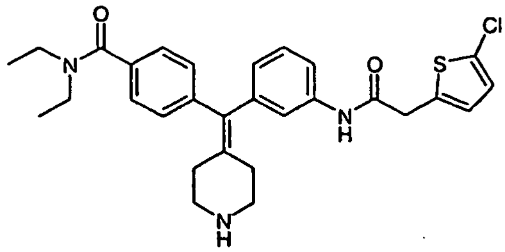

Using the same method as for COMPOUND 20 and using INTERMEDIATE 6 (0.164 g, 0.354 mmol) and 5-chlorothiophene-2-acetic acid (86 mg, 0.53 mmol) provided COMPOUND 24 (168 mg, 75%) as a colourless solid. Purity (HPLC): >99% (215 nm), >99% (254 nm), >99% (280 nm); $^1$H NMR (400 MHz, $CD_3OD$) δ 1.11 (br t, J = 7.03 Hz, 3H), 1.23 (br t, J = 7.03 Hz, 3H), 2.54-2.65 (m, 4H), 3.20-3.34 (m, 6H), 3.48-3.58 (m, 2H), 3.80 (d, J = 0.78 Hz, 2H), 6.77-6.79 (m, 1H), 6.82 (d, J = 3.71 Hz, 1H), 6.88-6.92 (m, 1H); 7.26 (d,-J = 8.40 Hz, 2H), 7.28-7.34 (m,2H), 7.36-(d, J= 8:20 Hz, 2H), 7.57-7.60 (m, 1H). Found: C, 54.64; H, 4.85; N, 5.92. $C_{29}H_{32}N_3O_2SCl$ x 1.6 $CF_3CO_2H$ x 0.2 $H_2O$ has C, 54.51; H, 4.84; N, 5.93%.

**COMPOUND 25: *N,N*-diethyl-4-[(3-{[(2S)-2-phenylpropanoyl]amino}phenyl)(piperidin-4-ylidene)methyl]benzamide**

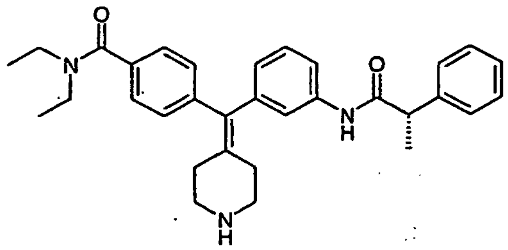

Using the same method as for COMPOUND 20 and using INTERMEDIATE 6 (0.152 g, 0.328 mmol) and (S)-(+)-2-phenylpropionic acid (74 mg, 0.49 mmol) provided COMPOUND 25 (129 mg, 65%) as a colourless solid. Purity (HPLC): >99% (215 nm), >99% (254 nm), >99% (280 nm); Optical purity (Chiral HPLC): > 99%; $^1$H NMR (400 MHz, $CD_3OD$) δ 1.11 (br t, J = 6.64 Hz, 3H), 1.22 (br t, J = 7.23 Hz, 3H), 1.49 (d, J = 7.03 Hz, 3H), 2.54-2.63 (m, 4H), 3.19-3.35 (m, 6H), 3.48-3.58 (m, 2H), 3.78 (q, J = 7.03 Hz, 1H), 6.84-6.89 (m, 1H), 7.20-7.28 (m, 4H), 7.27-7.40 (m, 7H), 7.53-7.56 (m, 1H). Found: C, 61.47; H, 5.79; N, 6.08. $C_{32}H_{37}N_3O_2$ x 1.6 $CF_3CO_2H$ x 0.5 $H_2O$ has C, 61.53; H, 5.81; N, 6.12%.

**COMPOUND 26: *N,N*-diethyl-4-[(3-{[(2R)-2-phenylpropanoyl]amino}phenyl)(piperidin-4-ylidene)methyl]benzamide**

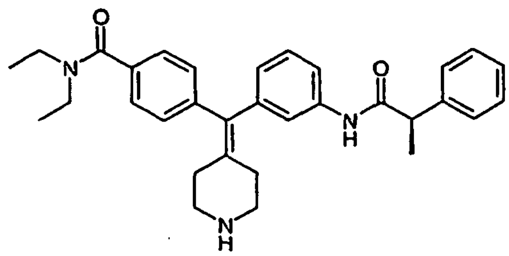

Using the same method as for COMPOUND 20 and using INTERMEDIATE 6 (0.309 g, 0.666 mmol) and (R)-(-)-2-phenylpropionic acid (0.150 g, 0.999 mmol) provided COMPOUND 26 (175 mg, 43%) as a colourless solid. Purity (HPLC): >99% (215 nm), >99% (254 nm), >99% (280 nm); Optical purity (Chiral HPLC): > 99%; $^1$H NMR (400 MHz,

$CD_3OD$) δ 1.11 (br t, J = 7.42 Hz, 3H), 1.23 (br t, J = 6.83 Hz, 3H), 1.48 (d, J = 7.03 Hz, 3H), 2.55-2.62 (m, 4H), 3.21-3.32 (m, 6H), 3.49-3.57 (m, 2H), 3.78 (q, J = 7.03 Hz, 1H), 6.86 (dt, J = 7.23, 1.37 Hz, 1H), 7.20-7.27 (m, 3H), 7.27-7.40 (m, 8H), 7.53-7.55 (m, 1H). Found: C, 60.28; H, 5.79; N, 6.16. $C_{32}H_{37}N_3O_2$ x 1.7 $CF_3CO_2H$ x 0.9 $H_2O$ has C, 60.25; H, 5.78; N, 5.95%.

**COMPOUND 27: *N,N*-diethyl-4-[(3-{[(2S)-2-phenylbutanoyl]amino}phenyl)(piperidin-4-ylidene)methyl]benzamide**

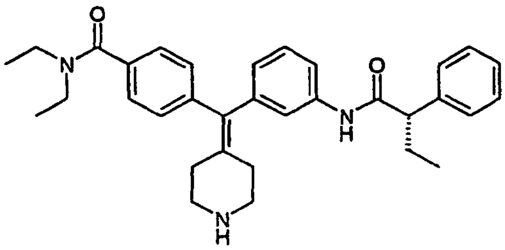

Using the same method as for COMPOUND 20 and using INTERMEDIATE 6 (0.346 g, 0.746 mmol) and (S)-(+)-2-phenylbutyric acid (0.184 g, 1.12 mmol) provided COMPOUND 27 (148 mg, 32%) as a colourless solid. Purity (HPLC): >99%; Optical purity (Chiral HPLC): > 99%; $^1H$ NMR (400 MHz, $CD_3OD$) δ 0.92 (t, J = 7.42 Hz, 3H), 1.11 (br t, J = 7.03 Hz, 3H), 1.23 (br t, J = 7.23 Hz, 3H), 1.72-1.85 (m, 1H), 2.05-2.17 (m, 1H), 2.54-2.62 (m, 4H), 3.21-3.32 (m, 6H), 3.46-3.57 (m, 3H), 6.85-6.89 (m, 1H), 7.20-7.40 (m, 11H), 7.52-7.55 (m, 1H). Found: C, 51.82; H, 4.66; N, 4.75. $C_{33}H_{39}N_3O_2$ x 3.6 $CF_3CO_2H$ x 0.6 $H_2O$ has C, 51.86; H, 4.74; N, 4.51 %.

**COMPOUND 28: *N,N*-diethyl-4-[(3-{[(2R)-2-phenylbutanoyl]amino}phenyl)(piperidin-4-ylidene)methyl]benzamide**

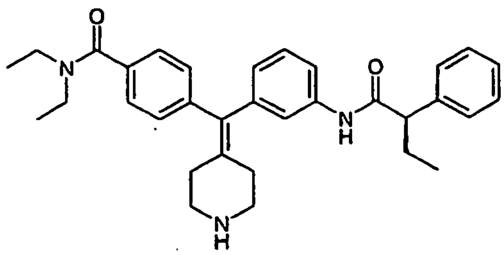

Using the same method as for COMPOUND 19 and using INTERMEDIATE 6 (0.309 g, 0.666 mmol) and (R)-(-)-2-phenylbutyric acid (0.354 g, 0.932 mmol) provided COMPOUND 28 (189 mg, 45%) as a colourless solid. Purity (HPLC): >99%; Optical purity (Chiral HPLC): > 99%; $^1H$ NMR (400 MHz, $CD_3OD$) δ 0.92 (t, J = 7.42 Hz, 3H), 1.11 (br t, J = 7.62 Hz, 3H), 1.23 (br t, J = 6.64 Hz, 3H), 1.72-1.84 (m, 1H), 2.06-2.16 (m, 1H), 2.55-2.62 (m, 4H), 3.21-3.32 (m, 6H), 3.47-3.57 (m, 3H), 6.87 (dt, J = 7.42, 1.37 Hz, 1H), 7.22-7.33 (m, 7H), 7.34 (d, J = 8.40 Hz, 2H), 7.36-7.40 (m, 2H), 7.53-7.55 (m, 1H). Found: C, 64.16; H, 6.79; N, 6.26. $C_{33}H_{39}N_3O_2$ x 1.0 $CF_3CO_2H$ x 1.8 $H_2O$ has C, 64.07; H, 6.70; N, 6.40%.

**COMPOUND 29: 4-[{3-[benzoyl(methyl)amino]phenyl}(piperidin-4-ylidene)methyl]-*N,N*-diethylbenzamide**

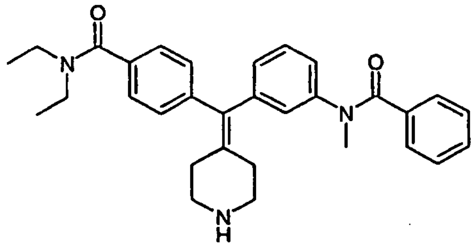

To a solution of INTERMEDIATE 6 (0.153 g, 0.330 mmol) in dichloromethane (10 mL) was added benzoyl chloride (42 μL, 0.36 mmol) and triethylamine (0.15 mL, 1.1 mmol). The reaction was stirred for 24 h at room temperature under an atmosphere of nitrogen. The mixture was washed with saturated aqueous sodium bicarbonate and the aqueous layer extracted with two portions of dichloromethane. The combined organic extracts were dried ($Na_2SO_4$), filtered and concentrated. The crude material (0.186 g, 0.330 mmol) was dissolved in THF (5 mL). To the solution was added sodium hydride (60% dispersion in mineral oil) (12 mg, 0.30 mmol). The reaction was stirred for 1 hour under $N_2$, after which methyl iodide (20 μL, 0.32 mmol) was added. The reaction was stirred overnight at room temperature. Saturated aqueous ammonium chloride was added and the reaction mixture was extracted with two portions of dichloromethane. The combined organic extracts were dried ($Na_2SO_4$), filtered and concentrated. The residue was dissolved in dichloromethane (10 mL) and trifluoroacetic acid (0.5 mL) was added. After 2 hours, the solution was concentrated and the residue was purified by reverse phase chromatography, eluting 10% to 45% acetonitrile in water containing 0.1% trifluoroacetic acid. The product was obtained as its TFA salt and was lyophilized to give COMPOUND 29 (56 mg, 74%) as an off-white solid. Purity (HPLC)>99%; $^1$H NMR (400 MHz, $CD_3OD$)-δ 1.12 (t; J = 6.64 Hz, 3H), 1.24 (t, J = 6.64 Hz, 3H), 2.00-2.09 (m, 2H), 2.44-2.52 (m, 2H), 2.98-3.06 (m, 2H), 3.14-3.21 (m, 2H), 3.23-3.34 (m, 2H), 3.47 (s, 3H), 3.49-3.58 (m, 2H), 6.82 (br s, 1H), 6.91 (m, 1H), 7.06 (d, J = 8.01 Hz, 2H), 7.19-7.38 (m, 9H). Found: C, 52.24; H, 5.27; N, 4.96. $C_{31}H_{35}N_3O_2$ x 2.6 $CF_3CO_2H$ x 3.0 $H_2O$ has C, 52.25; H, 5.28; N, 5.05%.

**COMPOUND 30: 4-[{3-[(anilinocarbonyl)amino]phenyl}(piperidin-4-ylidene)methyl]-*N,N*-diethylbenzamide**

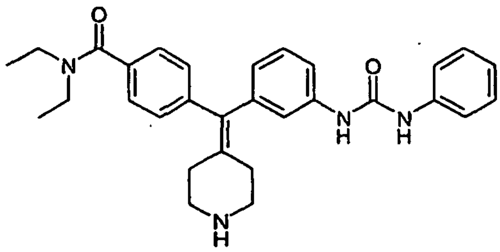

To a solution of the INTERMEDIATE 6 (0.127 g, 0.274 mmol) in DMF was added phenylisocyanate (38 μL, 0.36 mmol). The reaction was stirred overnight at room temperature under nitrogen. The reaction mixture was concentrated and the residue was diluted with dichloromethane. The solution was washed with one portion of saturated aqueous ammonium chloride and the organic phase was dried ($Na_2SO_4$), filtered and concentrated. The residue was dissolved in dichloromethane (5 mL) and trifluoroacetic acid (1 mL) was added. After 4 hours, the solution was concentrated and purified by reverse phase chromatography (gradient 10-45% acetonitrile in water containing 0.1% trifluoroacetic acid). The product was obtained as its TFA salt and was lyophilized to give COMPOUND 30 (123 mg, 75%) as a colourless solid. Purity (HPLC) >99%; $^1$H NMR (400 MHz, $CD_3OD$) δ 1.12 (br t, J = 6.83 Hz, 3H), 1.23 (br t, J = 6.83 Hz, 3H), 2.55-2.67 (m, 4H), 3.22-3.35 (m, 6H), 3.47-4.59 (m, 2H), 6.78-6.82 (m, 1H), 6.99-7.04 (m, 1H), 7.15 (ddd, J = 8.20, 2.15, 0.98 Hz, 1H), 7.23-7.31 (m, 5H), 7.36 (d, J = 8.40 Hz, 2H), 7.38-7.42 (m, 2H), 7.49-7.51 (m, 1H). Found: C, 60.78; H, 5.63; N, 8.66. $C_{30}H_{34}N_4O_2$ x 1.4 $CF_3CO_2H$ x 0.3 $H_2O$ has C, 60.83; H, 5.60; N, 8.65%.

**COMPOUND 31: 4-[(3-{[(benzylamino)carbonyl]amino}phenyl)(piperidin-4-ylidene)methyl]-*N,N*-diethylbenzamide**

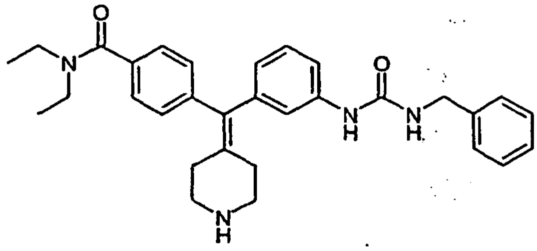

To a solution of INTERMEDIATE 6 (0.350 g, 0.755 mmol) in 1,2-dichloroethane was added benzyl isocyanate (0.14 mL, 1.13 mmol). The reaction was stirred overnight at room temperature under nitrogen. Trifluoroacetic acid (2 mL) was then added. After 4 hours, saturated aqueous sodium bicarbonate was added until bubbling ceased. The layers were separated and the aqueous layer was extracted with two portions of dichloromethane. The combined organic extracts were dried ($Na_2SO_4$), filtered and concentrated. The residue was purified by reverse phase chromatography (gradient 10-45% acetonitrile in water containing 0.1% trifluoroacetic acid). The product was obtained as its TFA salt and was lyophilized to give COMPOUND 31 (201 mg, 44%) as a colourless solid. Purity (HPLC) >99%; $^1$H NMR (400 MHz, $CD_3OD$) δ 1.12 (br t, J = 7.03 Hz, 3H), 1.23 (br t, J = 7.23 Hz, 3H), 2.55-2.65 (m, 4H), 3.21-3.28 (m, 4H), 3.28-3.33 (m, 2H), 3.49-3.59 (m, 2H), 4.37 (s, 2H), 6.75 (ddd, J = 2.73, 1.56, 1.17 Hz, 1H), 7.10 (ddd, J = 8.01, 2.15, 0.98 Hz, 1H), 7.19-7.25 (m, 3H), 7.27 (d, J = 8.40 Hz, 2H), 7.29-7.33 (m, 3H), 7.35 (d, J = 8.40 Hz, 2H), 7.42-7.45 (m, 1H). Found: C, 60.76; H, 5.81; N, 8.35. $C_{31}H_{36}N_4O_2$ x 1.4 $CF_3CO_2H$ x 0.7 $H_2O$ has C, 60.69; H, 5.85; N, 8.38%.

**CONIPOUND 32: *N*-{3-[{4-[(diethylamino)carbonyl]phenyl}(piperidin-4-ylidene)methyl]phenyl}piperidine-1-carboxamide**

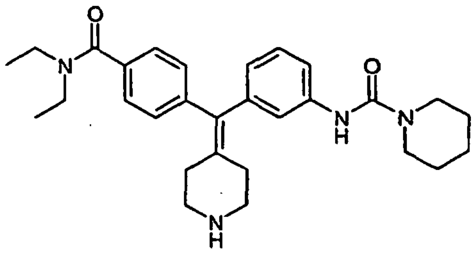

To a solution of INTERMEDIATE 6 (0.113 g, 0.244 mmol) in pyridine (8 mL) was added 1-piperidinecarbonyl chloride (34 μL, 0.27 mmol). The reaction was stirred for 18 hours at room temperature under nitrogen. The mixture was concentrated and the residue was diluted with dichloromethane. The solution was washed with 1M HC1 (1x) and the organic layer was dried ($Na_2SO_4$), filtered and concentrated. The residue was dissolved in dichloromethane (10 mL) and trifluoroacetic acid (1 mL) was added. After 2 hours, the reaction mixture was concentrated and the residue was purified by reverse phase chromatography (gradient 10-45% acetonitrile in water containing 0.1% trifluoroacetic acid). The product was obtained as its TFA salt and was lyophilized to give COMPOUND 32 (36 mg, 25%) as a colourless solid. Purity (HPLC) >99%; $^1$H NMR (400 MHz, $CD_3OD$) δ 1.12 (br t, J = 8.81 Hz, 3H), 1.23 (br t, J = 7.81 Hz, 3H), 1.53-1.63 (m, 4H), 1.63-1.72 (m, 2H), 2.52-2.67 (m, 4H), 3.20-3.33 (m, 6H), 3.47-3.50 (m, 4H), 3.54-3.58 (m, 2H), 6.76-6.80 (m, 1H), 7.1 S-7.20 (m, 1 H), 7.20-7.25 (m, 1H), 7.27 (d, J = 8.40 Hz, 2H), 7.29-7.31 (m, 1H), 7.35 (d, J = 8.40 Hz, 2H). Found: C, 56.68; H, 6.02; N, 7.93. $C_{29}H_{38}N_4O_2$ x 1.8 $CF_3CO_2H$ x 0.6 $H_2O$ has C, 56.69; H, 5.98; N, 8.11 %.

**COMPOUND 33: *N,N*-diethyl-4-[{3-[(phenylsulfonyl)amino]phenyl}(piperidin-4-ylidene)methyl]benzamide**

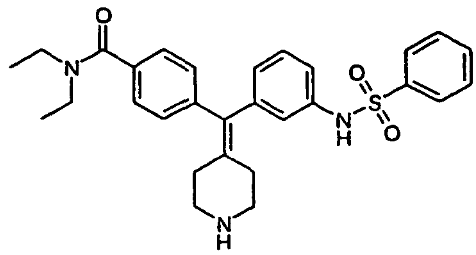

A solution of INTERMEDIATE 6 (0.119 g, 0.257 mmol), benzenesulfonyl chloride (36 μL, 0.28 mmol) and triethylamine (0.11 mL, 0.77 mmol) in dichloromethane (10 mL) was stirred overnight at room temperature under nitrogen. Trifluoroacetic acid (1 mL) was added and the reaction was stirred for an additional 2 hours. Saturated aqueous sodium bicarbonate was added until bubbling ceased. The layers were separated and the aqueous layer was extracted with two portions of dichloromethane. The combined organic extracts were dried ($Na_2SO_4$), filtered and concentrated. The residue was purified by reverse phase chromatography, eluting 10% to 45% acetonitrile in water containing 0.1% trifluoroacetic acid. The product was obtained as its TFA salt and was lyophilized to give COMPOUND 33 (63 mg, 40%) as a colourless solid. Purity (HPLC) >99%; $^1$H NMR (400 MHz, $CD_3OD$) δ 1.09 (t, J = 6.44 Hz, 3H), 1.20 (t, J = 7.23 Hz, 3H), 2.40-2.46 (m, 2H), 2.49-2.56 (m, 2H), 3.13-3.24 (m, 6H), 3.46-3.55 (m, 2H), 6.82 (ddd, J = 7.62, 1.56, 0.98 Hz, 1H), 6.89 (m, 1H), 6.94 (m, 1H), 7.11-7.18 (m, 3H), 7.32 (d, J = 8.40 Hz, 2H), 7.38-7.45 (m, 2H), 7.52 (m, 1H), 7.59-7.64 (m, 2H). Found: C, 44.05; H, 4.28; N, 4.04. $C_{29}H_{33}N_3O_3S$ x 4.0 $CF_3CO_2H$ x 2.7 $H_2O$ has C, 44.07; H, 4.24; N, 4.17%.

**COMPOUND 34: 4-[{3-[(benzylsulfonyl)amino]phenyl}(piperidin-4-ylidene)methyl]-*N,N*-diethylbenzamide**

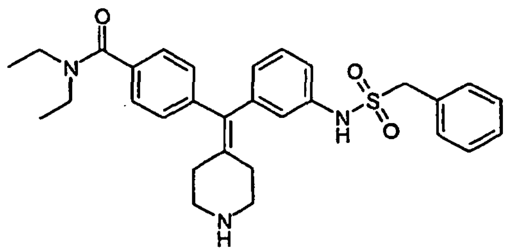

Using the same method as for COMPOUND 33 and using INTERMEDIATE 6 (0.104 g, 0.225 mmol) and α-toluenesulfonyl chloride (47 mg, 0.25 mmol) provided COMPOUND 34 (64 mg, 45%) as a colourless solid. Purity (HPLC): >99%; $^1$H NMR (400 MHz, $CD_3OD$) δ 1.09 (t, J = 6.83 Hz, 3H), 1.22 (t, J = 7.03 Hz, 3H), 2.55-2.62 (m, 4H), 3.21-3.32 (m, 6 H), 3.48-3.58 (m, 2 H), 4.34 (s, 2H), 6.88 (m, 1H), 6.96 (m, 1H), 7.04 (dd, J = 2.15, 0.98 Hz, 1H), 7.19-7.35 (m, 8H), 7.38 (d, J = 8.20 Hz, 2H). Found: C, 48.57; H, 5.04; N, 4.27. $C_{30}H_{35}N_3O_3S$ x 2.8 $CF_3CO_2H$ x 2.5 $H_2O$ has C, 48.48; H, 4.89; N, 4.76%.

**COMPOUND 35: 4-[(3-anilinophenyl)(piperidin-4-ylidene)methyl]-*N,N*-diethylbenzamide**

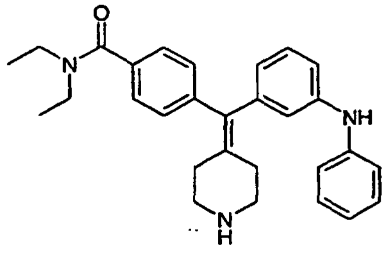

A SmithProcess vial was charged with INTERMEDIATE 6 (0.205 g, 0.442 mmol), bromobenzene (61 μL, 0.58 mmol), $Pd_2(dba)_3$ (16 mg, 0.017 mmol), sodium *tert*-butoxide (60 mg, 0.63 mmol), BINAP (22 mg, 0.035 mmol) and toluene (2.5 mL) and the cap was tightened thoroughly. The vessel was exposed to microwave irradiation in a Smith-Synthesizer for 5 min at 110 °C. The reaction tube was cooled to room temperature and the mixture was absorbed onto silica gel. Flash chromatography (eluting 20% to 50% ethyl acetate in hexanes) afforded the BOC-protected compound as a yellow oil. The oil was dissolved in dichloromethane (15 mL) and trifluoroacetic acid was added (1 mL). After 3 hours of stirring, the reaction was neutralized with 2M NaOH. The phases were separated and the aqueous phase was extracted with two portions of dichloromethane. The combined organic extracts were dried ($Na_2SO_4$), filtered and concentrated to afford the pure product as a yellow oil. The product was dissolved in a 1:5 mixture of dichloromethane/ether (12 mL) and 2.2 mL of 1M HCl in ether was added under an atmosphere of nitrogen. Concentration of the solution provided COMPOUND 35 (208 mg, 92%) as its HCl salt. Purity (HPLC) >99%; $^1H$ NMR (400 MHz, $CD_3OD$) δ 1.12 (br t, J = 6.64 Hz, 3H), 1.24 (br t, J = 6.64 Hz, 3H), 2.55-2.62 (m, 2H), 2.63-2.70 (m, 2H), 3.21-3.35 (m, 6H), 3.50-3.58 (m, 2H), 6.61-6.67 (m, 1H), 6.81-6.87 (m, 2H), 6.95-7.04 (m, 3H), 7.15-7.22 (m, 3H), 7.27 (d, J = 8.40 Hz, 2H), 7.36 (d, J = 8.40 Hz, 2H). Found: C, 70.20; H, 7.26; N, 8.33. $C_{29}H_{33}N_3O$ x 1.2 HCl x 0.7 $H_2O$ has C, 70.23; H, 7.24; N, 8.47%.

**COMPOUND 36: *N,N*-diethyl-4-[{3-[methyl(phenyl)amino]phenyl}(piperidin-4-ylidene)methyl]benzamide**

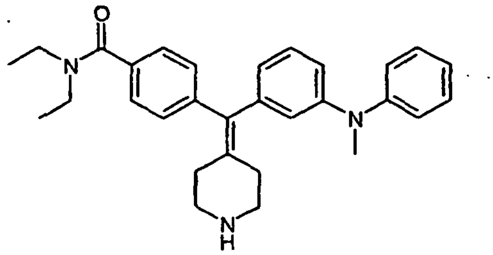

A SmithProcess vial was charged with INTERMEDIATE 6 (0.205 g, 0.442 mmol), bromobenzene (61 μL, 0.58 mmol), $Pd_2(dba)_3$ (16 mg, 0.017 mmol), sodium *tert*-butoxide (60 mg, 0.63 mmol), BINAP (22 mg, 0.035 mmol) and toluene (2.5 mL) and the cap was tightened thoroughly. The vessel was exposed to microwave irradiation in a Smith-Synthesizer for 5 min at 110 °C. The reaction tube was cooled to room temperature and the mixture was absorbed onto silica gel. Flash chromatography (eluting 0% to 50% ethyl acetate in hexanes) afforded the N-arylated intermediate as a yellow oil (0.238 g, 0.441 mmol). The oil was dissolved in dry DMF (10 mL) and NaH (60% dispersion in mineral oil) (42 mg, 1.0 mmol) was added. The reaction was stirred for 1 h, after which iodomethane (69 μL, 1.1 mmol) was added. After 4 h, saturated aqueous $NH_4Cl$ was added. The mixture was diluted with dichloromethane and washed with saturated aqueous $NH_4Cl$. The aqueous layer was extracted with two portions of dichloromethane and the combined organic extracts were dried ($Na_2SO_4$), filtered and concentrated. The residue was then dissolved in dichloromethane (10 mL) and trifluoroacetic acid (1 mL) was added. After 18 h, the solution was concentrated and purified by reverse phase chromatography, eluting 10% to 45% acetonitrile in water containing 0.1 % trifluoroacetic acid. The product was obtained as the trifluoroacetic acid salt and was lyophilized to give COMPOUND 36 (210 mg, 70% yield) as a yellow solid. Purity (HPLC) >99%; $^1H$ NMR (400 MHz, $CD_3OD$) δ 1.12 (t, J = 7.23 Hz, 3H), 1.23 (t, J = 7.23 Hz, 3H), 2.51-2.57 (m, 2H), 2.57-2.63 (m, 2H), 3.16-3.23 (m, 4H), 3.25 (s, 3H), 3.26-3.34 (m, 2H), 3.48-3.58 (m, 2H), 6.64-6.69 (m, 1H), 6.69-6.72 (m, 1H), 6.89 (ddd, J = 8.40, 2.54, 0.98 Hz, 1H), 6.95-7.00 (m, 1H), 7.00-7.03 (m, 2H), 7.19-7.28 (m, 5H), 7.34 (d, J = 8.40 Hz, 2H). Found: C, 63.98; H, 6.26; N, 6.79. $C_{30}H_{35}N_3O$ x 1.3 $CF_3CO_2H$ x 0.6 $H_2O$ has C, 63.91; H, 6.17; N, 6.86%.

**COMPOUND 37: *N,N*-diethyl-4-[{3-[ethyl(phenyl)amino]phenyl}(piperidin-4-ylidene)methyl]benzamide**

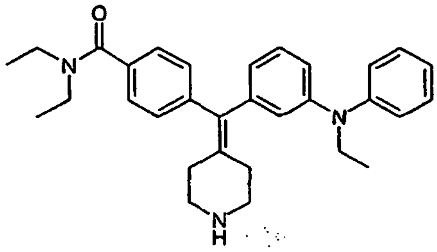

A SmithProcess vial was charged with INTERMEDIATE 6 (0.400 g, 0.863 mmol), bromobenzene (118 μL, 1.12 mmol), $Pd_2(dba)_3$ (32 mg, 0.035 mmol), sodium *tert*-butoxide (116 mg, 0.1.21 mmol), BINAP (43 mg, 0.069 mmol) and toluene (3.0 mL) and the cap was tightened thoroughly. The vessel was exposed to microwave irradiation in a Smith-Synthesizer for 5 min at 110 °C. The reaction tube was cooled to room temperature and the mixture was absorbed onto silica gel. Flash chromatography (eluting 0% to 50% ethyl acetate in hexanes) afforded the N-arylated intermediate as a yellow oil (0.424 g, 0.786 mmol). The oil was dissolved in dry DMF (20 mL) and NaH (60% dispersion in mineral oil) (75 mg, 1.9 mmol) was added. The reaction was stirred for 30 minutes, after which ethyl iodide (0.16 mL, 2.0 mmol) was added. After 4 h, the reaction mixture was diluted with ethyl acetate and washed with saturated aqueous $NH_4Cl$. The organic layer was dried ($Na_2SO_4$), filtered and concentrated. The residue was then dissolved in dichloromethane (10 mL) and trifluoroacetic acid (2 mL) was added. After 18 h, the solution was concentrated and purified by reverse phase chromatography eluting 10% to 45% acetonitrile in water containing 0.1% trifluoroacetic acid. The product was obtained as the trifluoroacetic acid salt and was lyophilized to give COMPOUND 37 (321 mg, 70% yield) as a yellow solid. Purity (HPLC) >99%; $^1H$ NMR (400 MHz, $CD_3OD$) δ 1.12 (br t, J = 6.64 Hz, 3H), 1.15 (t, J = 7.03 Hz, 3H), 1.24 (br t, J = 7.42 Hz, 3H), 2.52-2.57 (m, 2H), 2.58-2.63 (m, 2H), 3.16-3.24 (m, 4H), 3.24-3.34 (m, 2H), 3.49-3.59 (m, 2H), 3.76 (q, J = 7.03 Hz, 2H), 6.61-6.66 (m, 2H), 6.86 (ddd, J = 8.40, 2.54, 0.98 Hz, 1H), 6.95-6.98 (m, 1H), 6.99-7.03 (m, 2H), 7.17-7.28 (m, 5H), 7.34 (d, J = 8.40 Hz, 2H). Found: C, 63.42; H, 6.03; N, 6.53. $C_{31}H_{37}N_3O$ x 1.5 $CF_3CO_2H$ x 0.3 $H_2O$ has C, 63.40; H, 6.12; N, 6.52%.

**INTERMEDIATE 7: *tert*-butyl 4-[{4-[(diethylamino)carbonyl]phenyl}(3-hydroxyphenyl)methylene]piperidine-1-carboxylate**

A solution of INTERMEDIATE 5 (4.08 g, 9.04 mmol), 3-hydroxyphenylboronic acid (1.97 g, 14.3 mmol) and aqueous 2N sodium carbonate (11.3 mL, 22.6 mmol) in a 1:1 mixture of toluene/ethanol (200 mL) was degassed for 20 minutes. Palladium tetrakistriphenylphosphine (1.05 g, 0.909 mmol) was added and the reaction mixture was purged with nitrogen and heated to 90 °C. After 5 h, the reaction was cooled to room temperature and saturated aqueous ammonium chloride was added. The mixture was extracted with two portions of ethyl acetate and the combined organic extracts were dried ($Na_2SO_4$), filtered and concentrated. The residue was purified by flash chromatography eluting with 0% to 100% ethyl acetate in hexanes to yield INTERMEDIATE 7 as a white solid (4.24 g, 100%). $^1H$ NMR (400MHz, $CDCl_3$) δ 1.10 (t, J = 7.42 Hz, 3H), 1.20 (t, J = 7.03 Hz, 3H), 1.42 (s, 9H), 2.25-2.33 (m, 4H), 3.23-3.31 (m, 2H), 3.39-3.46 (m, 4H), 3.46-3.54 (m, 2H), 6.51 (dd, J = 2.15, 1.56 Hz, 1H), 6.57 (ddd, J = 7.62, 1.5 6, 0.98 Hz, 1H), 6.62 (ddd, J = 8.20, 2.54, 0.98 Hz, 1H), 7.06-7.12 (m, 1H), 7.19 (d, J = 8.40 Hz, 2H), 7.29 (d, J = 8.40 Hz, 2H).

**INTERMEDIATE 8: *tert*-butyl 4-[{4-[(diethylamino)carbonyl]phenyl}(3-{[(trifluoromethyl)sulfonyl]oxy}phenyl) methylene]piperidine-1-carboxylate**

To a solution of INTERMEDIATE 6 (1.95 g, 4.20 mmol) and triethylamine (3.00 mL, 21.5 mmol) in dichloromethane (15 mL) was added trifluoromethanesulfonic anhydride (0.95 mL, 5.65 mmol) dropwise at 0 °C. The reaction was allowed to warm to room temperature and was stirred for 15 h. The solution was diluted with dichloromethane and was washed with two portions of saturated aqueous ammonium chloride, then dried ($Na_2SO_4$), filtered and concentrated. The residue was purified by flash chromatography (eluting 20% to 50% ethyl acetate in hexanes) to yield INTERMEDIATE 8 (2.00 g, 80%) as a yellow foam. $^1H$ NMR (400MHz, $CDCl_3$) δ 1.08-1.18 (m, 3H), 1.20-1.29 (m, 3H), 1.47 (s, 9H), 2.29-2.38 (m, 4H), 3.23-3.34 (m, 2H), 3.44-3.50 (m, 4H), 3.50-3.60 (m, 2H), 7.02-7.05 (m, 1 H), 7.09-7.18 (m, 4H), 7.34 (d, J = 8.01 Hz, 2H), 7.36-7.41 (m, 1H).

**COMPOUND 38: *N,N*-diethyl-4-[(3-{[(1S)-1-phenylethyl]amino}phenyl)(piperidin-4-ylidene)methyl]benzamide**

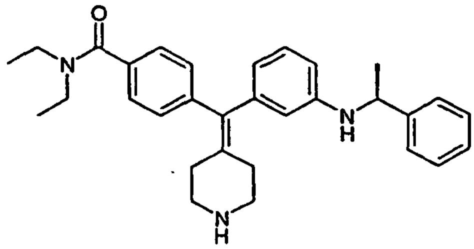

Into an oven-dried test tube were weighed $Pd_2(dba)_3$ (7.6 mg, 0.0083 mmol), BINAP (10.7 mg, 0.0172 mmol) and sodium *tert*-butoxide (24.9 mg, 0.260 mmol). The solid reagents were suspended in degassed toluene (3 mL). The test tube was sealed with a rubber septum and purged with nitrogen. (S)-(-)-1-phenylethylamine (30 μL, 0.23 mmol) was added to the mixture by syringe. The reaction was heated at 80 °C for 5 minutes. A solution of INTERMEDIATE 8 (97 mg, 0.16 mmol) was added dropwise by syringe over a 15 minute period. The reaction was stirred for 8 hours, then cooled to room temperature. The mixture was purified by flash chromatography eluting with 20% to 50% ethyl acetate in hexanes to give 66 mg of a yellow oil. The oil was dissolved in dichloromethane (8 mL) and trifluoroacetic acid (0.5 mL) was added. After 4 hours, the reaction mixture was concentrated and the residue was purified by reverse phase chromatography eluting 10% to 45% acetonitrile in water containing 0.1% trifluoroacetic acid. The product was obtained as its TFA salt and was lyophilized to give COMPOUND 38 (88 mg, 78%) as a colourless solid. Purity (HPLC) >99%; Optical purity (Chiral HPLC): > 99%; $^1$H NMR (400 MHz, $CD_3OD$) δ 1.12 (br t, J = 7.23 Hz, 3H), 1.23 (br t, J = 7.23 Hz, 3H), 1.51 (d, J = 6.83 Hz, 3H), 2.29-2.36 (m, 2H), 2.46-2.52 (m, 2H), 2.90-3.09 (m, 2H), 3.09-3.22 (m, 2H), 3.24-3.36 (m, 2H), 3.48-3.59 (m, 2H), 4.49 (q, J = 6.83 Hz, 1H), 6.31-6.34 (m, 1H), 6.36-6.41 (m, 1H), 6.59-6.65 (m, 1H), 7.04 (t, J = 8.01 Hz, 1H), 7.13 (d, J = 8.40 Hz, 2H), 7.17-7.23 (m, 1H), 7.25-7.34 (m, 6H). Found: C, 58.75; H, 5.56; N, 5.83. $C_{31}H_{37}N_30$ x 2.2 $CF_3CO_2H$ x 0.3 $H_2O$ has C, 58.74; H, 5.54; N, 5.80%.

**COMPOUND 39: *N,N*-diethyl-4-[(3-{[(1R)-1-phenylethyl]amino}phenyl)(piperidin-4-ylidene)methyl]benzamide**

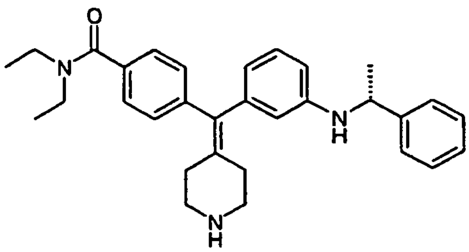

Using the same method as for COMPOUND 38 and using INTERMEDIATE 8 (0.398 g, 0.667 mmol) and (R)-(+)-2-phenylethylamine (0.14 mL, 1.0 mmol) provided COMPOUND 39 (101 mg, 22%) as a colourless solid. Purity (HPLC): >99%; Optical purity (Chiral HPLC): > 99%; $^1$H NMR (400 MHz, $CD_3OD$) δ 1.12 (br t, J = 7.23 Hz, 3H), 1.23 (br t, J = 7.23 Hz, 3H), 1.51 (d,J = 6.83 Hz, 3H), 2.29-2.36 (m, 2H), 2.46-2.52 (m, 2H), 2.90-3.09 (m, 2H), 3.09-3.22 (m, 2H), 3.24-3.36 (m, 2H), 3.48-3.59 (m, 2H), 4.49 (q, J = 6.83 Hz, 1H), 6.31-6.34 (m, 1H), 6.36-6.41 (m, 1H), 6.59-6.65 (m, 1H), 7.04 (t, J = 8.01 Hz, 1H), 7.13 (d, J = 8.40 Hz, 2H), 7.17-7.23 (m, 1H), 7.25-7.34 (m, 6H). Found: C, 49.48; H, 4.89; N, 4.58. $C_{31}H_{37}N_3O$ x 3.7 $CF_3CO_2H$ x 2.4 $H_2O$ has C, 49.45; H, 4.92; N, 4.50%.

**COMPOUND 40: 4-[(3-{[(1R)-1-cyclohexylethyl]amino}phenyl)(piperidin-4-ylidene)methyl]-*N,N*-diethylbenzamide**

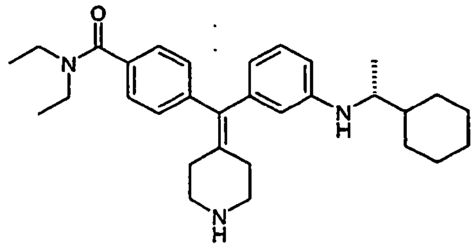

In a 2-necked flask equipped with a reflux condenser were added $Pd_2(dba)_3$ (30 mg, 0.033 mmol), BINAP (42 mg, 0.067 mmol) and sodium *tert*-butoxide (96 mg, 1.00 mmol). The solid materials were suspended in degassed toluene (10 mL). (R)-(-)-1-cyclohexylethylamine (0.14 mL, 0.94 mmol) was added by syringe. The reaction was heated at 80 °C for 5 minutes. A solution of INTERMEDIATE 8 (0.400 g, 0.670 mmol) was added dropwise by syringe over a 20 minute period. The reaction was stirred for 15 h and then cooled to room temperature. The mixture was purified by flash chromatography eluting with 0% to 50% ethyl acetate in hexanes to give 111 mg of a yellow oil. The oil was dissolved in dichloromethane (10 mL) and trifluoroacetic acid (1.0 mL) was added. After 4 hours, the reaction mixture was concentrated and the residue was purified by reverse phase chromatography eluting 10% to 45% acetonitrile in water containing 0.1 % trifluoroacetic acid. The product was obtained as its TFA salt and was lyophilized to give COMPOUND 38 (100 mg, 21%) as a colourless solid. Purity (HPLC) >99%; Optical purity (Chiral HPLC): > 99%; $^1H$ NMR (400 MHz, $CD_3OD$) δ 1.08-1.28 (m, 11H), 1.17 (d, J = 6.83 Hz, 3H), 1.52-1.85 (m, 6H), 2.57-2.65 (m, 4H), 3.23-3.34 (m, 6H), 3.41-3.49 (m, 1H), 3.49-3.59 (m, 2H), 7.05-7.08 (m, 1H), 7.19-7.24 (m, 2H), 7.28 (d, J = 8.40 Hz, 2H), 7.38 (d, J = 8.40 Hz, 2H), 7.47-7.53 (m, 1H). Found: C, 58.42; H, 6.22; 5.78. $C_{31}H_{43}N_3O$ x 2.2 $CF_3CO_2H$ x 0.2 $H_2O$ has C, 58.39; H, 6.31; N, 5.77%.

**COMPOUND 41: 4-[(3-{[(1S)-1-cyclohexylethyl]amino}phenyl)(piperidin-4-ylidene)methyl]-*N,N*-diethylbenzamide**

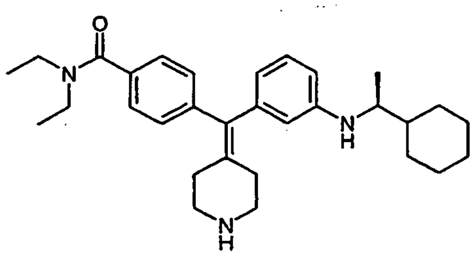

Using the same method as for COMPOUND 40 and using INTERMEDIATE 8 (0.400 g, 0.670 mmol) and (R)-(-)-1-cyclohexylethylamine (0.14 mL, 0.94 mmol) provided COMPOUND 41 (30 mg, 6%) as a colourless solid. Purity (HPLC) >97% (215 nm), >97% (254 nm), >99% (280 nm); Optical purity (Chiral HPLC): > 99%; $^1H$ NMR (400 MHz, $CD_3OD$) δ 1.08-1.28 (m, 11H), 1.17 (d, J = 6.83 Hz, 3H), 1.52-1.85 (m, 6H), 2.57-2.65 (m, 4H), 3.23-3.34 (m, 6H), 3.41-3.49 (m, 1H), 3.49-3.59 (m, 2H), 7.05-7.08 (m, 1H), 7.19-7.24 (m, 2H), 7.28 (d, J = 8.40 Hz, 2H), 7.38 (d, J = 8.40 Hz, 2H), 7.47-7.53 (m, 1H). Found: C, 59.88; H, 6.57; N, 5.98. $C_{31}H_{43}N_3O$ x 1.9 $CF_3CO_2H$ x 0.4 $H_2O$ has C, 59.92; H, 6.60; N, 6.02%.

**COMPOUND 42: *N,N*-diethyl-4-[{3-[(1-methyl-1-phenylethyl)amino]phenyl}(piperidin-4-ylidene)methyl]benzamide**

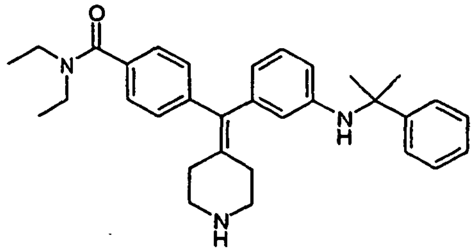

In a 2-necked flask equipped with a reflux condenser were added INTERMEDIATE 8 (0.560 g, 0.939 mmol), $Pd_2(dba)_3$ (43 mg, 0.047 mmol), 2-(di-*t*-butylphosphino)biphenyl (28 mg, 0.094 mmol) and potassium phosphate (0.280 g, 1.32 mmol). The solid materials were suspended in degassed toluene (10 mL). Cumylamine (0.152 g, 1.12 mmol) was added by syringe. The reaction was heated at 80 °C for 18 hours and then cooled to room temperature. The solution was diluted with ethyl acetate and washed with saturated aqueous sodium bicarbonate (1x) and brine (1x). The organic phase was dried ($Na_2SO_4$), filtered and concentrated. The residue was dissolved in dichloromethane (10 mL) and trifluoroacetic acid (2.0 mL) was added. After 4 hours, the reaction mixture was concentrated and the residue was purified by reverse phase chromatography eluting 10% to 45% acetonitrile in water containing 0.1 % trifluoroacetic acid. The product was obtained as its TFA salt and was lyophilized to give COMPOUND 42 (69 mg, 10%) as a colourless solid. Purity (HPLC) >99%; $^1$H NMR (400 MHz, $CD_3OD$) δ 1.13 (br t, J = 7.23 Hz, 3H), 1.24 (br t, J = 6.64 Hz, 3H), 1.76 (s, 6H), 2.33-2.39 (m, 2H), 2.47-2.53 (m, 2H), 3.10-3.16 (m, 2H), 3.16-3.22 (m, 2H), 3.24-3.34 (m, 2H), 3.49-3.59 (m, 2H), 6.33-6.38 (m, 1H), 6.83 (dd, J = 7.81 Hz, 1.37 Hz, 1H), 6.96-7.02 (m, 1H), 7.08 (d, J = 8.40 Hz, 2H), 7.24-7.31 (m, 1H), 7.31-7.39 (m, 5H), 7.41-7.47 (m, 2H). Found: C, 55.34; H, 5.25; N, 5.09. $C_{32}H_{39}N_3O$ x 2.9 $CF_3CO_2H$ x 0.4 $H_2O$ has C, 55.40; H, 5.25; N, 5.13%.

**COMPOUND 43: 4-[{3-[cyclohexyl(methyl)amino]phenyl}(piperidin-4-ylidene)methyl]-*N,N*-diethylbenzamide**

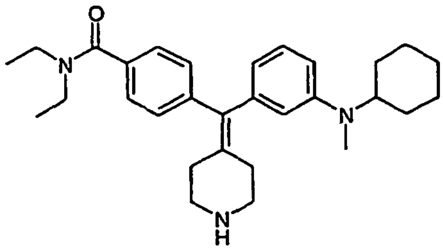

Using the same method as for COMPOUND 40 and using INTERMEDIATE 8 (0.400 g, 0.670 mmol) and *N*-methylcyclohexylamine (130 μL, 0.996 mmol) provided COMPOUND 43 (37 mg, 8%) as a colourless solid. Purity (HPLC) >94% (215 nm), >95% (254 nm), >99% (280 nm); $^1$H NMR (400 MHz, $CD_3OD$) δ 1.06-1.18 (m, 4H), 1.18-1.37 (m, 8H), 1.63-1.71 (m, 1H), 1.81-2.01 (m, 3H), 2.57-2.66 (m, 4H), 3.20-3.24 (s, 3H), 3.25-3.35 (m, 6H), 3.49-3.62 (m, 3H), 7.26-7.30 (m, 3H), 7.35-7.40 (m, 3H), 7.45 (ddd, J = 8.20, 2.34, 0.59 Hz, 1H), 7.56-7.63 (m, 1H). Found: C, 50.16; H, 5.26; N, 5.01. $C_{30}H_{41}N_3O$ x 3.6 $CF_3CO_2H$ x 1.1 $H_2O$ has C, 50.20; H, 5.30; N, 4.72%.

**COMPOUND 44: *N,N*-diethyl-4-[piperidin-4-ylidene(3-piperidin-1-ylphenyl)methyl]benzamide**

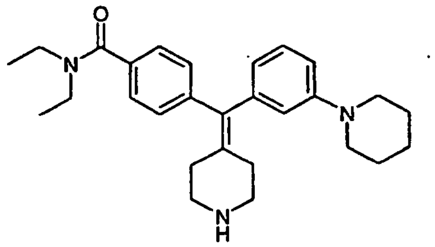

Using the same method as for COMPOUND 40 and using INTERMEDIATE 8 (0.400 g, 0.670 mmol) and piperidine (100 μL, 1.01 mmol) provided COMPOUND 44 (200 mg, 45%) as an orange solid. Purity (HPLC) >99%; $^1$H NMR (400 MHz, $CD_3OD$) δ 1.13(br t, J = 7.03 Hz, 3H), 1.23 (br t, J = 7.23 Hz, 3H), 1.73-1.81 (m, 2H), 1.95-2.04 (m, 4H), 2.55-2.64 (m, 4H), 3.24-3.33 (m, 6H), 3.49-3.61 (m, 6H), 7.28 (d, J = 8.40 Hz, 2H), 7.25-7.40 (m, 3H), 7.42-7.45 (m, 1H), 7.53-7.62 (m, 2H). Found: C, 50.78; H, 5.55; N, 5.14. $C_{28}H_{37}N_3O$ x 2.9 $CF_3CO_2H$ x 2.1 $H_2O$ has C, 50.74; H, 5.56; N, 5.25%.

**COMPOUND 45: *N,N*-diethyl-4-[piperidin-4-ylidene(3-pyrrolidin-1-ylphenyl)methyl]benzamide**

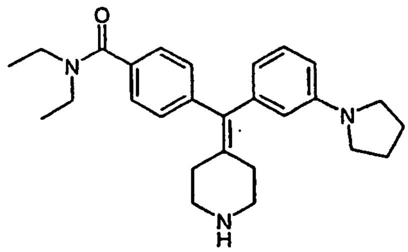

Using the same method as for COMPOUND 40 and using INTERMEDIATE 8 (0.400 g, 0.670 mmol) and pyrrolidine (56 μL, 1.0 mmol) provided COMPOUND 45 (174 mg, 40%) as a yellow solid. Purity (HPLC) >99%; $^1$H NMR (400 MHz, $CD_3OD$) δ 1.12 (br t, J = 7.62 Hz, 3H), 1.24 (br t, J = 7.62 Hz, 3H), 1.98-2.04 (m, 4H), 2.56-2.64 (m, 4H), 3.20-3.34 (m, 10H), 3.49-3.58 (m, 2H), 6.38-6.40 (m, 1H), 6.47-6.51 (m, 1H), 6.57 (ddd, J = 8.01, 2.34, 0.59 Hz, 1H), 7.14-7.20 (m, 1H), 7.28 (d, J = 8.40 Hz, 2H), 7.35 (d, J = 8.40 Hz, 2H). Found: C, 52.86; H, 5.45; N, 5.76. $C_{27}H_{35}N_3O$ x 2.6 $CF_3CO_2H$ x 1.0 $H_2O$ has C, 52.83; H, 5.45; N, 5.74%.

**COMPOUND 46: *N,N*-diethyl-4-[[3-[(2-ethyl-1-oxobutyl)amino]phenyl]-4-piperidinylidenemethyl]-benzamide**

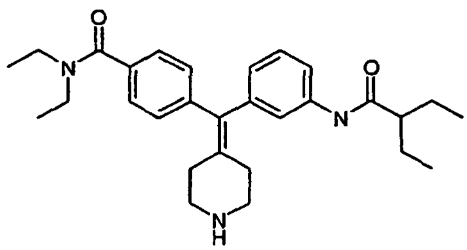

To a solution of INTERMEDIATE 6 (225 mg, 0.485 mmol) and 2-ethylbutyryl chloride (98 mg, 0728 mmol) in 1,2-dichloroethane (20 mL) was added triethylamine (0.203 mL, 1.456 mmol). The reaction was heated overnight at 50 °C, diluted with $CH_2Cl_2$ and washed with saturated aqueous $NaHCO_3$ (1x). The layers were separated and the aqueous layer was extracted with additional $CH_2Cl_2$ (1x). The combined organic phases was dried over $Na_2SO_4$, filtered and

concentrated *in vacuo.* The residue was dissolved in $CH_2Cl_2$ (15 mL) and trifluoroacetic acid (1.5 mL) was added. The reaction was stirred overnight at room temperature then concentrated *in vacuo.* The residue was purified by reverse phase HPLC (gradient 15-40% $CH_3CN$ in $H_2O$ containing 0.1 % trifluoroacetic acid) to give COMPOUND 46 (213 mg, 76% yield) as its TFA salt. This material was lyophilized from $CH_3CN/H_2O$ to produce a colorless solid. Purity (HPLC): > 99%; $^1H$ NMR (400 MHz, $CD_3OD$) δ 0.92 (t, J = 7.32 Hz, 6H), 1.08-1.16 (m, 3H), 1.19-1.28 (m, 3H), 1.45-1.70 (m, 4H), 2.15-2.25 (m, 1H), 2.61 (dt, J = 15.72, 5.91 Hz, 4H), 3.22-3.34 (m, 6H), 3.48-3.58 (m, 2H), 6.90 (d, J = 7.62 Hz, 1H), 7.25-7.32 (m, 3H), 7.36 (d, J = 8.01 Hz, 3H), 7.56 (s, 1H). Found: C, 59.65; H, 5.46; N, 5.91. $C_{31}H_{35}N_3O_2$ x 1.8 $CF_3CO_2H$ x 0.6 $H_2O$ has C, 59.57; H, 5.49; N, 6.02%.

**COMPOUND 47: *N*-[3-[[4-[(diethylamino)carbonyl]phenyl]-4-piperidinylidenemethyl]phenyl]-1-methyl-1*H*-1,2,3-benzotriazole-5-carboxamide**

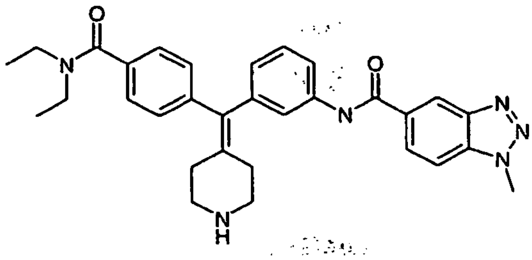

Using the same method as for COMPOUND 46 and using INTERMEDIATE 6 (225 mg, 0.485 mmol) and 1-methyl-1*H*-1,2,3-benzotriazole-5-carbonyl chloride (142 mg, 0.728 mmol) afforded COMPOUND 47 (169 mg, 55% yield) as its TFA salt. This material was lyophilized from $CH_3CN/H_2O$ to produce a colorless solid. Purity (HPLC): > 99%; $^1H$ NMR (400 MHz, $CD_3OD$) δ 1.12 (br t, J = 6.59 Hz, 3H), 1.23 (br t, J = 7.03 Hz, 3H), 2.61 (t, J = 6.15 Hz, 2H), 2.67 (t, J = 5.96 Hz, 2H), 3.24-3.34 (m, 6H), 3.53 (br q, J = 6.48 Hz, 2H), 4.38 (s, 3H), 6.95 (ddd, J = 7.62, 1.56, 1.07 Hz, 1H), 7.30 (d, J = 8.40 Hz, 2H), 7.33-7.39 (m, 3H), 7.54 (ddd, J = 8.15, 2.15, 1.03 Hz, 1H), 7.73 (t, J = 1.81 Hz, 1H), 7.88 (dd, J = 8.79, 0.78 Hz, 1H), 8.11 (dd, J = 8.69, 1.56 Hz, 1H), 8.59 (dd, J= 1.42, 0.83 Hz, 1H). Found: C, 63.88; H, 6.13; N, 6.66. $C_{30}H_{35}N_3O_2$ x 1.1 $CF_3CO_2H$ x 1.0 $H_2O$ has C, 63.79; H, 6.14; N, 6.72 %.

**COMPOUND 48: 6-chloro-*N*-[3-[[4-[(diethylamino)carbonyl]phenyl]-4-piperidinylidenemethyl]phenyl]-3-pyridinecarboxamide**

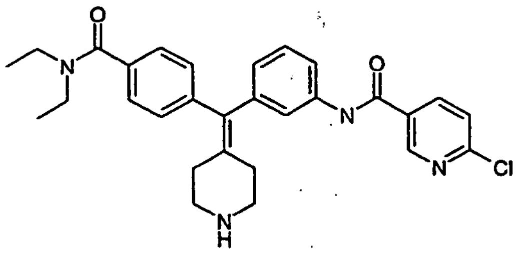

Using the same method as for COMPOUND 46 and using INTERMEDIATE 6 (225 mg, 0.485 mmol) and 6-chloropyridine-3-carbonyl chloride (187 mg, 1.063 mol), except that the reaction was heated for 66 hours, afforded COMPOUND 48 (230 mg, 65% yield) as its TFA salt. This material was lyophilized from $CH_3CN/H_2O$ to produce a colorless solid. Purity (HPLC): > 99%; $^1H$ NMR (400 MHz, $CD_3OD$) δ 1.11 (br t, J = 5.86 Hz, 3H), 1.23 (br t, J = 6.25 Hz, 3H), 2.60 (t, J = 5.86 Hz, 2H), 2.65 (t, J = 5.86 Hz, 2H), 3.23-3.33 (m, 6H), 3.48-3.58 (m, 2H), 6.96 (d, J = 7.62 Hz, 1H), 7.28 (d, J = 8.01 Hz, 2H), 7.32-7.40 (m, 3H), 7.50 (d, J = 8.01 Hz, 1H), 7.59 (d, J = 8.40 Hz, 1H), 7.68 (s, 1H), 8.27 (dd, J = 8.40, 2.54 Hz, 1H), 8.87 (d, J = 2.15 Hz, 1H). Found: C, 63.88; H, 6.13; N, 6.66. $C_{30}H_{35}N_3O_2$ x 1.1 $CF_3CO_2H$ x 1.0 $H_2O$ has C, 63.79; H, 6.14; N, 6.72 %.

**COMPOUND 49: *N*-[3-[[4-[(diethylamino)carbonyl]phenyl]-4-piperidinylidenemethyl]phenyl]-2-methoxy-benzamide**

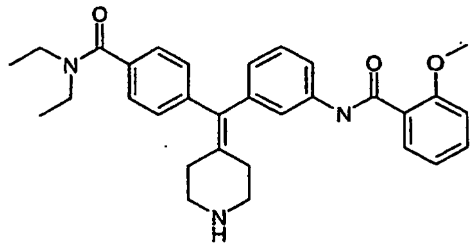

Using the same method as for COMPOUND 46 and using INTERMEDIATE 6 (225 mg, 0.485 mmol) and 2-methoxybenzoyl chloride (124 mg, 0.728 mmol) afforded COMPOUND 49 (221 mg, 75% yield) as its TFA salt. This material was lyophilized from $CH_3CN/H_2O$ to produce a colorless solid. Purity (HPLC): > 99%; $^1H$ NMR (400 MHz, $CD_3OD$) δ 1.12 (br t, J = 6.64 Hz, 3H), 1.23 (br t, J = 6.25 Hz, 3H), 2.61 (t, J = 6.05 Hz, 2H), 2.65 (t, J = 6.05 Hz, 2H), 3.23-3.34 (m, 6H), 3.53 (br q, J = 6.77 Hz, 2H), 4.00 (s, 3H), 6.92 (dt, J = 7.57,1.29 Hz, 1H), 7.08 (dt, J = 7.42, 0.78 Hz, 1H), 7.18 (d, J = 8.40 Hz, 1H), 7.28-7.43 (m, 6H), 7.53 (ddd, J = 8.59, 7.23, 1.76 Hz, 1H), 7.76 (t, J = 1.76 Hz, 1H), 7.85 (dd, J = 7.81, 1.76 Hz, 1H). Found: C, 63.88; H, 6.13; N, 6.66. $C_{30}H_{35}N_3O_2$ x 1.1 $CF_3CO_2H$ x 1.0 $H_2O$ has C, 63.79; H, 6.14; N, 6.72 %.

**COMPOUND 50: *N*-[3-[[4-[(diethylamino)carbonyl]phenyl]-4-piperidinylidenemethyl]phenyl]-2-quinoxalinecarboxamide**

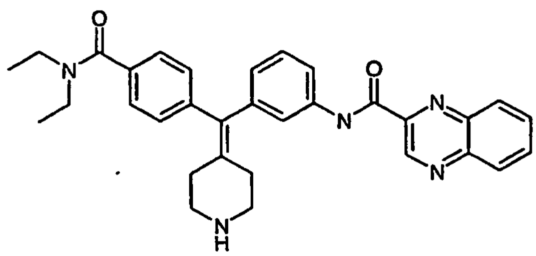

Using the same method as for COMPOUND 46 and using INTERMEDIATE 6 (225 mg, 0.485 mmol) and 2-quinoxaloyl chloride (140 mg, 0.728 mmol) afforded COMPOUND 50 (241 mg, 58% yield) as its TFA salt. This material was lyophilized from $CH_3CN/H_2O$ to produce a colorless solid. Purity (HPLC): > 99%; $^1H$ NMR (400 MHz, $CD_3OD$) δ 1.12 (br t, J = 6.64 Hz, 3H), 1.23 (br t, J = 6.25 Hz, 3H), 2.61 (t, J = 6.05 Hz, 2H), 2.65 (t, J = 6.05 Hz, 2H), 3.23-3.34 (m, 6H), 3.53 (br q, J = 6.77 Hz 2H), 4.00 (s, 3H), 6.92 (dt, J = 7.57, 1.29 Hz, 1H), 7.08 (dt, J = 7.42, 0.78 Hz, 1H), 7.18 (d, J = 8.40 Hz, 1H), 7.28-7.43 (m, 6H), 7.53 (ddd, J = 8.59, 7.23, 1.76 Hz, 1H), 7.76 (t, J = 1.76 Hz, 1H), 7.85 (dd, J = 7.81, 1.76 Hz, 1H). Found: C, 63.88; H, 6.13; N, 6.66. $C_{30}H_{35}N_3O_2$ x 1.1 $CF_3CO_2H$ x 1:0 $H_2O$ has C, 63.79; H, 6.14; N, 6.72 %

**COMPOUND 51: *N*-[3-[[4-[(diethylamino)carbonyl]phenyl]-4-piperidinylidenemethyl]phenyl]-2,5-difluoro-benzamide**

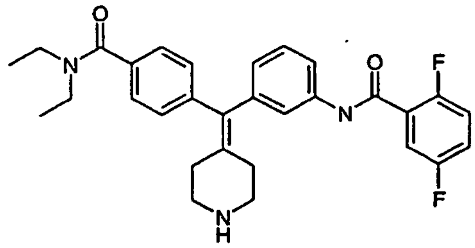

Using the same method as for COMPOUND 46 and using INTERMEDIATE 6 (225 mg, 0.485 mmol) and 2,5-difluorobenzoyl chloride (90 μL, 0.728 mmol) afforded COMPOUND 51 (233 mg, 78% yield) as its TFA salt. This material was lyophilized from $CH_3CN/H_2O$ to produce a colorless solid. Purity (HPLC): > 99%; $^1H$ NMR (400 MHz, $CD_3OD$) δ 1.12 (br t, J = 6.64 Hz, 3H), 1.23 (br t, J = 6.25 Hz, 3H), 2.61 (t, J = 6.05 Hz, 2H), 2.65 (t, J = 6.05 Hz, 2H), 3.23-3.34 (m, 6H), 3.53 (br q, J = 6.77 Hz, 2H), 4.00 (s, 3H), 6.92 (dt, J = 7.57, 1.29 Hz, 1H), 7.08 (dt, J = 7.42, 0.78 Hz, 1H), 7.18 (d, J = 8.40 Hz, 1H), 7.28-7.43 (m, 6H), 7.53 (ddd, J = 8.59, 7.23, 1.76 Hz, 1H), 7.76 (t, J = 1.76 Hz, 1H), 7.85 (dd, J = 7.81, 1.76 Hz, 1H). Found: C, 63.88; H, 6.13; N, 6.66. $C_{30}H_{35}N_3O_2$ x 1.1 $CF_3CO_2H$ x 1.0 $H_2O$ has C, 63.79; H, 6.14; N, 6.72 %.

**COMPOUND 52: 3-chloro-*N*-[3-[[4-[(diethylamino)carbonyl]phenyl]-4-piperidinylidenemethyl]phenyl]-2-thiophenecarboxamide**

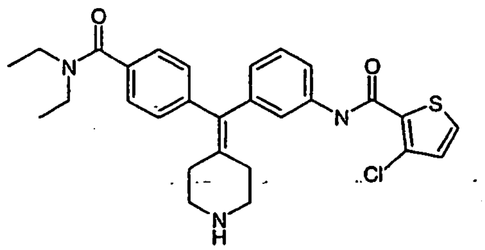

Using the same method as for COMPOUND 46 and using INTERMEDIATE 6 (225 mg, 0.485 mmol) and 3-chlorothiopherie-2-carbonyl chloride (132 mg, 0.728 mmol) afforded COMPOUND 52 (253 mg, 84% yield) as its TFA salt. This material was lyophilized from $CH_3CN/H_2O$ to produce a colorless solid. Purity (HPLC): > 99%; $^1H$ NMR (400 MHz, $CD_3OD$) δ 1.12 (br t, J = 6.64 Hz, 3H), 1.23 (br t, J = 6.25 Hz, 3H), 2.61 (t, J = 6.05 Hz, 2H), 2.65 (t, J = 6.05 Hz, 2H), 3.23-3.34 (m, 6H), 3.53 (br q, J = 6.77 Hz, 2H), 4.00 (s, 3H), 6.92 (dt, J = 7.57, 1.29 Hz, 1H), 7.08 (dt, J = 7.42, 0.78 Hz, 1H), 7.18 (d, J = 8.40 Hz, 1H), 7.28-7.43 (m, 6H), 7.53 (ddd, J = 8.59, 7.23, 1.76 Hz, 1H), 7.76 (t, J = 1.76 Hz, 1H), 7.85 (dd, J = 7.81, 1.76 Hz, 1H). Found: C, 63.88; H, 6.13; N, 6.66. $C_{30}H_{35}N_3O_2$ x 1.1 $CF_3CO_2H$ x 1.0 $H_2O$ has C, 63.79; H, 6.14;N,6.72%.

**COMPOUND 53: *N*-[3-[[4-[(diethylamino)carbonyl]phenyl]-4-piperidinylidenemethyl]phenyl]-3-methyl-benzamide**

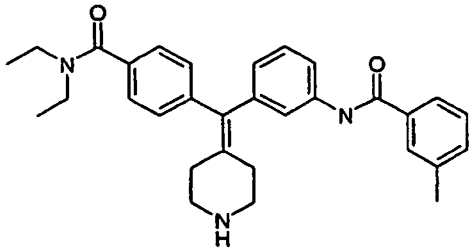

Using the same method as for COMPOUND 46 and using INTERMEDIATE 6 (225 mg, 0.485 mmol) and 3-methylbenzoyl chloride (96 μL, 0.728 mmol) afforded COMPOUND 53 (227 mg, 79% yield) as its TFA salt. This material was lyophilized from $CH_3CN/H_2O$ to produce a colorless solid. Purity (HPLC): > 99%; $^1H$ NMR (400 MHz, $CD_3OD$) δ 1.12 (br t, J = 6.64 Hz, 3H), 1.23 (br t, J = 6.25 Hz, 3H), 2.61 (t, J = 6.05 Hz, 2H), 2.65 (t, J = 6.05 Hz, 2H), 3.23-3.34 (m, 6H), 3.53 (br q, J = 6.77 Hz, 2H), 4.00 (s, 3H), 6.92 (dt, J = 7.57, 1.29 Hz, 1H), 7.08 (dt, J = 7.42, 0.78 Hz, 1H), 7.18 (d, J = 8.40 Hz, 1H); 7.28-7.43 (m, 6H), 7.53 (ddd, J = 8.59, 7.23, 1.76 Hz, 1H), 7.76 (t, J = 1.76.Hz, 1H), 7.85 (dd, J = 7.81, 1.76 Hz, 1H). Found: C, 63.88; H, 6.13; N, 6.66. $C_{30}H_{35}N_3O_2$ x 1: 1 $CF_3CO_2H$ x 1.0 $H_2O$ has C, 63.79; H, 6.14; N, 6.72 %.

**COMPOUND 54: *N,N*-diethyl-4-[[3-[[(methylphenylamino)carbonyl]amino]phenyl]-4-piperidinylidenemethyl]-benzamide**

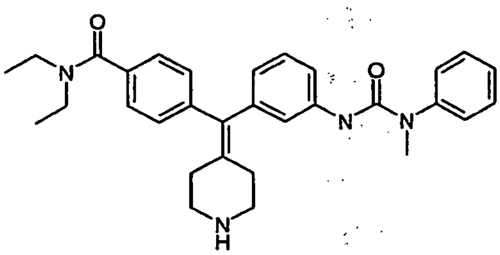

To a solution of INTERMEDIATE 6 (225 mg, 0.485 mmol) and *N*-methyl-*N*-phenylcarbamoyl chloride (370 mg, 2.183 mmol) in 1,2-dichloroethane (20 mL) was added triethylamine (0.203 mL, 1.456 mmol). The reaction was heated for 6 days at 50 °C, diluted with $CH_2Cl_2$ and washed with saturated aqueous $NaHCO_3$ (1x). The layers were separated and the aqueous layer was extracted with additional $CH_2Cl_2$ (1x). The combined organic phases was dried over $Na_2SO_4$, filtered and concentrated *in vacuo.* The residue was dissolved in $CH_2Cl_2$ (15 mL) and trifluoroacetic acid (1.5 mL) was added. The reaction was stirred overnight at room temperature then concentrated *in vacuo.* The residue was purified by reverse phase HPLC (gradient 10-40% $CH_3CN$ in $H_2O$ containing 0.1% trifluoroacetic acid) to give COMPOUND 54 (213 mg, 76% yield) as its TFA salt. This material was lyophilized from $CH_3CN/H_2O$ to produce a colorless solid. Purity (HPLC): > 99%; $^1H$ NMR (400 MHz, $CD_3OD$) δ 1.11 (br t, J = 6.25 Hz, 3H), 1.23 (br t, J = 6.74 Hz, 3H), 2.58 (dt, J = 12.25, 6.08 Hz, 4H), 3.19-3.28 (m, 6H), 3.30 (s, 3H), 3.53 (br q, J = 7.16 Hz, 2H), 6.79 (dt, J = 7.66, 1.42 Hz, 1H), 7.12 (ddd, J = 8.20, 2.15, 1.17 Hz, 1H), 7.19 (d, J = 7.62 Hz, 1H), 7.22-7.26 (m, 2H), 7.29 (t, J = 1.76 Hz, 1H), 7.31-7.36 (m, 5H), 7.42-7.49 (m, 2H). Found: C, 59.65; H, 5.46; N, 5.91. $C_{31}H_{35}N_3O_2$ x 1.8 $CF_3CO_2H$ x 0.6 $H_2O$ has C, 59.57; H, 5.49; N, 6.02%.

## Claims

**1.** A compound of formula I, or a pharmaceutically acceptable salt thereof:

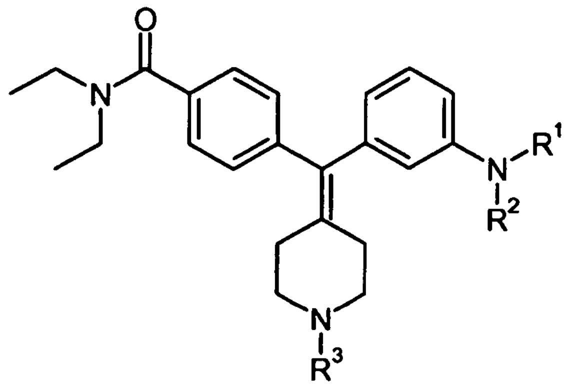

I

wherein

$R^1$ is selected from $C_{3-6}$alkyl, $C_{6-10}$aryl, $C_{2-9}$heteroaryl, $C_{6-10}$aryl-$C_{1-4}$alkyl, $C_{2-9}$heteroaryl-$C_{1-4}$alkyl, $C_{3-10}$cycloalkyl, $C_{3-10}$acycloalkyl-$C_{1-4}$alkyl, $R^8$-C(=O)-, $R^8$-S(=O)$_2$-, $R^8$-S(=O)-, $R^8$-NHC(=O)-, $R^8$-C(=S)- and $R^8$-NH-C(=S)-, wherein $R^8$ is selected from $C_{3-6}$alkyl, $C_{6-10}$aryl, $C_{2-9}$heteroaryl, $C_{6-10}$aryl-$C_{1-4}$alkyl, $C_{2-9}$heteroaryl-$C_{1-4}$alkyl, $C_{3-10}$cycloalkyl, and $C_{3-10}$cycloalkyl-$C_{1-4}$alkyl, wherein said $C_{3-6}$alkyl, $C_{6-10}$aryl, $C_{2-9}$heteroaryl, $C_{6-10}$aryl-$C_{1-4}$alkyl, $C_{2-9}$heteroaryl-$C_{1-4}$alkyl, $C_{3-10}$cycloalkyl, and $C_{3-10}$cycloalkyl-$C_{1-4}$alkyl used in defining $R^1$ and $R^8$ are optionally substituted with one or more groups selected from -R, $-NO_2$, -OR, -Cl, -Br, -I, -F, $-CF_3$, -C(=O)R, -C(=O)OH, $-NH_2$, -SH, -NHR, $-NR_2$, -SR, $-SO_3H$, $-SO_2R$, -S(=O)R, -CN, -OH, -C(=O)OR, -C(=O)$NR_2$, -NRC(=O)R, and -NRC(=O)-OR, wherein R is, independently, selected from -H, $C_{1-6}$alkyl and phenyl;

$R^2$ is selected from -H and $C_{1-6}$alkyl optionally substituted with one or more groups selected from $-CF_3$, -OH, $C_{1-3}$alkoxy, and halogen, or $R^1$ and $R^2$ are $C_{1-3}$alkylene that together form a portion of a ring; and

$R^3$ is selected from -H, $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

**2.** A compound according to claim 1, wherein

$R^1$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl, wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen;

$R^2$ is selected from -H and $C_{1-3}$alkyl; and

$R^3$ is selected from -H and $C_{1-6}$alkyl-O-C(=O)-.

**3.** A compound according to claim 2,
wherein $R^1$ is $R^9$-$CH_2$-, wherein $R^9$ is selected from phenyl, pyridyl, thienyl, furyl, imidazolyl, triazolyl, pyrrolyl, thiazolyl, N-oxido-pyridyl, benzyl, pyridylmethyl, thienylmethyl, furylmethyl, imidazolylmethyl, triazolylmethyl, pyrrolylmethyl, thiazolylmethyl and N-oxido-pyridylmethyl, optionally substituted with one or more groups selected from $C_{1-4}$alkyl, $-CF_3$, -OH, $C_{1-3}$alkoxy, phenoxy and halogen; and
$R^2$ and $R^3$ are hydrogen.

**4.** A compound according to claim 3,
wherein $R^9$ is selected from benzyl, phenyl, pyridyl, thienyl, furyl, imidazolyl, pyrrolyl and thiazolyl, optionally substituted with one or more groups selected from $C_{1-4}$alkyl, $-CF_3$, -OH, $C_{1-3}$alkoxy, phenoxy, and halogen.

**5.** A compound according to claim 4, wherein
$R^9$ is selected from benzyl, phenyl, pyridyl, thienyl, furyl, imidazolyl, pyrrolyl and thiazolyl.

**6.** A compound according to claim 1, wherein

$R^1$ is selected from $C_{3-6}$alkyl, $C_{3-10}$cycloakl, and $C_{3-10}$cycloalkyl-$C_{1-4}$alkyl, wherein said $C_{3-6}$alkyl, $C_{3-10}$cycloalkyl, and $C_{3-10}$cycloalkyl-$C_{1-4}$alkyl are optionally substituted with one or more groups selected from $C_{1-4}$alkyl, -$CF_3$, -OH, $C_{1-3}$alkoxy, phenoxy, and halogen;
$R^2$ is -H or $C_{1-3}$alkyl; and
$R^3$ is -H, $C_{1-6}$alkyl, $C_{3-6}$cycloalkyl, and $C_{3-6}$cycloalkyl-$C_{1-4}$alkyl, wherein said $C_{1-6}$alkyl, $C_{3-6}$cycloalkyl, $C_{3-6}$cycloalkyl-$C_{1-4}$alkyl are optionally substituted with one or more groups selected from $C_{1-4}$alkyl,-$CF_3$, -OH, $C_{1-3}$alkoxy, phenoxy, and halogen.

**7.** A compound according to claim 6, wherein

$R^1$ is selected from 1-propyl, 2-propyl, 1-butyl, 2-butyl, t-butyl, 2-methyl-1-propyl, cyclopentyl, cyclohexyl, cycloheptyl, cyclooctyl, and cyclononyl;
$R^2$ is selected from -H, methyl, ethyl, 1-propyl and 2-propyl; and
$R^3$ is selected from -H, methyl, ethyl, allyl, 3,3-dimethyl-allyl, cyclopropylmethyl, 2-methoxy-ethyl, and 3-methoxy-1-propyl.

**8.** A compound according to claim 1, wherein

$R^1$ is selected from $R^8$-C(=O)-, $R^8$-S(=O)$_2$-, $R^8$-S(=O)-, $R^8$-NHC(=O)-, $R^8$-C(=S)- and $R^8$-NH-C(=S)-, wherein $R^8$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl; wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl are optionally substituted with $C_{1-4}$alkyl, -$CF_3$, -OH, $C_{1-3}$alkoxy, phenoxy, and halogen;
$R^2$ is -H; and
$R^3$ is selected from -H and $C_{1-6}$alkyl-O-C(=O)-.

**9.** A compound according to claim 8, wherein
$R^8$ is selected from phenyl, benzyl, phenethyl and cyclohexyl, wherein said phenyl, benzyl, phenethyl and cyclohexyl are optionally substituted with one or more groups selected from methyl, methoxy and halogen.

**10.** A compound according to claim 1, wherein

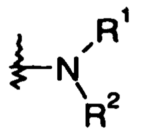

of formula I is selected from

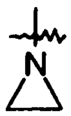 , 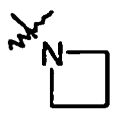 , 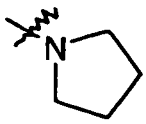 and 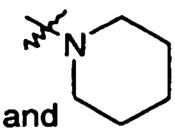 ;

and
$R^3$ is selected from -H and $C_{1-6}$alkyl-O-C(=O)-.

**11.** A compound according to claim 1 selected from:

1) 4-[[3-(benzylamino)phenyl](piperidin-4-ylidene)methyl]-N,N-diethylbenzamide,
2) N,N-diethyl-4-[{3-[(3-furylmethyl)amino]phenyl}(piperidin-4-ylidene)methyl]benzamide,
3) N,N-diethyl-4-(piperidin-4-ylidene{3-[(thien-3-ylmethyl)amino]phenyl}methyl)benzamide,
4) N,N-diethyl-4-[{3-[(2-phenylethyl)amino]phenyl}(piperidin-4-ylidene)methyl]benzamide,

5) 4-[{3-[(4-chlorobenzyl)amino]phenyl}(piperidin-4-ylidene)methyl]-N,N-diethylbenzamide,
6) N,N-diethyl-4-[piperidin-4-ylidene(3-{[3-(trifluoromethyl)benzyl]amino}phenyl)methyl]benzamide,
7) 4-[{3-[(2-chlorobenzyl)amino]phenyl}(piperidin-4-ylidene)methyl]-N,N-diethylbenzamide,
8) N,N-diethyl-4-[piperidin-4-ylidene(3-{[4-(trifluoromethyl)benzyl]amino}phenyl)methyl]benzamide,
9) N,N-diethyl-4-[{3-[(2-furylmethyl)amino]phenyl}(piperidin-4-ylidene)methyl]benzamide,
10) N,N-diethyl-4-(piperidin-4-ylidene{3-[(thien-2-ylmethyl)amino]phenyl}methyl)benzamide,
11) 4-[{3-[(cyclohexylmethyl)amino]phenyl}(piperidin-4-ylidene)methyl]-N,N-diethylbenzamide,
12) N,N-diethyl-4-{piperidin-4-ylidene[3-(propylamino)phenyl]methyl}benzamide,
13) 4-[[3-(cyclohexylamino)phenyl](piperidin-4-ylidene)methyl]-N,N-diethylbenzamide,
14) 4-[[3-(cyclopentylamino)phenyl](piperidin-4-ylidene)methyl]-N,N-diethylbenzamide,
15) 4-[[3-(cycloheptylamino)phenyl](piperidin-4-ylidene)methyl]-N,N-diethylbenzamide,
16) 4-[{3-[cyclopentyl(methyl)amino]phenyl}(piperidin-4-ylidene)methyl]-N,N-diethylbenzamide,
17) 4-[[3-(benzoylamino)phenyl](piperidin-4-ylidene)methyl]-N,N-diethylbenzamide,
18) N,N-diethyl-4-[{3-[(phenylacetyl)amino]phenyl}(piperidin-4-ylidene)methyl]benzamide,
19) 4-[{3-[(cyclohexylcarbonyl)amino]phenyl}(piperidin-4-ylidene)methyl]-N,N-diethylbenzamide,
20) 4-[{3-[(cyclohexylacetyl)amino]phenyl}(piperidin-4-ylidene)methyl]-N,N-diethylbenzamide,
21) 4-[(3-{ [(2-chlorophenyl)acetyl]amino}phenyl)(piperidin-4-ylidene)methyl]-N,N-diethylbenzamide,
22) 4-[(3-{[(3-chlorophenyl)acetyl]amino}phenyl)(piperidin-4-ylidene)methyl]-N,N-diethylbenzamide,
23) N,N-diethyl-4-[(3-{[(5-methylthien-2-yl)acetyl]amino}phenyl)(piperidin-4-ylidene)methyl]benzamide,
24) 4-[(3-{[(5-chlorothien-2-yl)acetyl]amino}phenyl)(piperidin-4-ylidene)methyl]-N,N-diethylbenzamide,
25) N,N-diethyl-4-[(3-{[(2S)-2-phenylpropanoyl]amino}phenyl)(piperidin-4-ylidene)methyl]benzamide,
26) N,N-diethyl-4-[(3-{[(2R)-2-phenylpropanoyl]amino}phenyl)(piperidin-4-ylidene)methyl]benzamide,
27) N,N-diethyl-4-[(3-{[(2S)-2-phenylbutanoyl]amino}phenyl)(piperidin-4-ylidene)methyl]benzamide,
28) N,N-diethyl-4-[(3-{ [(2R)-2-phenylbutanoyl]amino}phenyl)(piperidin-4-ylidene)methyl]benzamide,
29) 4-[{3-[benzoyl(methyl)amino]phenyl}(piperidin-4-ylidene)methyl]-N,N-diethylbenzamide,
30) 4-[{3-[(anilinocarbonyl)amino]phenyl}(piperidin-4-ylidene)methyl]-N,N-diethylbenzamide,
31) 4-[(3-{[(benzylamino)carbonyl]amino}phenyl)(pipendin-4-ylidene)methyl]-N,N-diethylbenzamide,
32) N-{3-[{4-[(diethylamino)carbonyl]phenyl}(piperidin-4-ylidene)methyl]phenyl}piperidine-1-carboxamide,
33) N,N-diethyl-4-[(3-[(phenylsulfonyl)amino]phenyl)(piperidin-4-ylidene)methyl]benzamide,
34) 4-[{3-[(benzylsulfonyl)amino]phenyl}(piperidin-4-ylidene)methyl]-N,N-diethylbenzamide,
35) 4-[(3-anilinophenyl)(piperidin-4-ylidene)methyl]-N,N-diethylbutunimide,
36) N,N-diethyl-4-[{3-[methyl(phenyl)amino]phenyl}(piperidin-4-ylidene)methyl]benzamide,
37) N,N-diethyl-4-[{3-[ethyl(phenyl)amino]phenyl}(piperidin-4-ylidene)methyl]benzamide,
38) N,N-diethyl-4-[(3-{[(1S)-1-phenylethyl]amino}phenyl)(piperidin-4-ylidene)methyl]benzamide.
39) N,N-diethyl-4-[(3-{[(1R)-1-phenylethyl]amino}phenyl)(piperidin-4-ylidene)methyl]benzamide,
40) 4-[(3-{[(1R)-1-cyclohexylethyl]amino}phenyl)(piperidin-4-ylidene)methyl]-N,N-diethylbenzamide,
41) 4-[(3-{[(1S)-1-cyclohexylethyl]amino}phenyl)(piperidin-4-ylidene)methyl]-N,N-diethylbenzamide,
42) N,N-diethyl-4-[{3-[(1-methyl-1-phenylethyl)amino]phenyl}(piperidin-4-ylidene)methyl]benzamide,
43) 4-[{3-[cyclohexyl(methyl)amino]phenyl}(piperidin-4-ylidene)methyl]-N,N-diethylbenzamide,
44) N,N-diethyl-4-[piperidin-4-ylidene(3-piperidin-1-ylphenyl)methyl]benzamide,
45) N,N-diethyl-4-[piperidin-4-ylidene(3-pyrrolidin-1-ylphenyl)methyl]benzamide,
46) N,N-diethyl-4-[[3-[(2-ethyl-1-oxobutyl)amino]phenyl]-4-piperidinylidenemethyl]-benzamide,
47) N-[3-[[4-[(diethylamino)carbonyl]phenyl]-4-piperidinylidenemethyl]phenyl]-1-methyl-1H-1,2,3-benzotriazole-5-carboxamide,
48) 6-chloro-N-[3-[[4-[(diethylamino)carbonyl]phenyl]-4-piperidinylidenemethyl]phenyl]-3-pyridinecarboxamide,
49) N-[3-[[4-[(diethylamino)carbonyl]phenyl]-4-piperidinylidenemethyl]phenyl]-2-methoxy-benzamide,
50) N-[3-[[4-[(diethylamino)carbonyl]phenyl]-4-piperidinylidenemethyl]phenyl]-2-quinoxalinecarboxamide,
51) N-[3-[[4-[(diethylamino)carbonyl]phenyl]-4-piperidinylidenemethyl]phenyl]-2,5-difluoro-benzamide,
52) 3-chloro-N-[3-[[4-[(diethylamino)carbonyl]phenyl]-4-piperidinylidenemethyl]phenyl]-2-thiophenecarboxamide,
53) N-[3-[[4-[(diethylamino)carbonyl]phenyl]-4-piperidinylidenemethyl]phenyl]-3-methyl-benzamide,
54) N,N-diethyl-4-[[3-([(methylphenylamino)carbonyl]amino)phenyl]-4-piperidinylidenemethyl]-benzamide, and pharmaceutically acceptable salts thereof.

**12.** A compound according to any one of claims 1-11 for use as a medicament.

**13.** The use of a compound according to any one of claims 1-11 in the manufacture of a medicament for the therapy of

pain, anxiety or functional gastrointestinal disorders.

**14.** A pharmaceutical composition comprising a compound according to any one of claims 1-11 and a pharmaceutically acceptable carrier.

**15.** A process for preparing a compound of formula III,

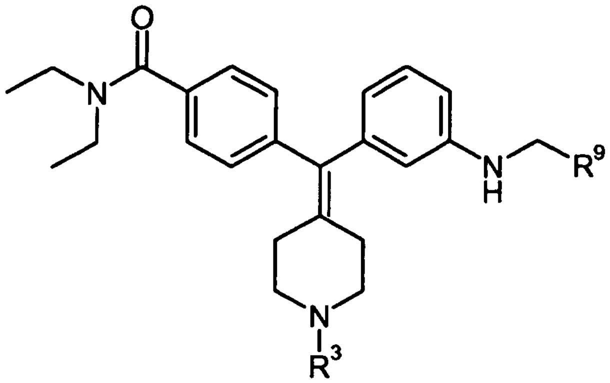

III

comprising:

reacting a compound of formula II,

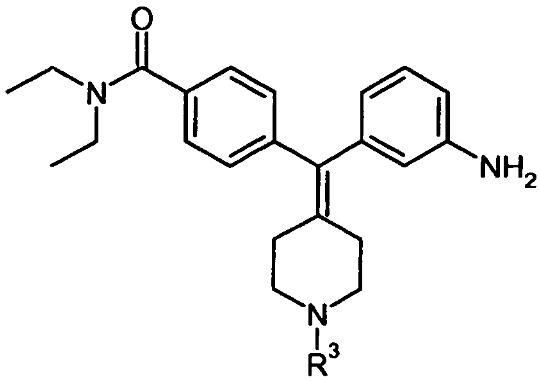

II

with $R^9$-CHO in the presence of a reducing agent to form the compound of formula III,

wherein

$R^9$ is selected from phenyl, pyridyl, thienyl, furyl, imidazolyl, triazolyl, pyrrolyl, thiazolyl, N-oxido-pyridyl, benzyl, pyridylmethyl, thienylmethyl, furylmethyl, imidazolylmethyl, triazolylmethyl, pyrrolylmethyl, thiazolylmethyl and N-oxido-pyridylmethyl, optionally substituted with one or more groups selected from $C_{1-4}$alkyl, $-CF_3$, -OH, $C_{1-3}$alkoxy, phenoxy and halogen; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

**16.** A process for preparing a compound of formula IV,

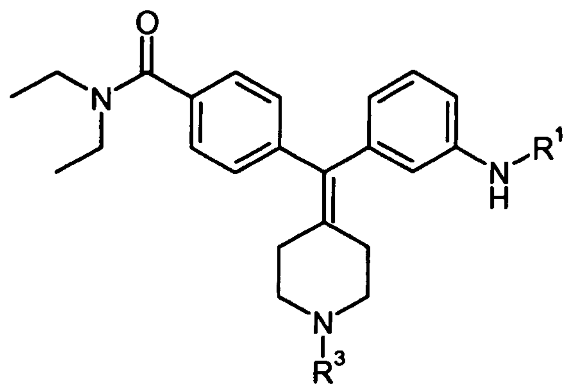

IV

comprising: reacting a compound of formula II,

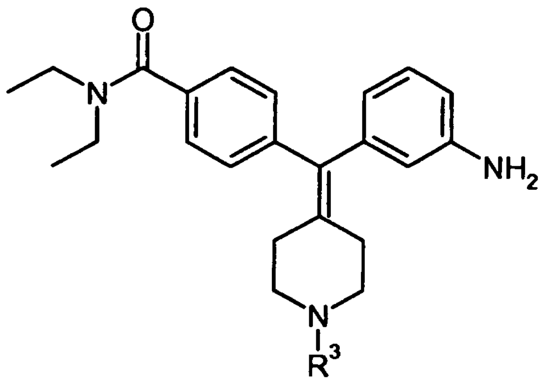

II

with $R^1$-X to form the compound of formula IV,
wherein

X is halogen;
$R^1$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl, wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

**17.** A process for preparing a compound of formula I,

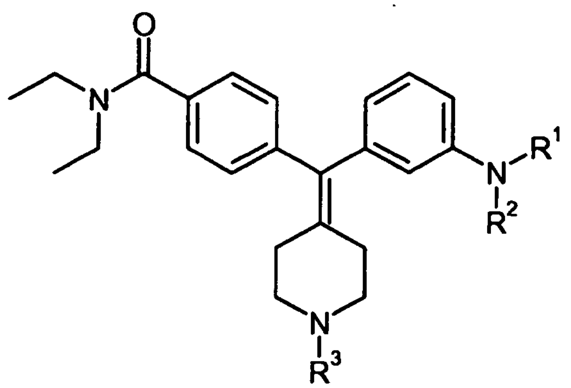

I

comprising: reacting a compound of formula V,

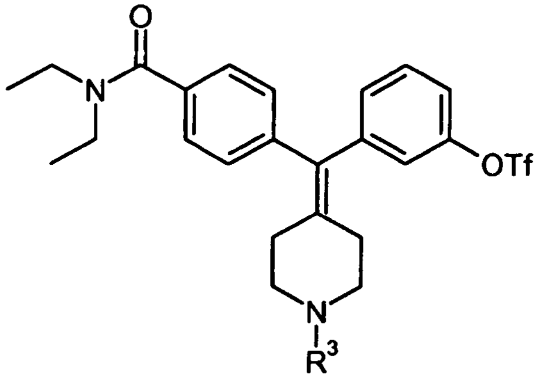

V

with $R^1R^2NH$ to form the compound of formula I,
wherein

$R^1$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl, wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, $C_{3-10}$cycloalkyl-$C_{1-4}$alkyl are optionally substituted with one or more groups selected from $C_{1-4}$alkyl, $-CF_3$, -OH, $C_{1-3}$alkoxy, phenoxy, and halogen;
$R^2$ is selected from -H and $C_{1-6}$alkyl optionally substituted with one or more groups selected from $-CF_3$, -OH, $C_{1-3}$alkoxy, and halogen, or $R^1$ and $R^2$ are Cl-3alkylene that together form a portion of a ring; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

**18.** A process for preparing a compound of formula VI,

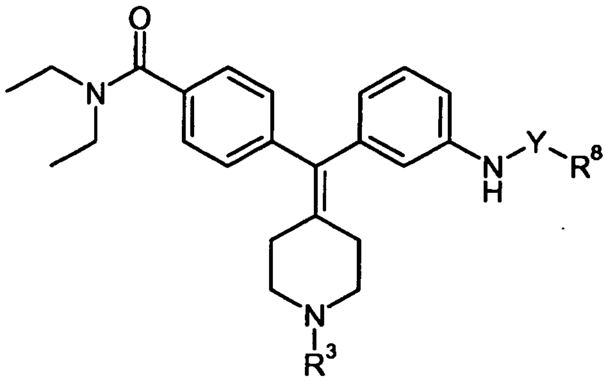

VI

comprising: reacting a compound of formula VII,

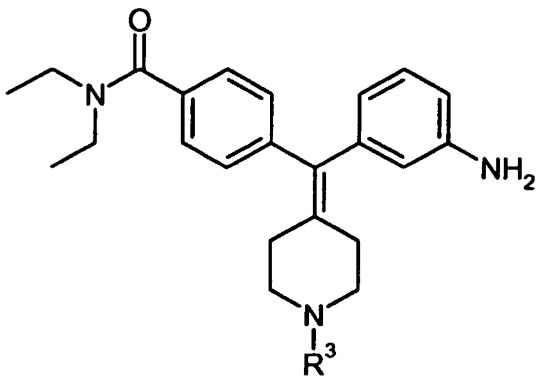

VII

with $R^8$-Y-X or $R^8$-Y-O-Y-$R^8$ to form the compound of formula VI:
wherein

X is halogen;
Y is selected from -C(=O)- and -S(=O)$_2$-;
$R^8$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl; wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl are optionally substituted with $C_{1-4}$alkyl, - $CF_3$, -OH, $C_{1-3}$alkoxy, phenoxy, and halogen; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen.

**19.** A process for preparing a compound of formula VIII,

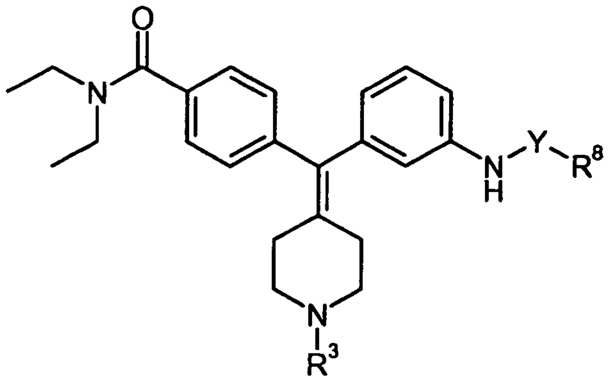

**VIII**

comprising: reacting a compound of formula VII,

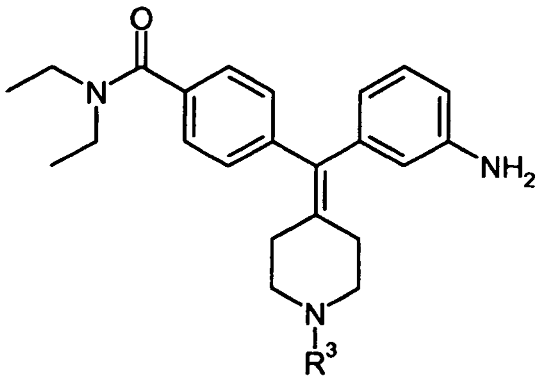

VII

with $R^8$-Z to form the compound of formula VIII:
wherein

Z is selected from -NCO and -NCS;
Y is selected from -C(=O)NH- and -C(=S)NH-;
$R^8$ is selected from $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl; wherein said $C_{3-6}$alkyl, $C_{6-10}$aryl, $C_{2-6}$heteroaryl, $C_{6-10}$aryl-$C_{1-4}$alkyl, $C_{2-6}$heteroaryl-$C_{1-4}$alkyl, $C_{3-10}$cycloalkyl, and $C_{3-10}$cycloalkyl-$C_{1-4}$alkyl are optionally substituted with $C_{1-4}$alkyl, - $CF_3$, -OH, $C_{1-3}$alkoxy, phenoxy, and halogen; and
$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, -$NO_2$, -$CF_3$, $C_{1-6}$alkoxy and halogen.

**20.** A compound of formula V,

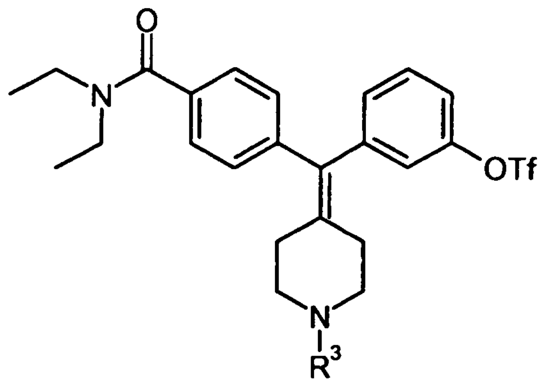

V

wherein

$R^3$ is selected from $C_{1-6}$alkyl-O-C(=O)-, $C_{1-6}$alkyl, $C_{3-6}$cycloalkyl, and $C_{3-6}$cycloalkyl-$C_{1-4}$alkyl, wherein said $C_{1-6}$alkyl-O-C(=O)-, $C_{1-6}$alkyl, $C_{3-6}$cycloalkyl, and $C_{3-6}$cycloalkyl-$C_{1-4}$alkyl are optionally substituted with one or more groups selected from $C_{1-6}$alkyl, halogenated $C_{1-6}$alkyl, $-NO_2$, $-CF_3$, $C_{1-6}$alkoxy and halogen and Tf = $--SO_2CF_3$.

**Patentansprüche**

**1.** Verbindungen der Formel I und deren pharmazeutisch annehmbare Salze:

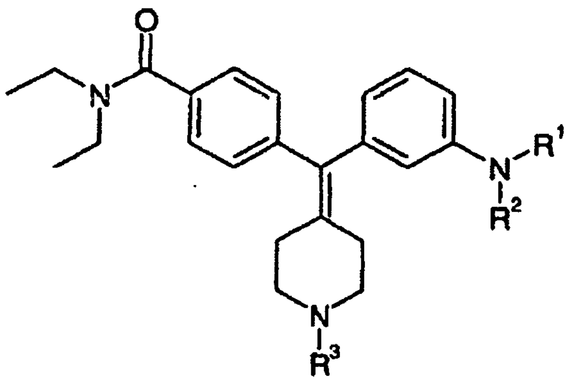

I

wobei

$R^1$ ausgewählt ist aus $C_{3-6}$-Alkyl, $C_{6-10}$-Aryl, $C_{2-9}$-Heteroaryl, $C_{6-10}$-Aryl-$C_{1-4}$-alkyl, $C_{2-9}$-Heteroaryl-$C_{1-4}$-alkyl, $C_{3-10}$-Cycloalkyl, $C_{3-10}$-Cycloalkyl-$C_{1-4}$-alkyl, $R^8$-C(=O) -, $R^8$-S(=O)$_2$-, $R^8$-S(=O) -, $R^8$-NHC(=O)- , $R^8$-C(=S)- und $R^8$-NH-C(=S)- , wobei $R^8$ ausgewählt ist aus $C_{3-6}$-Alkyl, $C_{6-10}$-Aryl, $C_{2-9}$-Heteroaryl, $C_{6-10}$-Aryl-$C_{1-4}$-alkyl, $C_{2-9}$-Heteroaryl-, $C_{1-4}$-alkyl, $C_{3-10}$-Cycloalkyl und $C_{3-10}$-Cycloalkyl-$C_{1-4}$-alkyl, wobei die bei der Definition von $R^1$ und $R^8$ verwendeten $C_{3-6}$-Alkyl-, $C_{6-10}$-Aryl-, $C_{2-9}$-Heteroaryl-, $C_{6-10}$-Aryl-$C_{1-4}$-alkyl-, $C_{2-9}$-Heteroaryl-$C_{1-4}$-alkyl-, $C_{3-6}$-Cycloalkyl und $C_{3-6}$-Cycloalkyl-$C_{1-4}$-alkylreste gegebenenfalls durch eine oder mehrere Gruppen ausgewählt aus -R, $-NO_2$, -OR, -Cl, -Br, -I, -F, $-CF_3$, -C (=O) R, OH, $-NH_2$, -SH, -NHR, $-NR_2$, -SR, $-SO_3H$, $-SO_2R$, -S(=O)R, -CN, -OH, -C(=O)OR, -C(=O)$NR_2$, -NRC(=O)R und -NRC(=O)-OR substituiert sind, wobei die Reste R unabhängig voneinander aus -H, $C_{1-6}$-Alkyl und Phenyl ausgewählt sind;

$R^2$ ausgewählt ist aus -H und gegebenenfalls durch eine oder mehrere Gruppen ausgewählt aus Halogen, $-CF_3$, -OH und $C_{1-3}$-Alkoxy substituierten $C_{1-6}$-Alkyl und Halogen, oder $R^1$ und $R^2$ für $C_{1-3}$-Alkylen stehen, so daß sie zusammen einen Teil eines Rings bilden; und

$R^3$ ausgewählt aus -H, $C_{1-6}$-Alkyl-O-C (=O) -, $C_{1-6}$-Alkyl, $C_{3-6}$-Cycloalkyl und $C_{3-6}$-Cycloalkyl-$C_{1-4}$-alkyl, wobei $C_{1-6}$-Alkyl-O-C (=O) -, $C_{1-6}$-Alkyl, $C_{3-6}$-Cycloalkyl und $C_{3-6}$-Cycloalkyl-$C_{1-4}$-alkyl gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy und Halogen substituiert sind.

**2.** Verbindungen nach Anspruch 1, wobei

$R^1$ ausgewählt ist aus $C_{3-6}$-Alkyl, $C_{6-10}$-Aryl, $C_{2-6}$-Heteroaryl, $C_{6-10}$-Aryl-$C_{1-4}$-alkyl, $C_{2-6}$-Heteroaryl-$C_{1-4}$-alkyl, $C_{3-10}$-Cycloalkyl, $C_{3-10}$-Cycloalkyl-$C_{1-4}$-alkyl, wobei diese $C_{3-6}$-Alkyl-, $C_{6-10}$-Aryl-, $C_{2-6}$-Heteroaryl-, $C_{6-10}$-Aryl-$C_{1-4}$-alkyl-, $C_{2-6}$-Heteroaryl-$C_{1-4}$-alkyl-, $C_{3-10}$-Cycloalkyl-, $C_{3-10}$-Cycloalkyl-$C_{1-4}$-alkylreste gegebenenfalls durch eine oder mehrere Gruppen ausgewählt aus $C_{1-4}$-Alkyl, Halogen, $-CF_3$, -OH, $C_{1-3}$-Alkoxy und Phenoxy substituiert sind, und Halogen;
$R^2$ ausgewählt ist aus -H und $C_{1-3}$-Alkyl; und
$R^3$ ausgewählt ist aus -H und $C_{1-6}$-Alkyl-O-C(=O)-.

**3.** Verbindungen nach Anspruch 2,
wobei $R^1$ für $R^9$-$CH_2$- steht, wobei $R^9$ ausgewählt ist aus Phenyl, Pyridyl, Thienyl, Furyl, Imidazolyl, Triazolyl, Pyrrolyl, Thiazolyl, N-Oxido-pyridyl, Benzyl, Pyridylmethyl, Thienylmethyl, Furylmethyl, Imidazolylmethyl, Triazolylmethyl, Pyrrolylmethyl, Thiazolylmethyl und N-Oxido-pyridylmethyl, gegebenenfalls substiuiert durch eine oder mehrere Gruppen ausgewählt aus $C_{1-4}$-Alkyl, Halogen, $-CF_3$, -OH, $C_{1-3}$-Alkoxy und Phenoxy, und Halogen; und $R^2$ und $R^3$ für Wasserstoff stehen.

**4.** Verbindungen nach Anspruch 3,
wobei $R^9$ ausgewählt ist aus Benzyl, Phenyl, Pyridyl, Thienyl, Furyl, Imidazolyl, Pyrrolyl und Thiazolyl, gegebenenfalls substituiert durch eine oder mehrere Gruppen ausgewählt aus $C_{1-4}$-Alkyl, Halogen, $-CF_3$, -OH, $C_{1-3}$-Alkoxy und Phenoxy, und Halogen.

**5.** Verbindungen nach Anspruch 4,
wobei $R^9$ ausgewählt ist aus Benzyl, Phenyl, Pyridyl, Thienyl, Furyl, Imidazolyl, Pyrrolyl und Thiazolyl.

**6.** Verbindungen nach Anspruch 1, wobei

$R^1$ ausgewählt ist aus $C_{3-6}$-Alkyl, $C_{3-10}$-Cycloalkyl und $C_{3-10}$-Cycloalkyl-$C_{1-4}$-alkyl, wobei diese $C_{3-6}$-Alkyl-, $C_{3-10}$-Cycloalkyl- und $C_{3-10}$-Cycloalkyl-$C_{1-4}$-alkylreste gegebenenfalls durch eine oder mehrere Gruppen ausgewählt aus $C_{1-4}$-Alkyl, Halogen, $-CF_3$, -OH, $C_{1-3}$-Alkoxy und Phenoxy substituiert sind, und Halogen;
$R^2$ für -H oder $C_{1-3}$-Alkyl steht; und
$R^3$ für -H, $C_{1-6}$-Alkyl, $C_{3-6}$-Cycloalkyl oder $C_{3-6}$-Cycloalkyl-$C_{1-4}$-alkyl steht, wobei diese $C_{1-6}$-Alkyl-, $C_{3-6}$-Cycloalkyl- und $C_{3-6}$-Cycloalkyl-$C_{1-4}$-alkylreste gegebenenfalls durch eine oder mehrere Gruppen ausgewählt aus $C_{1-4}$-Alkyl, Halogen, $-CF_3$, -OH, $C_{1-3}$-Alkoxy und Phenoxy substituiert sind, und Halogen.

**7.** Verbindung nach Anspruch 6, wobei

$R^1$ ausgewählt ist aus 1-Propyl, 2-Propyl, 1-Butyl, 2-Butyl, t-Butyl, 2-Methyl-1-propyl, Cyclopentyl, Cyclohexyl, Cycloheptyl, Cyclooctyl und Cyclononyl;
$R^2$ ausgewählt ist aus -H, Methyl, Ethyl, 1-Propyl und 2-Propyl; und
$R^3$ ausgewählt ist aus -H, Methyl, Ethyl, Allyl, 3,3-Dimethylallyl, Cyclopropylmethyl, 2-Methoxyethyl und 3-Methoxy-1-propyl.

**8.** Verbindungen nach Anspruch 1, wobei

$R^1$ ausgewählt ist aus $R^8$-C(=O)-, $R^8$-$S(=O)_2$-, $R^8$-S(=O)-, $R^8$-NHC(=O)-, $R^8$-C(=S)- und $R^8$-NH-C(=S)-, wobei $R^8$ ausgewählt ist aus $C_{3-6}$-Alkyl, $C_{6-10}$-Aryl, $C_{2-6}$-Heteroaryl, $C_{6-10}$-Aryl-$C_{1-4}$-alkyl, $C_{2-6}$-Heteroaryl-$C_{1-4}$-alkyl, $C_{3-10}$-Cycloalkyl und $C_{3-10}$-Cycloalkyl-$C_{1-4}$-alkyl; wobei die $C_{3-6}$-Alkyl-, $C_{6-10}$-Aryl-, $C_{2-6}$-Heteroaryl-, $C_{6-10}$-Aryl-$C_{1-4}$-alkyl-, $C_{2-6}$-Heteroaryl-$C_{1-4}$-alkyl-, $C_{3-6}$-Cycloalkyl- und $C_{3-6}$-Cycloalkyl-$C_{1-4}$-alkylreste gegebenenfalls durch $C_{1-4}$-Alkyl, Halogen, $-CF_3$, -OH, $C_{1-3}$-Alkoxy oder Phenoxy substituiert sind, und Halogen;
$R^2$ für -H steht; und
$R^3$ ausgewählt ist aus -H und $C_{1-6}$-Alkyl-O-C(=O)-.

**9.** Verbindungen nach Anspruch 8, wobei
$R^8$ ausgewählt ist aus Phenyl, Benzyl, Phenethyl und Cyclohexyl, wobei diese Phenyl-, Benzyl-, Phenethyl- und Cyclohexylreste gegebenenfalls durch eine oder mehrere Gruppen ausgewählt aus Methyl, Methoxy und Halogen substituiert sind.

**10.** Verbindungen nach Anspruch 1, wobei

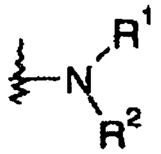

der Formel I ausgewählt ist aus

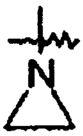, 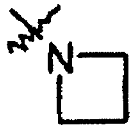, 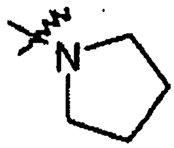 und 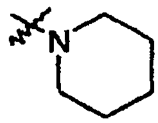 ;

und
$R^3$ ausgewählt ist aus -H und $C_{1-6}$-Alkyl-O-C(=O)-.

**11.** Verbindungen nach Anspruch 1, ausgewählt aus:

1) 4-[[3-(Benzylamino)phenyl](piperidin-4-yliden)methyl]-N,N-diethylbenzamid,
2) N,N-Diethyl-4-[{3-[(3-furylmethyl)amino]phenyl}(piperidin-4-yliden)methyl]benzamid,
3) N,N-Diethyl-4-(piperidin-4-yliden{3-[(thien-3-ylmethyl)amino]phenyl}methyl)benzamid,
4) N,N-Diethyl-4-[{3-[(2-phenylethyl) amino]phenyl}(piperidin-4-yliden) methyl]benzamid,
5) 4-[{3-[(4-Chlorbenzyl)amino]phenyl}(piperidin-4-yliden)methyl]-N,N-diethylbenzamid,
6) N,N-Diethyl-4-[piperidin-4-yliden(3-{[3-(trifluormethyl)benzyl]amino}phenyl) methyl]benzamid,
7) 4-[{3-[(2-Chlorbenzyl)amino]phenyl}(piperidin-4-yliden)methyl]-N,N-diethylbenzamid,
8) N,N-Diethyl-4-[piperidin-4-yliden(3-{[4-(trifluormethyl)benzyl]amino}phenyl) methyl]benzamid,
9) N,N-Diethyl-4-[{3-[(2-furylmethyl) amino]phenyl}(piperidin-4-yliden) methyl]benzamid,
10) N,N-Diethyl-4-(piperidin-4-yliden{3-[(thien-2-ylmethyl)amino]phenyl}methyl)benzamid,
11) 4-[{3-[Cyclohexylmethyl)amino]phenyl}-(piperidin-4-yliden)methyl]-N,N-diethylbenzamid,
12) N,N-Diethyl-4-{piperidin-4-yliden[3-propylamino)phenyl]methyl}benzamid,
13) 4-[[3-(Cyclohexylamino)phenyl](piperidin-4-yliden)methyl]-N,N-diethylbenzamid,
14) 4-[[3-(Cyclopentylamino)phenyl](piperidin-4-yliden)methyl]-N,N-diethylbenzamid,
15) 4-[[3-(Cycloheptylamino)phenyl](piperidin-4-yliden)methyl]-N,N-diethylbenzamid,
16) 4-[{3-[Cyclopentyl(methyl)amino]phenyl}(piperidin-4-yliden)methyl]-N,N-diethylbenzamid,
17) 4-[[3-(Benzoylamino)phenyl](piperidin-4-yliden)methyl]-N,N-diethylbenzamid,
18) N,N-Diethyl-4-[{3-[(phenylacetyl)amino]phenyl}(piperidin-4-yliden)methyl]benzamid,
19) 4-[{3-[(Cyclohexylcarbonyl)amino]phenyl}(piperidin-4-yliden)methyl]-N,N-diethylbenzamid,
20) 4-[{3-[(Cyclohexylacetyl)amino]phenyl}(piperidin-4-yliden)methyl]-N,N-diethylbenzamid,
21) 4-[(3-{[(2-Chlorphenyl)acetyl]amino}phenyl)(piperidin-4-yliden)methyl]-N,N-diethylbenzamid,
22) 4-[(3-{[(3-Chlorphenyl)acetyl]amino}phenyl)(piperidin-4-yliden)methyl]-N,N-diethylbenzamid,
23) N,N-Diethyl-4-[(3-{[(5-methylthien-2-yl)acetyl]amino}phenyl)(piperidin-4-yliden)methyl]benzamid,
24) 4-[(3-{[(5-Chlorthien-2-yl)acetyl]amino}phenyl)(piperidin-4-yliden)methyl]-N,N-diethylbenzamid,
25) N,N-Diethyl-4-[(3-{[(2S)-2-phenylpropanoyl]amino}phenyl)(piperidin-4-yliden)methyl]benzamid,
26) N,N-Diethyl-4-[(3-{[(2R)-2-phenylpropanoyl]amino}phenyl)(piperidin-4-yliden)methyl]benzamid,
27) N,N-Diethyl-4-[(3-{[(2S)-2-phenylbutanoyl]amino}phenyl)(piperidin-4-yliden)methyl]benzamid,
28) N,N-Diethyl-4-[(3-{[(2R)-2-phenylbutanoyl]amino}phenyl)(piperidin-4-yliden)methyl]benzamid,

29) 4-[{3-[Benzoyl(methyl)amino]phenyl}(piperidin-4-yliden)methyl]-N,N-diethylbenzamid,
30) 4-[{3-[(Anilincarbonyl)amino]phenyl}(piperidin-4-yliden)methyl]-N,N-diethylbenzamid,
31) 4-[(3-{[(Benzylamino)carbonyl]amino}phenyl)(piperidin-4-yliden)methyl]-N,N-diethylbenzamid,
32) N-{3-[{4-[(Diethylamino)carbonyl]phenyl}(piperidin-4-yliden)methyl]phenyl}piperidin-1-carboxamid,
33) N,N-Diethyl-4-[{3-[(phenylsulfonyl)amino]phenyl}(piperidin-4-yliden)methyl]benzamid,
34) 4-[{3-[(Benzylsulfonyl)amino]phenyl}(piperidin-4-yliden)methyl]-N,N-diethylbenzamid,
35) 4-[(3-Anilinophenyl)(piperidin-4-yliden)methyl]-N,N-diethylbenzamid,
36) N,N-Diethyl-4-[{3-[methyl(phenyl)amino]phenyl}(piperidin-4-yliden)methyl]benzamid,
37) N,N-Diethyl-4-[{3-[ethyl(phenyl)amino]phenyl}(piperidin-4-yliden)methyl]benzamid,
38) N,N-Diethyl-4-[(3-{[(1S)-1-phenylethyl]amino}phenyl)(piperidin-4-yliden)methyl]benzamid,
39) N,N-Diethyl-4-[(3-{[(1R)-1-phenylethyl]amino}phenyl)(piperidin-4-yliden)methyl]benzamid,
40) 4-[(3-{[(1R)-1-Cyclohexylethyl]amino}phenyl)(piperidin-4-yliden)methyl]-N,N-diethylbenzamid,
41) 4-[(3-{[(1S)-1-Cyclohexylethyl]amino}phenyl)(piperidin-4-yliden)methyl]-N,N-diethylbenzamid,
42) N,N-Diethyl-4-[{3-[(1-methyl-1-phenylethyl)amino]phenyl}(piperidin-4-yliden)methyl]benzamid,
43) 4-[{3-[Cyclohexyl (methyl) amino] phenyl} (piperidin-4-yliden)methyl]-N,N-diethylbenzamid,
44) N,N-Diethyl-4-[piperidin-4-yliden(3-piperidin-1-ylphenyl)methyl]benzamid,
45) N,N-Diethyl-4-[piperidin-4-yliden(3-pyrrolidin-1-ylphenyl)methyl]benzamid,
46) N,N-Diethyl-4-[[3-[(2-ethyl-1-oxobutyl)amino]phenyl](-4-piperidinylidenmethyl]benzamid,
47) N[3-[[4-[(Diethylamino)carbonyl]phenyl]-4-piperidinylidenmethyl]phenyl]-1-methyl-1H-1,2,3-benzotriazol-5-carboxamid,
48) 6-Chlor-N-[3-[[4-[(diethylamino)carbonyl]phenyl]-4-piperidinylidenmethyl]phenyl]-3-pyridincarboxamid,
49) N-[3-[[4-[(Diethylamino)carbonyl]phenyl]-4-piperidinylidenmethyl]phenyl]-2-methoxybenzamid,
50) N-[3-[[4-[(Diethylamino)carbonyl]phenyl]-4-piperidinylidenmethyl]phenyl]-2-chinoxalincarboxamid,
51) N-[3-[[4-[(Diethylamino)carbonyl]phenyl]-4-piperidinylidenmethyl]phenyl]-2,5-difluorbenzamid,
52) 3-Chlor-N-[3-[[4-[(diethylamino)carbonyl]phenyl]-4-piperidinylidenmethyl]phenyl]-2-thiophencarboxamid,
53) N-[3-[[4-[(Diethylamino)carbonyl]phenyl]-4-piperidinylidenmethyl]phenyl]-3-methylbenzamid,
54) N,N-Diethyl-4-[[3-[[(methylphenylamino)carbonyl]amino]phenyl]-4-piperidinylidenmethyl]phenyl]benzamid
und deren pharmazeutisch annehmbaren Salzen.

**12.** Verbindungen nach einem der Ansprüche 1-11 zur Verwendung als Medikament.

**13.** Verwendung einer Verbindung nach einem der Ansprüche 1-11 bei der Herstellung eines Medikaments für die Therapie von Schmerzen, Angst oder funktionellen gastrointestinalen Erkrankungen.

**14.** Pharmzeutische Zusammensetzung, enthaltend eine Verbindung nach einem der Ansprüche 1-11 und einen pharmazeutisch annehmbaren Träger.

**15.** Verfahren zur Herstellung einer Verbindung der Formel III

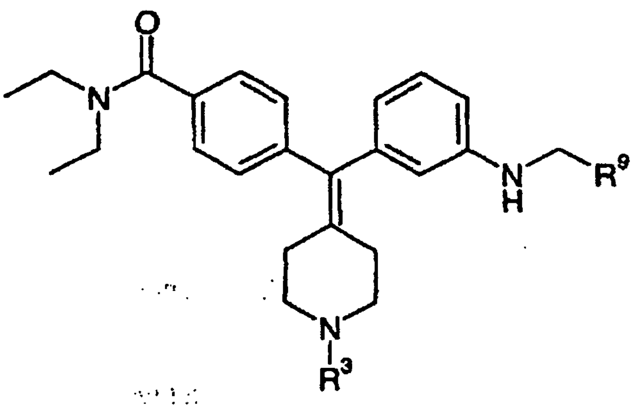

III

bei dem man:

eine Verbindung der Formel II

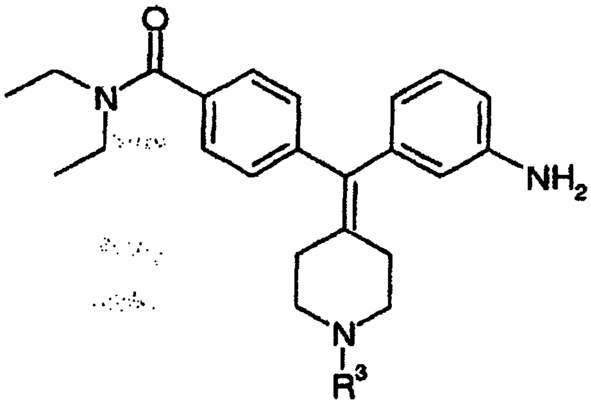

II

in Gegenwart eines Reduktionsmittels unter Bildung der Verbindung der Formel III mit $R^9$-CHO umsetzt

wobei

$R^9$ ausgewählt ist aus Phenyl, Pyridyl, Thienyl, Furyl, Imidazolyl, Triazolyl, Pyrrolyl, Thiazolyl, N-Oxido-pyridyl, Benzyl, Pyridylmethyl, Thienylmethyl, Furylmethyl, Imidazolylmethyl, Triazolylmethyl, Pyrrolylmethyl, Thiazolylmethyl und N-Oxido-pyridylmethyl, gegebenenfalls substituiert durch eine oder mehrere Gruppen ausgewählt aus $C_{1-4}$-Alkyl, Halogen, -$CF_3$, -OH, $C_{1-3}$-Alkoxy, Phenoxy und Halogen; und
$R^3$ ausgewählt ist aus $C_{1-6}$-Alkyl-O-C(=O)-, $C_{1-6}$-Alkyl, $C_{3-6}$-Cycloalkyl und $C_{3-6}$-Cycloalkyl-$C_{1-4}$-alkyl, wobei diese $C_{1-6}$-Alkyl-O-C(=O)-, $C_{1-6}$-Alkyl-, $C_{3-6}$-Cycloalkyl- und $C_{3-6}$-Cycloalkyl-$C_{1-4}$-alkyl-Reste gegebenenfalls durch eine oder mehrere Gruppen ausgewählt aus $C_{1-6}$-Alkyl, halogeniertem $C_{1-6}$-Alkyl, -$NO_2$, -$CF_3$, $C_{1-6}$-Alkoxy und Halogen substituiert sind.

**16.** Verfahren zur Herstellung einer Verbindung der Formel IV

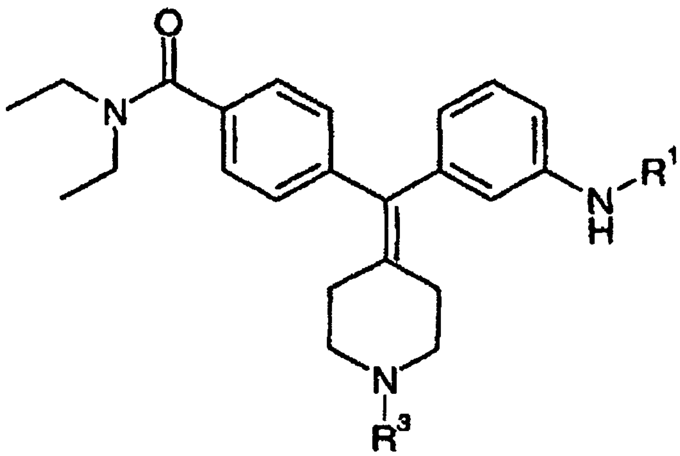

IV

bei dem man: eine Verbindung der Formel II

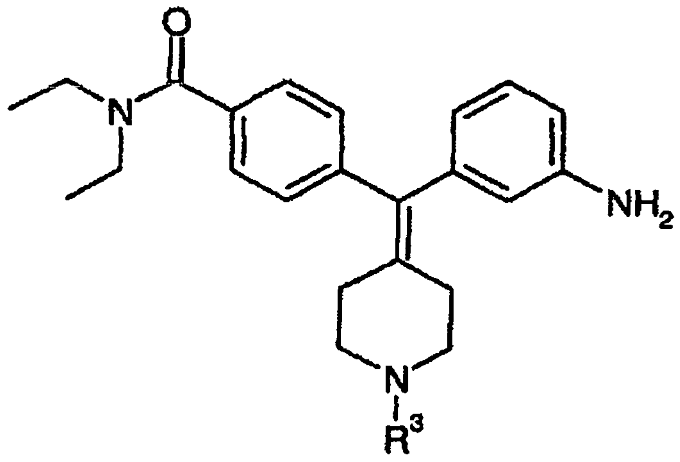

**II**

unter Bildung der Verbindung der Formel IV mit $R^1$-X umsetzt, wobei

X für Halogen steht;
$R^1$ ausgewählt ist aus $C_{3-6}$-Alkyl, $C_{6-10}$-Aryl, $C_{2-6}$-Heteroaryl, $C_{6-10}$-Aryl-$C_{1-4}$-alkyl, $C_{2-6}$-Heteroaryl-$C_{1-4}$-alkyl, $C_{3-10}$-Cycloalkyl, $C_{3-10}$-Cycloalkyl-$C_{1-4}$-alkyl, wobei diese $C_{3-6}$-Alkyl-, $C_{6-10}$-Aryl-, $C_{2-6}$-Heteroaryl-, $C_{6-10}$-Aryl-$C_{1-4}$-alkyl-, $C_{2-6}$-Heteroaryl-$C_{1-4}$-alkyl-, $C_{3-10}$-Cycloalkyl-, $C_{3-10}$-Cycloalkyl-$C_{1-4}$-alkylreste gegebenenfalls durch eine oder mehrere Gruppen ausgewählt aus $C_{1-4}$-Alkyl, Halogen, -$CF_3$,- OH, $C_{1-3}$-Alkoxy und Phenoxy substituiert sind, und Halogen; und
$R^3$ ausgewählt ist aus $C_{1-6}$-Alkyl-O-C(=O)-, $C_{1-6}$-Alkyl, $C_{3-6}$-Cycloalkyl und $C_{3-6}$-Cycloalkyl-$C_{1-4}$-alkyl, wobei diese $C_{1-6}$-Alkyl-O-C (=O) -, $C_{1-6}$-Alkyl-, $C_{3-6}$-Cycloalkyl- und $C_{3-6}$-Cycloalkyl-$C_{1-4}$-alkylreste gegebenenfalls durch eine oder mehrere Gruppen ausgewählt aus $C_{1-6}$-Alkyl, halogeniertem $C_{1-6}$-Alkyl, -$NO_2$, -$CF_3$, $C_{1-6}$-Alkoxy und Halogen substituiert sind.

**17.** Verfahren zur Herstellung einer Verbindung der Formel I

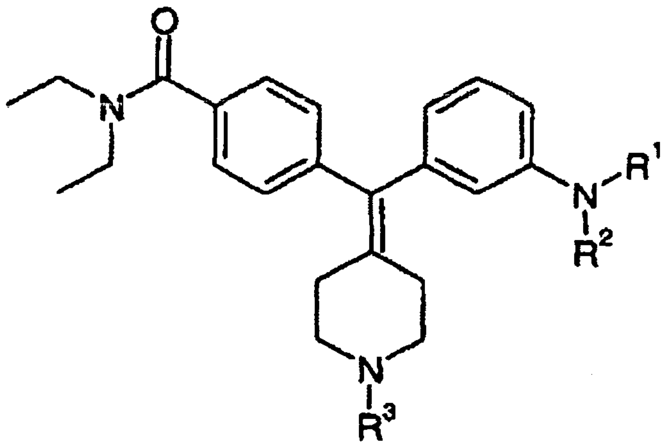

**I**

bei dem man: eine Verbindung der Formel V

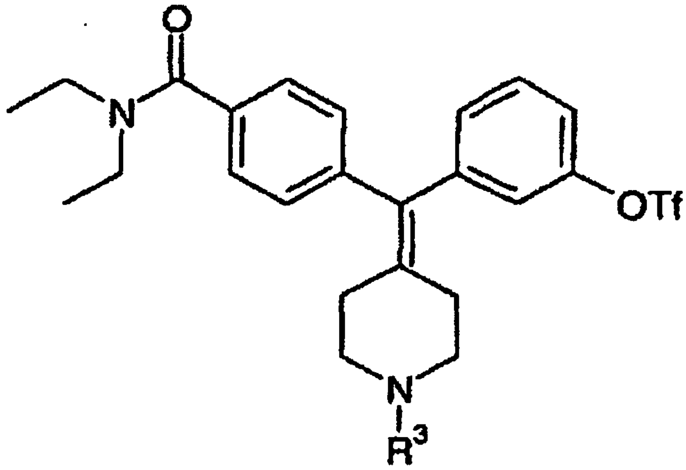

V

unter Bildung der Verbindung der Formel I mit $R^1R^2NH$ umsetzt, wobei

$R^1$ ausgewählt ist aus $C_{3-6}$-Alkyl, $C_{6-10}$-Aryl, $C_{2-6}$-Heteroaryl, $C_{6-10}$-Aryl-$C_{1-4}$-alkyl, $C_{2-6}$-Heteroaryl-$C_{1-4}$-alkyl, $C_{3-10}$-Cycloalkyl, $C_{3-10}$-Cycloalkyl-$C_{1-4}$-alkyl, wobei diese $C_{3-6}$-Alkyl-, $C_{6-10}$-Aryl-, $C_{2-6}$-Heteroaryl-, $C_{6-10}$-Aryl-$C_{1-4}$-alkyl-, $C_{2-6}$-Heteroaryl-$C_{1-4}$-alkyl-, $C_{3-10}$-Cycloalkyl-, $C_{3-10}$-Cycloalkyl-$C_{1-4}$-alkylreste gegebenenfalls durch eine oder mehrere Gruppen ausgewählt aus $C_{1-4}$-Alkyl, Halogen, $-CF_3$, -OH, $C_{1-3}$-Alkoxy und Phenoxy substituiert sind, und Halogen;
$R^2$ ausgewählt ist aus -H und gegebenenfalls durch eine oder mehrere Gruppen ausgewählt aus Halogen, $-CF_3$, -OH und $C_{1-3}$-Alkoxy substituierten $C_{1-6}$-Alkyl und Halogen, oder $R^1$ und $R^2$ für $C_{1-3}$-Alkylen stehen, so daß sie zusammen einen Teil eines Rings bilden; und
$R^3$ ausgewählt ist aus $C_{1-6}$-Alkyl-O-C(=O)-, $C_{1-6}$-Alkyl, $C_{3-6}$-Cycloalkyl und $C_{3-6}$-Cycloalkyl-$C_{1-4}$-alkyl, wobei diese $C_{1-6}$-Alkyl-O-C(=O)-, $C_{1-6}$-Alkyl-, $C_{3-6}$-Cycloalkyl- und $C_{3-6}$-Cycloalkyl-$C_{1-4}$-alkylreste gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy und Halogen substituiert sind.

**18.** Verfahren zur Herstellung einer Verbindung der Formel VI

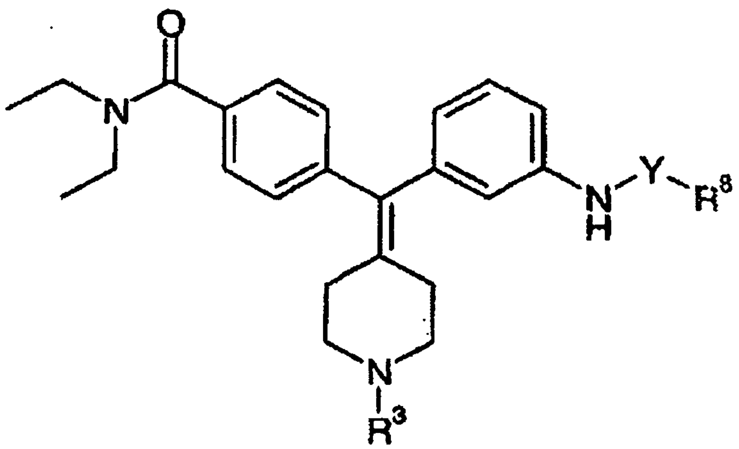

VI

bei dem man: eine Verbindung der Formel VII

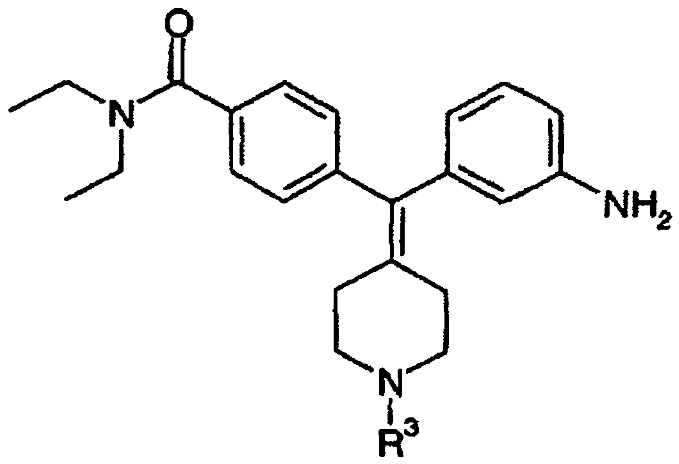

VII

unter Bildung der Verbindung der Formel VI mit $R^8$-Y-X oder $R^8$-Y-O-Y-$R^8$ umsetzt:
wobei

X für Halogen steht;
Y ausgewählt ist aus -C(=O)- und -S(=O)$_2$-;
$R^8$ ausgewählt ist aus $C_{3-6}$-Alkyl, $C_{6-10}$-Aryl, $C_{2-6}$-Heteroaryl, $C_{6-10}$-Aryl-$C_{1-4}$-alkyl, $C_{2-6}$-Heteroaryl-$C_1$-4-alkyl, $C_{3-10}$-Cycloalkyl und $C_{3-10}$-Cycloalkyl-$C_{1-4}$-alkyl, wobei diese $C_{3-6}$-Alkyl-, $C_{6-10}$-Aryl-, $C_{2-6}$-Heteroaryl-, $C_{6-10}$-Aryl-$C_{1-4}$-alkyl-, $C_{2-6}$-Heteroaryl-$C_{1-4}$-alkyl-, $C_{3-6}$-Cycloalkyl- und $C_{3-6}$-Cycloalkyl-$C_{1-4}$-alkylreste gegebenenfalls durch $C_{1-4}$-Alkyl, Halogen, -$CF_3$, -OH, $C_{1-3}$-Alkoxy oder Phenoxy substituiert sind, und Halogen; und
$R^3$ ausgewählt ist aus $C_{1-6}$-Alkyl-O-C(=O)-, $C_{1-6}$-Alkyl, $C_{3-6}$-Cycloalkyl und $C_{3-6}$-Cycloalkyl-$C_{1-4}$-alkyl, wobei diese $C_{1-6}$-Alkyl-O-C(=O)-, $C_{1-6}$-Alkyl-, $C_{3-6}$-Cycloalkyl- und $C_{3-6}$-Cycloalkyl-$C_{1-4}$-alkylreste gegebenenfalls durch eine oder mehrere Gruppen ausgewählt aus $C_{1-6}$-Alkyl, halogeniertem $C_{1-6}$-Alkyl, -$NO_2$, -$CF_3$, $C_{1-6}$-Alkoxy und Halogen substituiert sind.

**19.** Verfahren zur Herstellung einer Verbindung der Formel VIII

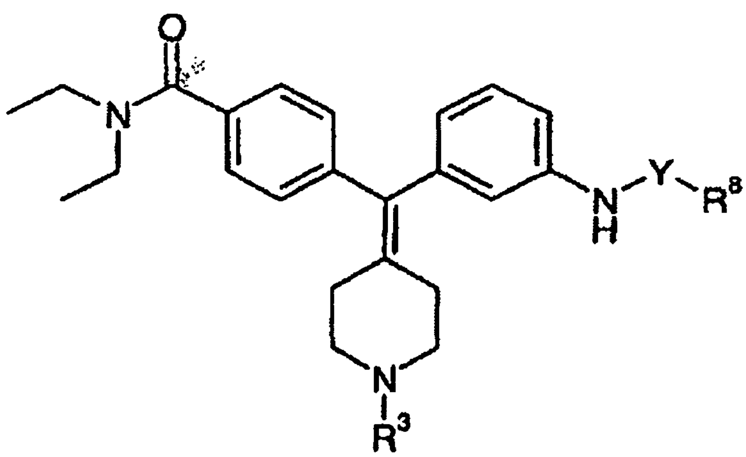

VIII

bei dem man: eine Verbindung der Formel VII

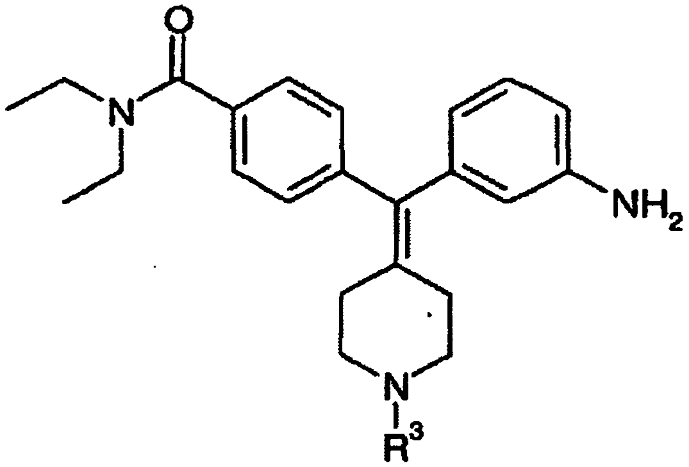

VII

unter Bildung der Verbindung der Formel VIII mit $R^8$-Z umsetzt:
wobei

Z ausgewählt ist aus -NCO und -NCS;
Y ausgewählt ist aus -C(=O)NH- und -C(=S)NH-;
$R^8$ ausgewählt ist aus $C_{3-6}$-Alkyl, $C_{6-10}$-Aryl, $C_{2-6}$-Heteroaryl, $C_{6-10}$-Aryl-$C_{1-4}$alkyl, $C_{2-6}$-Heteroaryl-$C_{1-4}$-alkyl, $C_{3-10}$-Cycloalkyl und $C_{3-10}$-Cycloalkyl-$C_{1-4}$-alkyl, wobei diese $C_{3-6}$-Alkyl-, $C_{6-10}$-Aryl-, $C_{2-6}$-Heteroaryl-, $C_{6-10}$-Aryl-$C_{1-4}$alkyl-, $C_{2-6}$-Heteroaryl-$C_{1-4}$-alkyl-, $C_{3-6}$-Cycloalkyl- und $C_{3-6}$-Cycloalkyl-$C_{1-4}$-alkylreste gegebenenfalls durch $C_{1-4}$-Alkyl, Halogen, $-CF_3$, -OH, $C_{1-3}$-Alkoxy oder Phenoxy substituiert sind, und Halogen; und
$R^3$ ausgewählt ist aus $C_{1-6}$-Alkyl-O-C (=O) -, $C_{1-6}$-Alkyl, $C_{3-6}$-Cycloalkyl und $C_{3-6}$-Cycloalkyl-$C_{1-4}$-alkyl, wobei diese $C_{1-6}$-Alkyl-O-C(=O)-, $C_{1-6}$-Alkyl-, $C_{3-6}$-Cycloalkyl- und $C_{3-6}$-Cycloalkyl-$C_{1-4}$-alkylreste gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy und Halogen substituiert sind.

**20.** Verbindungen der Formel V

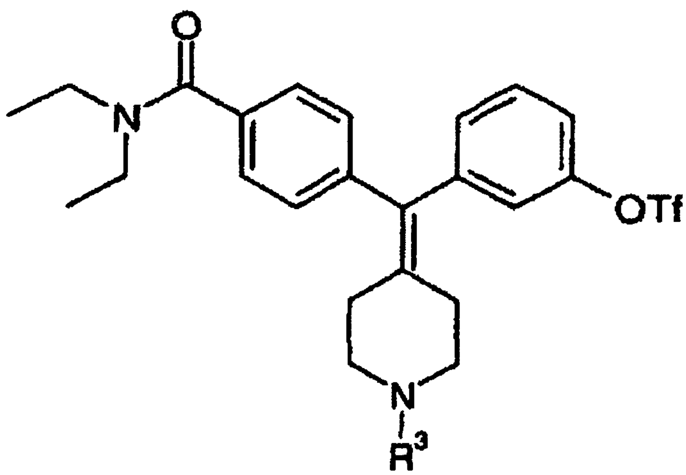

V

in denen

$R^3$ ausgewählt ist aus $C_{1-6}$-Alkyl-O-C (=O) -, $C_{1-6}$-Alkyl, $C_{3-6}$-Cycloalkyl und $C_{3-6}$-Cycloalkyl-$C_{1-4}$-alkyl, wobei diese $C_{1-6}$-Alkyl-O-C(=O)-, $C_{1-6}$-Alkyl-, $C_{3-6}$-Cycloalkyl- und $C_{3-6}$-Cycloalkyl-$C_{1-4}$-alkylreste gegebenenfalls durch eine oder mehrere Gruppen ausgewählt aus $C_{1-6}$-Alkyl, halogeniertem $C_{1-6}$-Alkyl, $-NO_2$, $-CF_3$, $C_{1-6}$-Alkoxy und Halogen substituiert sind und $T_f$ = $-SO_2CF_3$.

**Revendications**

1. Composé de formule I, ou sel pharmaceutiquement acceptable de celui-ci :

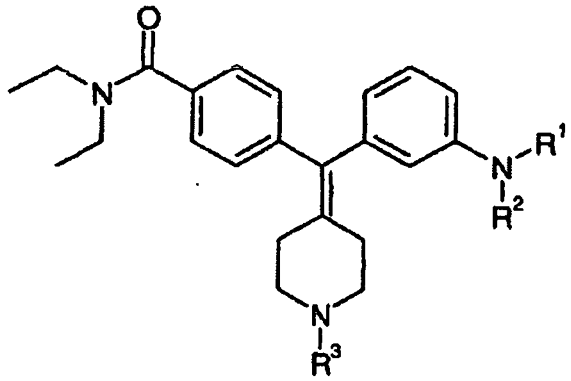

I

dans laquelle

$R^1$ est choisi parmi $C_{3-6}$alkyle, $C_{6-10}$aryle, $C_{2-9}$hétéroaryle, $C_{6-10}$aryl-$C_{1-4}$alkyle, $C_{2-9}$hétéroaryl-$C_{l-4}$alkyle, $C_{3-10}$cycloalkyle, $C_{3-10}$cycloalkyl-$C_{1-4}$alkyle, $R^8$-C (=O) -, $R^8$-$S(=O)_2$-, $R^8$-S(=O)-, $R^8$-NHC(=O)-, $R^8$-C(=S)-et $R^8$-NH-C (=S)-, où $R^8$ est choisi parmi $C_{3-6}$alkyle, $C_{6-10}$aryle, $C_{2-9}$hétéroaryle, $C_{6-10}$aryl-$C_{1-4}$alkyle, $C_{2-9}$hétéroaryl-$C_{1-4}$alkyle, $C_{3-10}$cycloalkyle et $C_{3-10}$cycloalkyl-$C_{1-4}$alkyle, où les dits $C_{3-6}$alkyle, $C_{6-10}$aryle, $C_{2-9}$hétéroaryle, $C_{6-10}$aryl-$C_{1-4}$alkyle, $C_{2-9}$hétéroaryl-$C_{1-4}$alkyle, $C_{3-6}$cycloalkyle et $C_{3-6}$cycloalkyl-$C_{1-4}$alkyle utilisés dans la définition de $R^1$ et $R^8$ sont éventuellement substitués par un ou plusieurs groupements choisis parmi -R, $-NO_2$, -OR, -Cl,- Br, -I, -F, $-CF_3$, -C (=O) R, -C (=O) OH, $-NH_2$, -SH, -NHR, $-NR_2$, -SR, $-SO_3H$, $-SO_2R$, -S (=O) R, -CN, -OH, -C (=O) OR, $-C(=O)NR_2$, -NRC(=O)R et -NRC(=O)-OR, R étant choisi indépendamment parmi -H, $C_{1-6}$alkyle et phényle ;

$R^2$ est choisi parmi -H et $C_{1-6}$alkyle éventuellement substitué par un ou plusieurs groupements choisis parmi halogène, $-CF_3$, -OH et $C_{1-3}$alcoxy, et halogène, ou $R^1$ et $R^2$ sont $C_{1-3}$alkylène qui ensemble forment une partie d'un cycle ; et

$R^3$ est choisi parmi -H, $C_{1-6}$alkyl-O-C (=O) -, $C_{1-6}$alkyle, $C_{3-6}$cycloalkyle et $C_{3-6}$cycloalkyl-$C_{1-4}$alkyle, où lesdits $C_{1-6}$alkyl-O-C (=O) -, $C_{1-6}$alkyle, $C_{3-6}$cycloalkyle et $C_{3-6}$cycloalkyl-$C_{1-4}$alkyle sont éventuellement substitués par un ou plusieurs groupements choisis parmi $C_{1-6}$alkyle, $C_{1-6}$alkyle halogéné, $-NO_2$, $-CF_3$, $C_{1-6}$alcoxy et halogène.

2. Composé selon la revendication 1, **caractérisé en ce que**

$R^1$ est choisi parmi $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle, $C_{3-10}$cycloalkyl-$C_{1-4}$alkyle où lesdits $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle, $C_{3-10}$cycloalkyl-$C_{1-4}$alkyle sont éventuellement substitués par un ou plusieurs groupements choisis parmi $C_{1-4}$alkyle, halogène, $-CF_3$, -OH, $C_{1-3}$alcoxy et phénoxy, et halogène ;

$R^2$ est choisi parmi -H et $C_{1-3}$alkyle ; et

$R^3$ est choisi parmi -H et $C_{1-6}$alkyl-O-C(=O)-.

**3.** Composé selon la revendication 2, **caractérisé en ce que**

$R^1$ est $R^9$-$CH_2$-, où $R^9$ est choisi parmi phényle, pyridyle, thiényle, furyle, imidazolyle, triazolyle, pyrrolyle, thiazolyle, N-oxydo-pyridyle, benzyle, pyridylméthyle, thiénylméthyle, furylméthyle, imidazolylméthyle, triazolylméthyle, pyrrolylméthyle, thiazolylméthyle et N-oxydo-pyridylméthyle, éventuellement substitué par un ou plusieurs groupements choisis parmi $C_{1-4}$alkyle, halogène, -$CF_3$, -OH, $C_{1-3}$alcoxy, phénoxy, et halogène ; et $R^2$ et $R^3$ sont hydrogène.

**4.** Composé selon la revendication 3, **caractérisé en ce que**

$R^9$ est choisi parmi benzyle, phényle, pyridyle, thiényle, furyle, imidazolyle, pyrrolyle et thiazolyle, éventuellement substitué par un ou plusieurs groupements choisis parmi $C_{1-4}$alkyle, halogène, -$CF_3$, -OH, $C_{1-3}$alcoxy, phénoxy, et halogène.

**5.** Composé selon la revendication 4, **caractérisé en ce que**

$R^9$ est choisi parmi benzyle, phényle, pyridyle, thiényle, furyle, imidazolyle, pyrrolyle et thiazolyle.

**6.** Composé selon la revendication 1, **caractérisé en ce que**

$R^1$ est choisi parmi $C_{3-6}$alkyle, $C_{3-10}$cycloalkyle et $C_{3-10}$cycloalkyl-$C_{1-4}$alkyle, où lesdits $C_{3-6}$alkyle, $C_{3-10}$cycloalkyle et $C_{3-10}$cycloalkyl-$C_{1-4}$alkyle sont éventuellement substitués par un ou plusieurs groupements choisis parmi $C_{1-4}$alkyle, halogène, -$CF_3$, -OH, $C_{1-3}$alcoxy, phénoxy, et halogène ;
$R^2$ est -H ou $C_{1-3}$alkyle ; et
$R^3$ est -H, $C_{1-6}$alkyle, $C_{3-6}$cycloalkyle ou $C_{3-6}$cycloalkyl-$C_{1-4}$alkyle, où lesdits $C_{1-6}$alkyle, $C_{3-6}$cycloalkyle et $C_{3-6}$cycloalkyl-$C_{1-4}$alkyle sont éventuellement substitués par un ou plusieurs groupements choisis parmi $C_{1-4}$alkyle, halogène, -$CF_3$, -OH, $C_{1-3}$alcoxy, phénoxy, et halogène.

**7.** Composé selon la revendication 6, **caractérisé en ce que**

$R^1$ est choisi parmi 1-propyle, 2-propyle, 1-butyle, 2-butyle, t-butyle, 2-méthyl-1-propyle, cyclopentyle, cyclohexyle, cycloheptyle, cyclooctyle et cyclononyle ;
$R^2$ est choisi parmi -H, méthyle, éthyle, 1-propyle et 2-propyle ; et
$R^3$ est choisi parmi -H, méthyle, éthyle, allyle, 3,3-diméthylallyle, cyclopropylméthyle, 2-méthoxyéthyle et 3-méthoxy-1-propyle.

**8.** Composé selon la revendication 1, **caractérisé en ce que**

$R^1$ est choisi parmi $R^8$-C(=O) -, $R^8$-S(=O)$_2$-, $R^8$<-S(=0)-, $R^8$-NHC(=O) -, $R^8$-C(=S)- et $R^8$-NH-C(=S)-, où $R^8$ est choisi parmi $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle et $C_{3-10}$cycloalkyl-$C_{1-4}$alkyle ; où lesdits $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-6}$cycloalkyle et $C_{3-6}$cycloalkyl-$C_{1-4}$alkyle sont éventuellement substitués par $C_{1-4}$alkyle, halogène, -$CF_3$, -OH, $C_{1-3}$alcoxy, phénoxy, et halogène ;
$R^2$ est -H ; et
$R^3$ est choisi parmi -H et $C_{1-6}$alkyl-O-C(=O)-.

**9.** Composé selon la revendication 8, **caractérisé en ce que**

$R^8$ est choisi parmi phényle, benzyle, phénéthyle et cyclohexyle, où lesdits phényle, benzyle, phénéthyle et cyclohexyle sont éventuellement substitués par un ou plusieurs groupements choisis parmi méthyle, méthoxy et halogène.

**10.** Composé selon la revendication 1, **caractérisé en ce que**

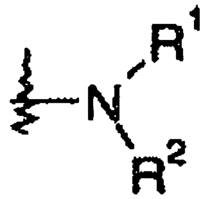

de la formule I est choisi parmi

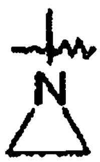 , 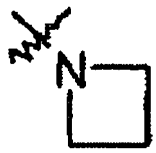 , 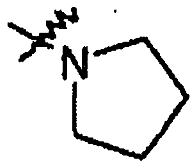 et 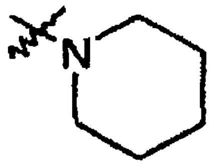 ;

et
$R^3$ est choisi parmi -H et $C_{1-6}$alkyl-O-C (=O) - .

**11.** Composé selon la revendication 1, **caractérisé en ce qu'**il est choisi parmi :

1) le 4-[[3-(benzylamino)phényl](pipéridin-4-ylidène)méthyl]-N,N-diéthylbenzamide,
2) le N,N-diéthyl-4-[{3-[(3-furylméthyl)amino]phényl}(pipéridin-4-ylidène)méthyl]benzamide,
3) le N,N-diéthyl-4-(pipéridin-4-ylidène{3-[(thién-3-ylméthyl)amino]phényl}méthyl)benzamide,
4) le N,N-diéthyl-4-[{3-[(2-phényléthyl)amino]phényl}(pipéridin-4-ylidène)méthyl]benzamide,
5) le 4-[{3-[(4-chlorobenzyl)amino]phényl}(pipéridin-4-ylidène)méthyl]N,N-diéthylbenzamide,
6) le N,N-diéthyl-4-[pipéridin-4-ylidène(3-{[3-(trifluorométhyl)benzyl]amino}phényl)méthyl]-benzamide,
7) le 4-[{3-[(2-chlorobenzyl)amino]phényl}(pipéridin-4-ylidène)méthyl]-N,N-diéthylbenzamide,
8) le N,N-diéthyl-4-[pipéridin-4-ylidène(3-{[4-(trifluorométhyl)benzyl]amino}phényl)méthyl]-benzamide,
9) le N,N-diéthyl-4-[{3-[(2-furylméthyl)amino]phényl}(pipéridin-4-ylidène)méthyl]benzamide,
10) le N,N-diéthyl-4-(pipéridin-4-ylidène{3-[(thién-2-ylméthyl)amino]phényl}méthyl)benzamide,
11) le 4-[{3-[(4cyclohexylméthyl)amino]phényl}(pipéridin-4-ylidène)méthyl]-N,N-diéthylbenzamide,
12) le N,N-diéthyl-4-{pipéridin-4-ylidène[3-propylamino)phényl]méthyl}benzamide,
13) le 4-[[3-(cyclohexylamino)phényl](pipéridin-4-ylidène)méthyl]-N,N-diéthylbenzamide,
14) le 4-[[3-(cyclopentylamino)phényl](pipéridin-4-ylidène)méthyl]-N,N-diéthylbenzamide,
15) le 4-[[3-(cycloheptylamino)phényl](pipéridin-4-ylidène)méthyl]-N,N-diéthylbenzamide,
16) le 4-[{3-[cyclopentyl(méthyl)amino]phényl}(pipéridin-4-ylidène)méthyl]-N,N-diéthylbenzamide,
17) le 4-[[3-(benzoylamino)phényl](pipéridin-4-ylidène)méthyl]-N,N-diéthylbenzamide,
18) le N,N-diéthyl-4-[{3-[(phénylacétyl)amino]phényl}(pipéridin-4-ylidène)méthyl]benzamide,
19) le 4-[{3-[(cyclohexylcarbonyl)amino]phényl}(pipéridin-4-ylidène)méthyl]-N,N-diéthylbenzamide,
20) le 4-[{3-[(cyclohexylacétyl)amino]phényl}(pipéridin-4-ylidène)méthyl]-N,N-diéthylbenzamide,
21) le 4-[(3-{[(cyclophényl)acétyl]amino}phényl)(pipéridin-4-ylidène)méthyl]-N,N-diéthylbenzamide,
22) le 4-[(3-{[(3-chlorophényl)acétyl]amino}phényl)(pipéridin-4-ylidène)méthyl]-N,N-diéthylbenzamide,
23) le N,N-diéthyl-4-[(3-{[(méthylthién-2-yl)acétyl]amino}phényl)(pipéridin-4-ylidène)méthyl]benzamide,
24) le 4-[(3-{[(5-chlorothién-2-yl)acétyl]amino}phényl)(pipéridin-4-ylidène)méthyl]-N,N-diéthylbenzamide,
25) le N,N-diéthyl-4-[(3-{[(2S)-2-phénylpropanoyl]amino}phényl)(pipéridin-4-ylidène)méthyl]benzamide,
26) le N,N-diéthyl-4-[(3-{[(2R)-2-phénylpropanoyl]amino}phényl)(pipéridin-4-ylidène)méthyl]benzamide,
27) le N,N-diéthyl-4-[(3-{[(2S)-2-phénylbutanoyl]amino}phényl)(pipéridin-4-ylidène)méthyl]benzamide,
28) le N,N-diéthyl-4-[(3-{[(2R)-2-phénylbutanoyl]amino}phényl)(pipéridin-4-ylidène)méthyl]benzamide,
29) le 4-[{3-[benzoyl(méthyl)amino]phényl}(pipéridin-4-ylidène)méthyl]-N,N-diéthylbenzamide,
30) le 4-[{3-[(anilinocarbonyl)amino]phényl}(pipéridin-4-ylidène)méthyl]-N,N-diéthylbenzamide,
31) le 4-[(3-{[(benzylamino)carbonyl]amino}phényl)(pipéridin-4-ylidène)méthyl]-N,N-diéthylbenzamide,
32) le N-{3-[{4-[(diéthylamino)carbonyl]phényl}(pipéridin-4-ylidène)méthyl]phényl}pipéridine-1-carboxamide,

33) le N,N-diéthyl-4-[{3-[(phénylsulfonyl)amino]phényl}(pipéridin-4-ylidène)méthyl]benzamide,
34) le 4-[{3-[(benzylsulfonyl)amino]phényl}(pipéridin-4-ylidène)méthyl]-N,N-diéthylbenzamide,
35) le 4-[(3-anilinophényl)(pipéridin-4-ylidène)méthyl]-N,N-diéthylbenzamide,
36) le N,N-diéthyl-4-[{3-[méthyl(phényl)amino]phényl}(pipéridin-4-ylidène)méthyl]benzamide,
37) le N,N-diéthyl-4-[{3-[éthyl(phényl)amino]phényl}(pipéridin-4-ylidène)méthyl]benzamide,
38) le N,N-diéthyl-4-[(3-{[(1S)-1-phényléthyl]amino}phényl)(pipéridin-4-ylidène)méthyl]benzamide,
39) le N,N-diéthyl-4-[(3-{[(1R)-1-phényléthyl]amino}phényl)(pipéridin-4-ylidène)méthyl]benzamide,
40) le 4-[(3-{[(1R)-1-cyclohexyléthyl]amino}phényl)(pipéridin-4-ylidène)méthyl]-N,N-diéthylbenzamide,
41) le 4-[(3-{[(1S)-1-cyclohexyléthyl]amino}phényl)(pipéridin-4-ylidène)méthyl]-N,N-diéthylbenzamide,
42) le N,N-diéthyl-4-[{3-[(1-méthyl-1-phényléthyl)amino]phényl}(pipéridin-4-ylidène)méthyl]benzamide,
43) le 4-[{3-[cyclohexyl(méthyl)amino]phényl}(pipéridin-4-ylidène)méthyl]-N,N-diéthylbenzamide,
44) le N,N-diéthyl-4-[pipéridin-4-ylidène(3-pipéridin-1-ylphényl)méthyl]benzamide,
45) le N,N-diéthyl-4-[pipéridin-4-ylidène(3-pyrrolidin-1-ylphényl)méthyl]benzamide,
46) le N,N-diéthyl-4-[[3-[(2-éthyl-1-oxobutyl)amino]phényl]-4-pipéridinylidèneméthyl]benzamide,
47) le N-[3-[[4-[(diéthylamino)carbonyl]phényl]-4-pipéridinylidèneméthyl]phényl]-1-méthyl-1H-1,2,3-benzotriazol-5-carboxamide,
48) le 6-chloro-N-[3-[[4-[(diéthylamino)carbonyl]phényl]-4-pipéridinylidèneméthyl]phényl]-3-pyridinecarboxamide,
49) le N-[3-[[4-[(diéthylamino)carbonyl]phényl]-4-pipéridinylidèneméthyl]phényl]-2-méthoxybenzamide,
50) le N-[3-[[4-[(diéthylamino)carbonyl]phényl]-4-pipéridinylidèneméthyl]phényl]-2-quinoxalinecarboxamide,
51) le N-[3-[[4-[(diéthylamino)carbonyl]phényl]-4-pipéridinylidèneméthyl]phényl]-2,5-difluorobenzamide,
52) le 3-chloro-N-[3-[[4-[(diéthylamino)carbonyl]phényl]-4-pipéridinylidèneméthyl]phényl]-2-thiophènecarboxamide,
53) le N-[3-[[4-[(diéthylamino)carbonyl]phényl]-4-pipéridinylidèneméthyl]phényl]-3-méthylbenzamide,
54) le N,N-diéthyl-4-[[3-[[(méthylphénylamino)carbonyl]amino]phényl]-4-pipéridinylidèneméthyl] -benzamide et les sels pharmaceutiquement acceptables de ceux-ci.

**12.** Composé selon l'une quelconque des revendications 1 à 11, destiné à être utilisé comme médicament.

**13.** Utilisation d'un composé selon l'une quelconque des revendications 1 à 11, dans la fabrication d'un médicament destiné à la thérapie de la douleur, de l'anxiété ou de troubles gastro-intestinaux fonctionnels.

**14.** Composition pharmaceutique comprenant un composé selon l'une quelconque des revendications 1 à 11 et un support pharmaceutiquement acceptable.

**15.** Procédé de préparation d'un composé de formule III

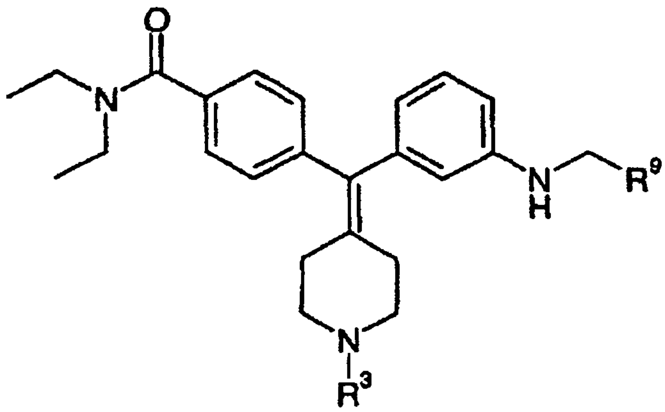

III

comprenant :

la réaction d'un composé de formule II

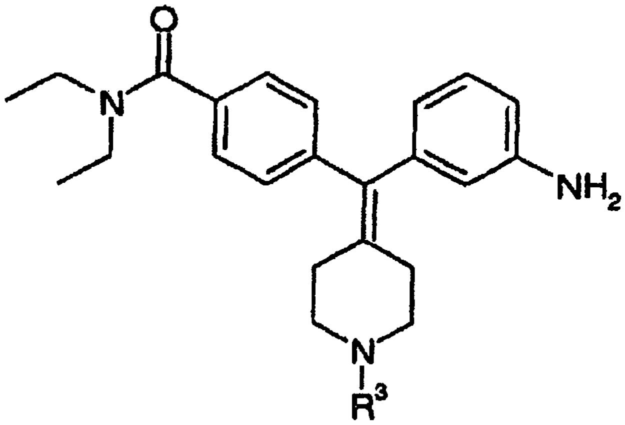

II

avec $R^9$-CHO en présence d'un agent de réduction pour former le composé de formule III,

dans lesquelles

$R^9$ est choisi parmi phényle, pyridyle, thiényle, furyle, imidazolyle, triazolyle, pyrrolyle, thiazolyle, N-oxydo-pyridyle, benzyle, pyridylméthyle, thiénylméthyle, furylméthyle, imidazolylméthyle, triazolylméthyle, pyrrolylméthyle, thiazolylméthyle et N-oxydo-pyridylméthyle, éventuellement substitués par un ou plusieurs groupements choisis parmi $C_{1-4}$alkyle, halogène, $-CF_3$, -OH, $C_{1-3}$alcoxy, phénoxy et halogène ; et

$R^3$ est choisi parmi $C_{1-6}$alkyl-O-C(=O)-, $C_{1-6}$alkyle, $C_{3-6}$cycloalkyle et $C_{3-6}$cycloalkyl-$C_{1-4}$alkyle, où lesdits $C_{1-6}$alkyl-O-C(=O)-, $C_{1-6}$alkyle, $C_{3-6}$cycloalkyle et $C_{3-6}$cycloalkyl-$C_{1-4}$alkyle sont éventuellement substitués par un ou plusieurs groupements choisis parmi $C_{1-6}$alkyle, $C_{1-6}$alkyle halogéné, $-NO_2$, $-CF_3$, $C_{1-6}$alcoxy et halogène.

**16.** Procédé de préparation d'un composé de formule IV,

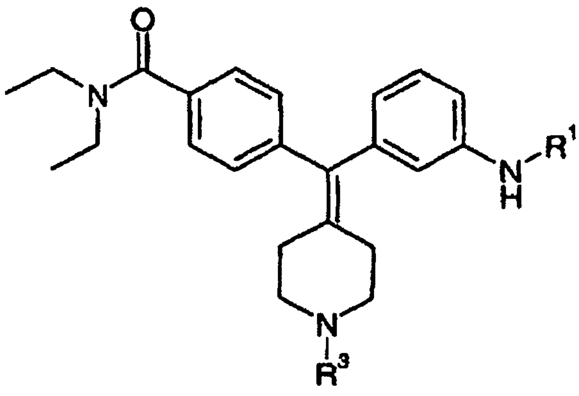

IV

comprenant : la réaction d'un composé de formule II

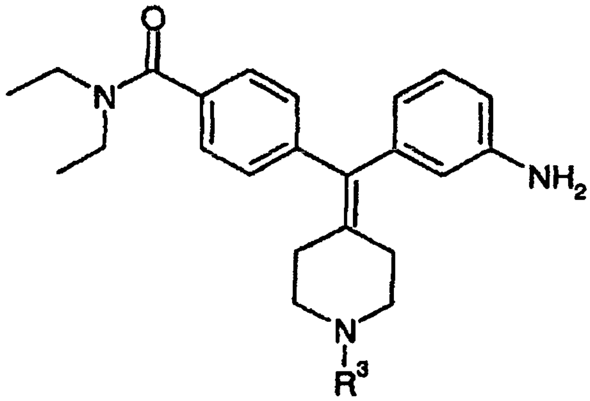

II

avec $R^1$-X pour former le composé de formule IV, dans lesquelles

X est halogène ;

$R^1$ est choisi parmi $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle, $C_{3-10}$cycloalkyl-$C_{1-4}$alkyle, où lesdits $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle, $C_{3-10}$cycloalkyl-$C_{1-4}$alkyle sont éventuellement substitués par un ou plusieurs groupements choisis parmi $C_{1-4}$alkyle, halogène, $-CF_3$, -OH, $C_{1-3}$alcoxy, phénoxy, et halogène ; et

$R^3$ choisi parmi $C_{1-6}$alkyl-O-C(=O)-, $C_{1-6}$alkyle, $C_{3-6}$cycloalkyle et $C_{3-6}$cycloalkyl-$C_{1-4}$alkyle, où lesdits $C_{1-6}$alkyl-O-C(=O)-, $C_{1-6}$alkyle, $C_{3-6}$cycloalkyle et $C_{3-6}$cycloalkyl-$C_{1-4}$alkyle sont éventuellement substitués par un ou plusieurs groupements choisi parmi $C_{1-6}$alkyle, $C_{1-6}$alkyle halogéné, $-NO_2$, $-CF_3$, $C_{1-6}$alcoxy et halogène.

**17.** Procédé de préparation d'un composé de formule I

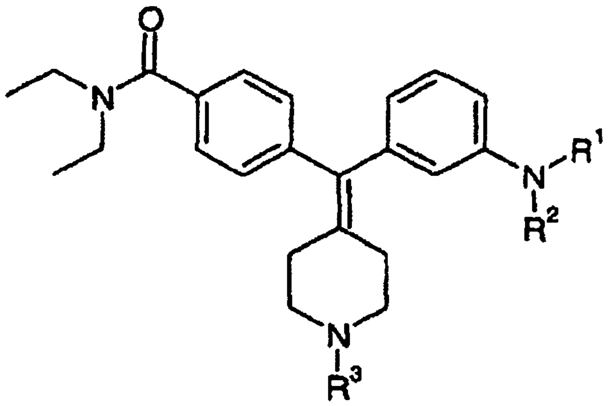

I

comprenant : la réaction d'un composé de formule V,

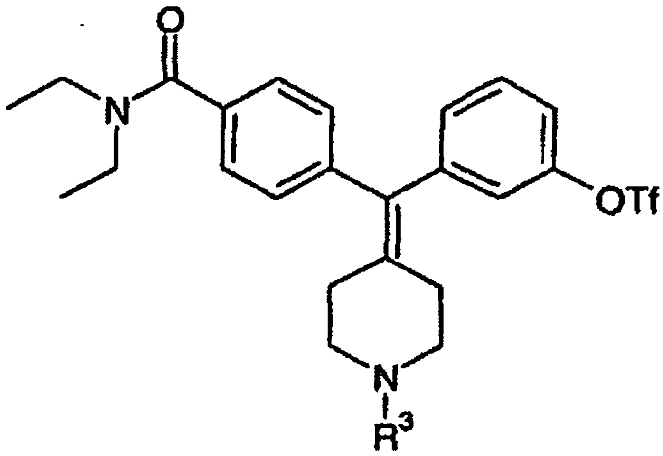

**V**

avec $R^1R^2NH$ pour former le composé de formule I, dans lesquelles

$R^1$ est choisi parmi $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cyloalkyle, $C_{3-10}$cycloalkyl-$C_{1-4}$alkyle où lesdits $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle, $C_{3-10}$cycloalkyl-$C_{1-4}$alkyle sont éventuellement substitués par un ou plusieurs groupements choisis parmi $C_{1-4}$alkyle, halogène, $-CF_3$, -OH, $C_{1-3}$alcoxy, phénoxy, et halogène ;

$R^2$ est choisi parmi -H et $C_{1-6}$alkyle éventuellement substitué par un ou plusieurs groupements choisis parmi halogène, $-CF_3$, -OH, $C_{1-3}$alcoxy et halogène, ou $R^1$ et $R^2$ sont $C_{1-3}$alkylène qui ensemble forment une partie d'un cycle ; et

$R^3$ est choisi parmi $C_{1-6}$alkyl-O-C(=O)-, $C_{1-6}$alkyle, $C_{3-6}$cycloalkyle et $C_{3-6}$cycloalkyl-$C_{1-4}$alkyle, où lesdits $C_{1-6}$alkyl-O-C (=O) -, $C_{1-6}$alkyle, $C_{3-6}$cycloalkyle et $C_{3-6}$cycloalkyl-$C_{1-4}$alkyle sont éventuellement choisis parmi un ou plusieurs groupements choisis parmi $C_{1-6}$alkyle, $C_{1-6}$alkyle halogéné, $-NO_2$, $-CF_3$, $C_{1-6}$alcoxy et halogène.

**18.** Procédé de préparation d'un composé de formule VI,

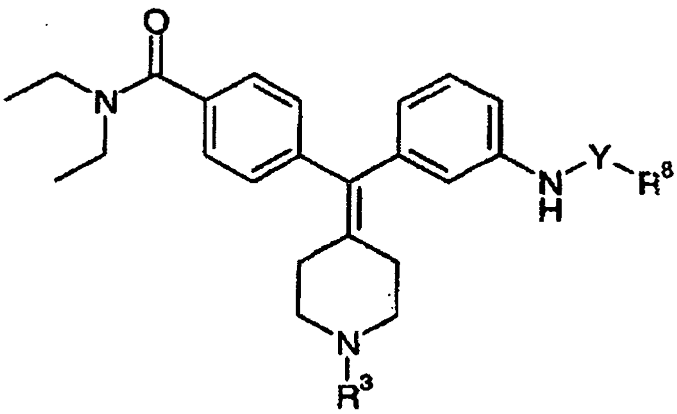

**VI**

comprenant : la réaction d'un composé de formule VII,

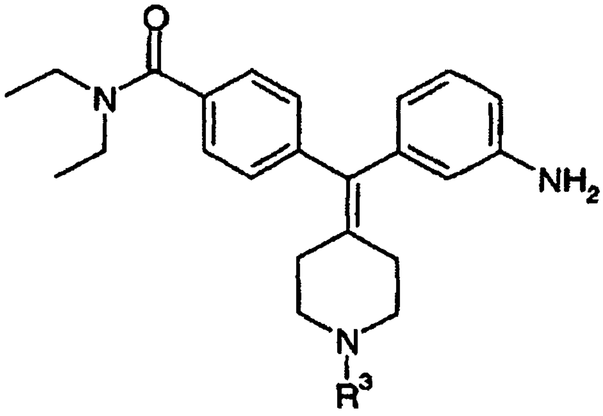

VII

avec $R^8$-Y-X ou $R^8$-Y-O-Y-$R^8$ pour former le composé de formule VI :
dans lesquelles

X est halogène ;
Y est choisi parmi -C(=O)- et -S(=O)$_2$- ;
$R^8$ est choisi parmi $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle et $C_{3-10}$cycloalkyl-$C_{1-4}$alkyle, où lesdits $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-6}$cycloalkyle et $C_{3-6}$cycloalkyl-$C_{1-4}$alkyle sont éventuellement substitués par $C_{1-4}$alkyle, halogène, -$CF_3$, -OH, $C_{1-3}$alcoxy, phénoxy, et halogène ; et
$R^3$ est choisi parmi. $C_{1-6}$alkyl-O-C(=O)-, $C_{1-6}$alkyle, $C_{3-6}$cycloalkyle et $C_{3-6}$cycloalkyl-$C_{1-4}$alkyle, où lesdits $C_{1-6}$alkyl-O-C (=O) -, $C_{1-6}$alkyle, $C_{3-6}$cycloalkyle et $C_{3-6}$cycloalkyl-$C_{1-4}$alkyle sont éventuellement substitués par un ou plusieurs groupements choisis parmi $C_{1-6}$alkyle, $C_{1-6}$alkyle halogéné, -$NO_2$, -$CF_3$, $C_{1-6}$alcoxy et halogène.

**19.** Procédé de préparation d'un composé de formule VIII,

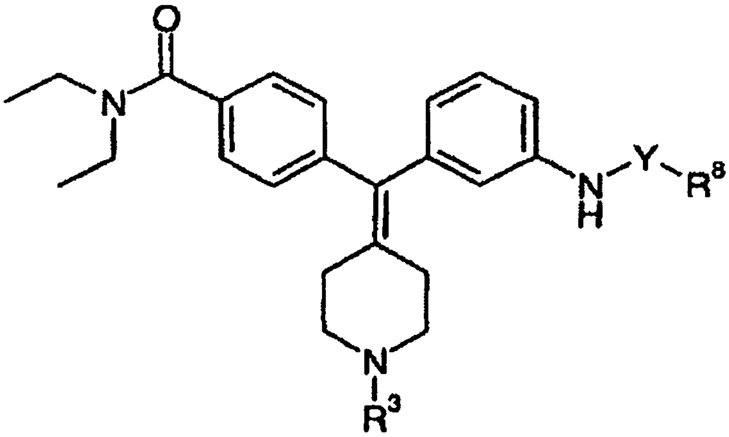

VIII

comprenant : la réaction d'un composé de formule VII,

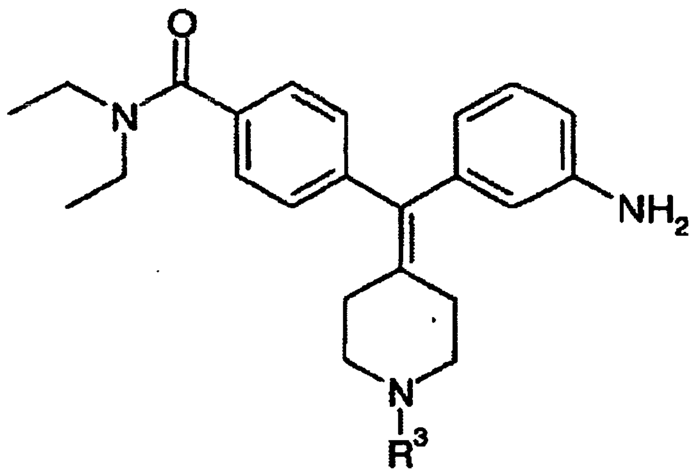

VII

avec $R^8$-Z pour former le composé de formule VIII:
dans lesquelles

Z est choisi parmi -NCO et -NCS ;
Y est choisi parmi -C(=O) NH- et -C(=S)NH- ;
$R^8$ est choisi parmi $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle et $C_{3-10}$cycloalkyl-$C_{1-4}$alkyle, où lesdits $C_{3-6}$alkyle, $C_{6-10}$aryle, $C_{2-6}$hétéroaryle, $C_{6-10}$aryl-$C_{1-4}$alkyle, $C_{2-6}$hétéroaryl-$C_{1-4}$alkyle, $C_{3-10}$cycloalkyle et $C_{3-10}$cycloalkyl-$C_{1-4}$alkyle sont éventuellement substitués par $C_{1-4}$alkyle, halogène, $-CF_3$, -OH, $C_{1-3}$alcoxy, phénoxy, et halogène ; et
$R^3$ est choisi parmi $C_{1-6}$alkyl-O-C (=O) -, $C_{1-6}$alkyle, $C_{3-6}$cycloalkyle et $C_{3-6}$cycloalkyl-$C_{1-4}$alkyle, où lesdits $C_{1-6}$alkyl-O-C (=O) -, $C_{1-6}$alkyle, $C_{3-6}$cycloalkyle et $C_{3-6}$cycloalkyl-$C_{1-4}$alkyle sont éventuellement substitués par un ou plusieurs groupements choisis parmi $C_{1-6}$alkyle, $C_{1-6}$alkyle halogéné, $-NO_2$, $-CF_3$, $C_{1-6}$alcoxy et halogène.

**20.** Composé de formule V,

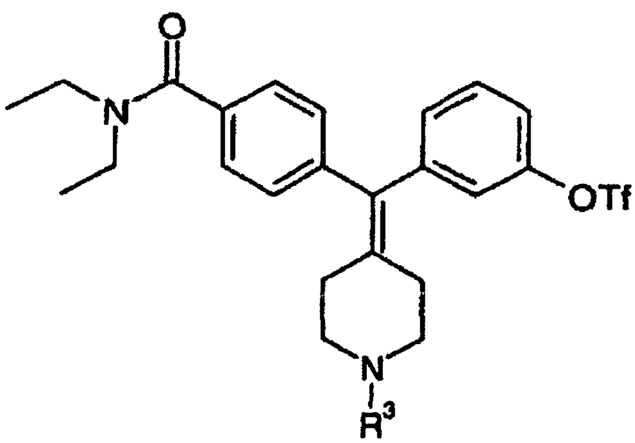

V

dans laquelle

$R^3$ est choisi parmi $C_{1-6}$alkyl-O-C (=O) -, $C_{1-6}$alkyle, $C_{3-6}$cycloalkyle et $C_{3-6}$cycloalkyl-$C_{1-4}$alkyle, où lesdits $C_{1-6}$alkyl-O-C (=O) -, $C_{1-6}$alkyle, $C_{3-6}$cycloalkyle et $C_{3-6}$cycloalkyl-$C_{1-4}$alkyle sont éventuellement choisis parmi un ou plusieurs groupements choisis parmi $C_{1-6}$alkyle, $C_{1-6}$alkyle halogéné, $-NO_2$, $-CF_3$, $C_{1-6}$alcoxy et halogène et

$T_f = -SO_2CF_3$.